(19) Europäisches Patentamt
European Patent Office
Office européen des brevets

(11) **EP 4 137 160 A1**

(12) **EUROPEAN PATENT APPLICATION**

(43) Date of publication:
**22.02.2023 Bulletin 2023/08**

(21) Application number: **22179414.2**

(22) Date of filing: **14.03.2014**

(51) International Patent Classification (IPC):
**A61K 47/68** (2017.01) **A61K 38/05** (2006.01)
**A61P 35/00** (2006.01)

(52) Cooperative Patent Classification (CPC):
**A61K 38/05; A61K 47/6803; A61K 47/6849;
A61P 35/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **15.03.2013 US 201361792834 P**

(62) Document number(s) of the earlier application(s) in
accordance with Art. 76 EPC:
**14722865.4 / 2 968 589**

(71) Applicant: **AbbVie Inc.**
**North Chicago, IL 60064 (US)**

(72) Inventors:
• **LEANNA, Marvin Robert**
**Grayslake, Ilinois 60030 (US)**

• **BECKER, Calvin Lawrence**
**Kenosha, Wisconsin 53142 (US)**

(74) Representative: **Redhouse, Juliet Lauren et al**
**Mathys & Squire**
**The Shard**
**32 London Bridge Street**
**London SE1 9SG (GB)**

Remarks:
•The complete document including Reference
Table(s) and the Sequence Listing(s) can be
downloaded from the EPO website
•This application was filed on 16-06-2022 as a
divisional application to the application mentioned
under INID code 62.
•Claims filed after the date of receipt of the divisional
application (Rule 68(4) EPC).

(54) **ANTIBODY DRUG CONJUGATE (ADC) PURIFICATION**

(57) The invention provides methods for obtaining compositions having antibody drug conjugates (ADCs) with specified drug to antibody ratios (DARs). Included in the invention is a method for purifying an ADC mixture having ADCs with a drug loaded species of 6 or more by contacting the mixture with a hydrophobic resin such that a composition comprising less than 15% of the 6 or more drug loaded species is obtained. The invention also provides a composition wherein 70% or more of the ADCs present have a drug loaded species of 4 or less, wherein the ADC comprises an anti-EGFR antibody and an auristatin.

## Figure 1

**Description**

**RELATED APPLICATIONS**

This application claims the benefit of U.S. Provisional Application No. 61/792,834, filed on March 15, 2013. The contents of the aforementioned priority document are hereby incorporated by reference in its entirety.

**BACKGROUND OF THE INVENTION**

Antibody drug conjugates (ADC) are an emerging class of potent anti-cancer agents, which have recently demonstrated remarkable clinical benefit. ADCs are comprised of a cytotoxic agent attached to an antibody via a stable linker. Putatively, by a series of events, including antigen binding at the cell surface, endocytosis, trafficking to the lysosome, ADC degradation, release of payload, interruption of cellular processing (e.g. mitosis) and apoptosis, ADCs may destroy cancer cells possessing an over-expression of cell-surface proteins. ADCs combine the antigen-driven targeting properties of monoclonal antibodies with the potent anti-tumor effects of cytoxic agents. For example, in 2011 ADCETRIS® (an anti-CD30 antibody-MMAE ADC) gained regulatory approval for the treatment of refractory Hodgkin lymphoma and systemic anaplastic lymphoma.

Studies have demonstrated deleterious effects of a high drug loaded ADCs (Liu *et al.* (2010) *Analy. Chem.* 82:5219). These deleterious effects of higher levels of conjugation include increased propensity towards aggregate formation (King et al. (2002) J Med. Chem. 45:4336; Hollander et al. (2008) Bioconjugate Chem 19:358; Burke et al. (2009) Bioconjugate Chem 20:1242; and Zhao et al. (2011) J Med. Chem. 54:3606).

Controlling the drug load of an ADC has been attempted using various methods, including: (i) limiting the molar excess of drug-linker intermediate or linker reagent relative to antibody, (ii) limiting the conjugation reaction time or temperature, and (iii) partial or limiting reductive conditions for cysteine thiol modification. While reduction methods that limit the number of attachment sites on the antibody have been used to achieve ADCs with fewer drugs per antibody (Alley et al. (2004) Proc Amer Assoc Cancer Res 45:Abst 627), there remains a need for methods and compositions that can provide optimal drug loaded species.

**SUMMARY OF INVENTION**

The invention provides effective methods for separating antibody drug conjugates (ADCs) having different drug loads, as well as compositions obtained using such methods. The invention also provides compositions where higher drug load species of ADCs are removed.

The invention features, in one embodiment, a method of obtaining a composition comprising Antibody Drug Conjugates (ADCs), said method comprising contacting an ADC mixture comprising a drug loaded species of 4 or less and a drug loaded species of 6 or more with a hydrophobic resin, wherein the amount of hydrophobic resin contacted with the ADC mixture is sufficient to allow binding of the drug loaded species of 6 or more to the resin but does not allow significant binding of the drug loaded species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin. In one embodiment, the composition comprises less than 10% of the drug loaded species of 6 or more. In another embodiment, the composition comprises 5% or less of the drug loaded species of 6 or more.

In one embodiment, the method of the invention includes the use of a hydrophobic resin weight which is 3 to 12 times the weight of the drug loaded species of 6 or more in the ADC mixture. In one embodiment, the hydrophobic resin weight is 4 to 8 times the weight of the drug loaded species of 6 or more in the ADC mixture. In a further embodiment, the hydrophobic resin weight is 5 to 10 times the weight of the drug loaded species of 6 or more in the ADC mixture.

In yet another embodiment, the hydrophobic resin weight is 6 to 12 times the weight of the drug loaded species of 6 or more in the ADC mixture, and wherein the ADC mixture comprises between 0 to 1 N NaCl, or an equivalent ionic strength thereof. In a further embodiment, the hydrophobic resin weight is 3 to 6 times the weight of the drug loaded species of 6 or more in the ADC mixture, and wherein the ADC mixture comprises between 1 to 2N NaCl, or an equivalent ionic strength thereof. In yet another embodiment, the hydrophobic resin weight is 3 to 7 times the weight of the drug loaded species of 6 or more in the ADC mixture, and wherein the auristatin is monomethylauristatin E (MMAE). In a further embodiment, the hydrophobic resin weight is 5 to 10 times the weight of the drug loaded species of 6 or more in the ADC mixture, and wherein the auristatin is monomethylauristatin F (MMAF). In a further embodiment, the hydrophobic resin weight is 3 to 7 times the weight of the drug loaded species of 6 or more in the ADC mixture, and wherein the auristatin is monomethylauristatin E (MMAE).

The invention further provides a method of producing a composition comprising ADCs with an average Drug-to-Antibody Ratio (DAR) of 4.5 or less and comprising less than 15% undesired ADCs, said method comprising contacting an ADC mixture with a hydrophobic resin, wherein the amount of hydrophobic resin contacted with the ADC mixture is sufficient to allow binding of the undesired ADCs; and removing the hydrophobic resin from the ADC mixture, such that the composition with an average DAR of 4.5 or less and comprising less than 15% undesired ADCs is produced, wherein the ADC comprises an antibody conjugated to an auristatin. In one embodiment, the composition with an average DAR of 4 or less comprises less than 10% undesired ADCs. In one embodiment, the undesired ADCs are 6 and 8 drug loaded species. In a further embodiment, the undesired ADCs are an 8 drug loaded species.

In one embodiment, the amount of hydrophobic resin added to the ADC mixture is a resin weight which is 3 to 12 times the weight of the undesired ADCs in the ADC mixture.

In a further embodiment, the amount of hydrophobic resin added to the ADC mixture is a resin weight which is 4 to 8 times the weight of the drug loaded species of 6 or more in the ADC mixture. In yet another embodiment, the amount of hydrophobic resin added to the ADC mixture is a resin weight which is 5 to 7 times the weight of the drug loaded species of 6 or more in the ADC mixture. In a further embodiment, the hydrophobic resin weight is 6 to 12 times the weight of the drug loaded species of 6 or more in the ADC mixture and wherein the ADC mixture comprises between 0 to 1 N NaCl, or an equivalent ionic strength thereof. In one embodiment, the hydrophobic resin weight is 3 to 6 times the weight of the drug loaded species of 6 or more in the ADC mixture and the ADC mixture comprises between 1 to 2 N NaCl, or an equivalent ionic strength thereof. In another embodiment, the hydrophobic resin weight is 3 to 7 times the weight of the drug loaded species of 6 or more in the ADC mixture, and wherein the auristatin is monomethylauristatin E (MMAE). In still another embodiment, the hydrophobic resin weight is 5 to 10 times the weight of the drug loaded species of 6 or more in the ADC mixture, and wherein the auristatin is monomethylauristatin F (MMAF). In yet a further embodiment, the hydrophobic resin weight is 3 to 7 times the weight of the drug loaded species of 6 or more in the ADC mixture, and wherein the auristatin is monomethylauristatin E (MMAE). In one embodiment, the hydrophobic resin weight is 5 to 10 times the weight of the drug loaded species of 6 or more in the ADC mixture, and wherein the auristatin is monomethylauristatin F (MMAF).

In one embodiment, the method of the invention is used to obtain a composition comprising ADCs with an average DAR of 4.5 or less. In one embodiment, the method of the invention is used to obtain a composition comprising ADCs with an average DAR of 4 or less. In one embodiment, the method of the invention is used to obtain a composition comprising ADCs with an average DAR of 3.5 or less. In one embodiment, the method of the invention is used to obtain a composition comprising ADCs with an average DAR of 3 or less. In one embodiment, the composition has an average DAR of 2.5 or less.

In one embodiment, the method of the invention features adding a hydrophobic resin to an ADC mixture to form a resin mixture, wherein the resin mixture has an ionic strength which is equal to or higher than the ADC mixture.

In a further embodiment of the invention, the ADC mixture was obtained following an ultrafiltration /diafiltration process.

In one embodiment of the invention, the hydrophobic resin used in the methods of the invention is a butyl hydrophobic resin.

In one embodiment of the invention, the method of the invention is a batch process or, alternatively, a circulation process or a flow through process.

In one embodiment, the invention features a composition obtained using the methods described herein.

The invention further features a composition comprising ADCs, wherein 70% of the ADCs have a drug loaded species of 4 or less, wherein the ADC comprises an anti-EGFR antibody (e.g., antibody 1) and an auristatin (e.g., MMAE or MMAF). In one embodiment of the invention, the composition comprises 75% ADCs present having a drug loaded species of 4 or less. In another embodiment of the invention, the composition comprises 80% ADCs present having a drug loaded species of 4 or less. In one embodiment of the invention, the composition comprises 85% ADCs present having a drug loaded species of 4 or less. In a further embodiment of the invention, the composition comprises 90% ADCs having a drug loaded species of 4 or less. In one embodiment, the composition of the invention comprises 95% ADCs present having a drug loaded species of 4 or less. In another embodiment, the composition of the invention comprises ADCs wherein 70% or more of the ADCs have a drug loaded species of 4 to 1, 3 to 1, or, alternatively, 2 to 1.

The invention further features a composition comprising ADCs with an average DAR of 4.5 or less, wherein the ADC comprises an anti-EGFR antibody (e.g., antibody 1) and an auristatin (e.g., MMAE or MMAF). In one embodiment of the invention, the composition comprises ADCs having an average DAR of 4 or less. In another embodiment of the invention, the composition comprises ADCs having an average DAR of 4 or less. In yet another embodiment of the invention, the composition comprises ADCs having an average DAR of 3.5 or less. In one embodiment of the invention, the composition comprises ADCs having an average DAR of 3 or less. In yet another embodiment of the invention, the composition comprises ADCs having an average DAR of 2.5 or less. In another embodiment, the composition of the invention comprises anti-EGFR ADCs (e.g., antibody 1 conjugated to MMAE or MMAF) with an average DAR of 4.5 to 0.001, 4 to 0.001, 3.5 to 0.001, 3 to 0.001, or, alternatively, 2.5 to 0.001.

In one embodiment, the methods and compositions of the invention include an ADC comprising an anti-Epidermal Growth Factor Receptor (EGFR) antibody. In one embodiment of the invention, the anti-EGFR antibody comprises a light chain variable region comprising a Complementarity Determining Region 1 (CDR1), CDR2, and CDR3 domain comprising the amino acid sequence as set forth in SEQ ID NO: 7, SEQ ID NO: 8, and SEQ ID NO: 9, respectively, and comprises a heavy chain variable region comprising a CDR1, CDR2, and CDR3 domain comprising the amino acid sequence as set forth in SEQ ID NO: 2, SEQ ID NO: 3, and SEQ ID NO: 4. In another embodiment of the invention, the anti-EGFR antibody comprises a light chain variable region comprising the amino acid sequence set forth in SEQ ID NO: 6 and a heavy chain variable region comprising the amino acid sequence set forth in SEQ ID NO: 1.

In another embodiment of the invention, the anti-EGFR ADC comprises CDRs (*i.e.,* light chain CDR1, CDR2, and CDR3) described in the light chain variable region set forth in the amino acid sequence of SEQ ID NO: 6, and CDRs (*i.e.,* heavy chain CDR1, CDR2, and CDR3) described in the amino acid sequence of SEQ ID NO: 1.

In a further embodiment, the methods and compositions of the invention feature an auristatin which is either monomethylauristatin E (MMAE) or monomethylauristatin F (MMAF).

In one embodiment, the MMAE is conjugated to the antibody via a valine-citrulline (vc) linker (vc-MMAE). In another embodiment, the MMAF is conjugated to the antibody via a maleimidocaproyl linker (mc-MMAF).

In one embodiment, the composition of the invention is a pharmaceutical composition.

Also included in the invention are methods of treating cancer in a subject comprising administering a composition described herein to the subject such that cancer is treated. In one embodiment, the cancer is selected from the group consisting of squamous tumors (including, squamous tumors of the lung, head and neck, cervical, etc.), glioblastoma, glioma, non-small cell lung cancer, lung cancer, colon cancer, head and neck cancer, breast cancer, squamous cell tumors, anal cancer, skin cancer, and vulvar cancer.

In one embodiment, the compositions of the invention are used to treat glioblastoma multiforme.

In one embodiment, the compositions of the invention are used to treat a solid tumor having overexpression of EGFR. In one embodiment, the compositions of the invention are used to treat a subject having an advanced solid tumor likely to overexpress EGFR.

In one embodiment, the compositions of the invention are administered intravenously.

## BRIEF DESCRIPTION OF THE DRAWINGS

**Figure 1** provides an overview of the processes described in the examples, including reduction of Antibody 1, conjugation of Antibody 1 to vcMMAE, and purification of the ADC using batch purification with HIC resin.

**Figure 2** graphically depicts HIC HPLC analysis of an Antibody 1 ADC solution before and after HIC resin batch purification.

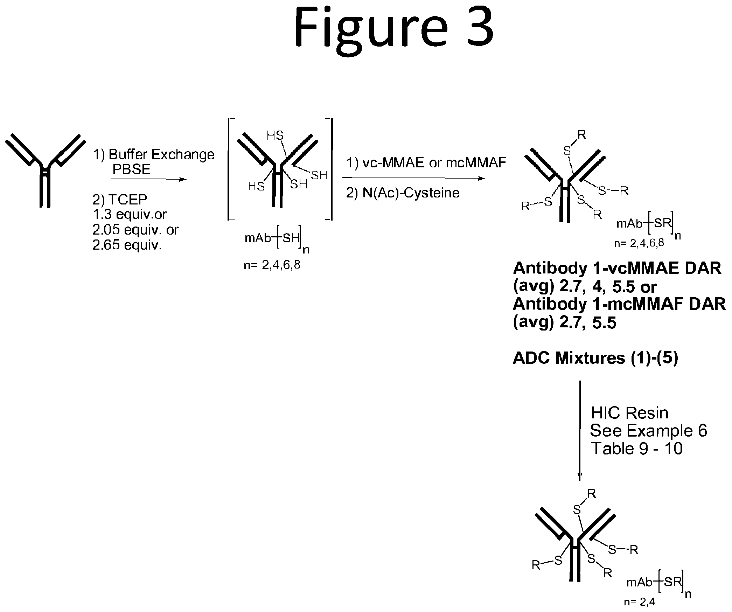

**Figure 3** provides an overview of the process described in Example 6 for the purification of ADC mixtures with average DARs of 2.7, 4, and 5.5.

## DETAILED DESCRIPTION

### I. Definitions

In order that the present invention may be more readily understood, certain terms are first defined. In addition, it should be noted that whenever a value or range of values of a parameter are recited, it is intended that values and ranges intermediate to the recited values are also intended to be part of this invention.

The term "antibody-drug-conjugate" or "ADC" refers to a binding protein, such as an antibody or antigen binding fragment thereof, chemically linked to one or more chemical drug(s) (also referred to herein as agent(s)) that may optionally be therapeutic or cytotoxic agents. In a preferred embodiment, an ADC includes an antibody, a cytotoxic or therapeutic drug, and a linker that enables attachment or conjugation of the drug to the antibody. An ADC typically has anywhere from 1 to 8 drugs conjugated to the antibody, including drug loaded species of 2, 4, 6, or 8. Non-limiting examples of drugs that may be included in the ADCs are mitotic inhibitors, antitumor antibiotics, immunomodulating agents, vectors for gene therapy, alkylating agents, antiangiogenic agents, antimetabolites, boron-containing agents, chemoprotective agents, hormones, antihormone agents, corticosteroids, photoactive therapeutic agents, oligonucleotides, radionuclide agents, topoisomerase inhibitors, tyrosine kinase inhibitors, and radiosensitizers.

The terms "anti-Epidermal Growth Factor antibody drug conjugate," "anti-EGFR antibody drug conjugate," or "anti-EGFR ADC", used interchangeably herein, refer to an ADC comprising an antibody that specifically binds to EGFR, whereby the antibody is conjugated to one or more chemical agent(s). In one embodiment, the anti-EGFR antibody drug conjugate is Antibody 1 conjugated to an auristatin, e.g., MMAE or MMAF. Amino acid sequences corresponding to the light and heavy chains of Antibody 1 are provided in SEQ ID NOs: 1-10.

The term "auristatin", as used herein, refers to a family of antimitotic agents. Auristatin derivatives are also included within the definition of the term "auristatin". Examples of auristatins include, but are not limited to, auristatin E (AE), monomethylauristatin E (MMAE), monomethylauristatin F (MMAF), and synthetic analogs of dolastatin.

The term "drug-to-antibody ratio" or "DAR" refers to the number of drugs, e.g., auristatin, attached to the antibody of the ADC. The DAR of an ADC can range from 1 to 8, although higher loads, e.g., 10, are also possible depending on the number of linkage site on an antibody. The term DAR may be used in reference to the number of drugs loaded onto an individual antibody, or, alternatively, may be used in reference to the average or mean DAR of a group of ADCs.

The term "undesired ADC species", as used herein, refers to any drug loaded species which is to be separated from an ADC species having a different drug load. In one embodiment, the term undesired ADC species may refer to drug loaded species of 6 or more, *i.e.,* ADCs with a DAR of 6 or more, including DAR6, DAR7, DAR8, and DAR greater than 8 (*i.e.,* drug loaded species of 6, 7, 8, or greater than 8). In a separate embodiment, the term undesired ADC species may refer to drug loaded species of 8 or more, *i.e.,* ADCs with a DAR of 8 or more, including DAR8, and DAR greater than 8 (*i.e.,* drug loaded species of 8, or greater than 8).

The term "ADC mixture", as used herein, refers to a composition containing a heterogeneous DAR distribution of ADCs. In one embodiment, an ADC mixture contains ADCs having a distribution of DARs of 1 to 8, *e.g.,* 2, 4, 6, and 8 (*i.e.,* drug loaded species of 2, 4, 6, and 8). Notably, degradation products may result such that DARs of 1, 3, 5, and 7 may also be included in the mixture. Further, ADCs within the mixture may also have DARs greater than 8. The ADC mixture results from interchain disulfide reduction followed by conjugation. In one embodiment, the ADC mixture comprises both ADCs with a DAR of 4 or less (*i.e.,* a drug loaded species of 4 or less) and ADCs with a DAR of 6 or more (*i.e.,* a drug loaded species of 6 or more).

As used herein, the term "hydrophobic resin" or "hydrophobic interaction resin" refers to a medium consisting of hydrophobic ligands used for purposes of purifying a mixture of molecules, wherein the presence of hydrophobic surface moieties on the molecules within the mixture facilitates an interaction with the medium such that interacting molecules are at least transiently bound to the medium. In one embodiment, the hydrophobic resin is a resin comprising alkyl moieties, e.g., a $C_4$-$C_8$ alkyl hydrophobic resin, which is a resin comprising a four to eight straight or branched chain carbon membered alkane radical group such as butyl, pentyl, hexyl, heptyl, or octyl group coupled to a solid support (e.g., agarose, silica, etc.). Examples of hydrophobic alkyl resins include a hydrophobic butyl resin or a hydrophobic hexyl resin. In one embodiment, the hydrophobic resin is a resin comprising aryl moieties, e.g., a hydrophobic phenyl resin. In one embodiment, the hydrophobic resin comprises an alkenyl moiety. In one embodiment, the hydrophobic resin comprises an ether moiety. In yet another embodiment, the hydrophobic resin comprises a phenyl moiety. The hydrophobic moieties (e.g., alkyl, aryl, etc.) may be linked to an inert substance (e.g., silica, agarose, and/or other polysaccharide polymers). In one embodiment, the resin is a methacrylate resin.

The term "ionic strength" broadly refers to a measure of the concentration of ions in a solution, *i.e.,* the conductivity of a solution. Exemplary salts that may be used to modulate the ionic strength of a solution include, but are not limited to sodium bromide, sodium chloride, sodium citrate, sodium iodide, sodium phosphate, sodium sulfate, potassium bromide, potassium chloride, potassium citrate, potassium iodide, potassium phosphate, potassium sulfate, cesium chloride, lithium chloride, or other salts of ammonia (e.g., $NH_4Cl$, $(NH_4)_2SO_4$), carbonates ($NaHCO_3$), citric acid ($NaH_2(C_3H_5O(COO)_3)$, $Na_2H(C_3H_5O(COO)_3)$, $Na_3H(C_3H_5O(COO)_3)$), phosphoric acid (e.g., $KH_2PO_4$, $K_2HPO_4$, $K_3PO_4$), nitrates ($KNO_3$), or any mixture of these components. Those skilled in the art appreciate that both the anion and the cation can be varied as is known to the person skilled in the art, as long as sufficient ionic strength is provided without precipitation or other undesired side-effects.

The term "anti-EGFR antibody" is meant to refer to an antibody that specifically binds to EGFR. An antibody "which binds" an antigen of interest, *i.e.,* EGFR, is one capable of binding that antigen with sufficient affinity such that the antibody is useful in targeting a cell expressing the antigen. Antibody 1 is an example of an anti-EGFR antibody.

The term "antibody" broadly refers to an immunoglobulin (Ig) molecule, generally comprised of four polypeptide chains, two heavy (H) chains and two light (L) chains, or any functional fragment, mutant, variant, or derivative thereof, that retains the essential target binding features of an Ig molecule.

In a full-length antibody, each heavy chain is comprised of a heavy chain variable region (abbreviated herein as HCVR or VH) and a heavy chain constant region. The heavy chain constant region is comprised of three domains, CHI, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Immunoglobulin molecules can be of any type (e.g., IgG, IgE, IgM, IgD, IgA and IgY) and class (e.g., IgG1, IgG2, IgG 3, IgG4, IgAl and IgA2) or subclass.

The term "antigen-binding portion" of an antibody (or simply "antibody portion"), as used herein, refers to one or more fragments of an antibody that retain the ability to specifically bind to an antigen (e.g., hIL-13). It has been shown that the antigen-binding function of an antibody can be performed by fragments of a full-length antibody. Such antibody embodiments may also be bispecific, dual specific, or multi-specific formats; specifically binding to two or more different antigens. Examples of binding fragments encompassed within the term "antigen-binding portion" of an antibody include (i) a Fab fragment, a monovalent fragment consisting of the VL, VH, CL and CH1 domains; (ii) a F(ab')$_2$ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546, Winter et al., PCT publication WO 90/05144 A1 herein incorporated by reference), which comprises a single variable domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R.J., et al. (1994) Structure 2:1121-1123). Such antibody binding portions are known in the art (Kontermann and Dubel eds., Antibody Engineering (2001) Springer-Verlag. New York. 790 pp. (ISBN 3-540-41354-5).

An "isolated antibody", as used herein, is intended to refer to an antibody that is substantially free of other antibodies having different antigenic specificities (e.g., an isolated antibody that specifically binds EGFR is substantially free of antibodies that specifically bind antigens other than EGFR). An isolated antibody that specifically binds EGFR may, however, have cross-reactivity to other antigens, such as EGFR molecules from other species. Moreover, an isolated antibody may be substantially free of other cellular material and/or chemicals.

The term "humanized antibody" refers to antibodies which comprise heavy and light chain variable region sequences from a non-human species (e.g., a mouse) but in which at least a portion of the VH and/or VL sequence has been altered to be more "human-like", *i.e.,* more similar to human germline variable sequences. In a particular embodiment, the term "humanized antibody" refers to an antibody or antibody variant, derivative or fragment, which specifically binds to an antigen of interest, and comprises a framework (FR) region having substantially the amino acid sequence of a human antibody, and comprises CDRs having substantially the amino acid sequence of a non-human antibody. As used herein, the term "substantially" in the context of a CDR refers to a CDR having an amino acid sequence at least 80%, preferably at least 85%, at least 90%, at least 95%, at least 98% or at least 99% identical to the amino acid sequence of a non-human antibody CDR. In one embodiment, one type of humanized antibody is a CDR-grafted antibody, in which human CDR sequences are introduced into non-human VH and VL sequences to replace the corresponding nonhuman CDR sequences.

As used herein, the term "CDR" refers to the complementarity determining region within antibody variable sequences. There are three CDRs in each of the variable regions of the heavy chain and the light chain, which are designated CDR1, CDR2 and CDR3, for each of the variable regions. The term "CDR set" as used herein refers to a group of three CDRs that occur in a single variable region capable of binding the antigen. The exact boundaries of these CDRs have been defined differently according to different systems. The system described by Kabat (Kabat et al., Sequences of Proteins of Immunological Interest (National Institutes of Health, Bethesda, Md. (1987) and (1991)) not only provides an unambiguous residue numbering system applicable to any variable region of an antibody, but also provides precise residue boundaries defining the three CDRs. These CDRs may be referred to as Kabat CDRs. Chothia and coworkers (Chothia &Lesk, J. Mol. Biol. 196:901-917 (1987) and Chothia et al., Nature 342:877-883 (1989)) found that certain sub- portions within Kabat CDRs adopt nearly identical peptide backbone conformations, despite having great diversity at the level of amino acid sequence. These sub-portions were designated as L1, L2 and L3 or HI, H2 and H3 where the "L" and the "H" designates the light chain and the heavy chains regions, respectively. These regions may be referred to as Chothia CDRs, which have boundaries that overlap with Kabat CDRs. Other boundaries defining CDRs overlapping with the Kabat CDRs have been described by Padlan (FASEB J. 9:133-139 (1995)) and MacCallum (J Mol Biol 262(5):732-45 (1996)). Still other CDR boundary definitions may not strictly follow one of the above systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although preferred embodiments use Kabat or Chothia defined CDRs.

The term "disorder" refers to any condition that would benefit from treatment with the formulations of the invention, e.g. a disorder requiring treatment with the anti-EGFR antibody in the formulation. This includes chronic and acute disorders or diseases including those pathological conditions that predispose the subject to the disorder in question.

The term "cancer" is meant to refer to or describe the physiological condition in mammals that is typically characterized by unregulated cell growth. Examples of cancer include, but are not limited to, carcinoma, lymphoma, blastoma, sarcoma, and leukemia or lymphoid malignancies. More particular examples of such cancers include glioblastoma, non-small cell lung cancer, lung cancer, colon cancer, head and neck cancer, breast cancer, squamous cell tumors, anal cancer, skin cancer, and vulvar cancer. In one embodiment, the compositions of the invention are administered to a patient having a tumor(s) containing amplifications of the EGFR gene, whereby the tumor expresses the truncated version of the EGFR de2-7. In one embodiment, the formulation of the invention comprising ADC-1 may be administered to a subject for the treatment of colorectal cancer, head and neck cancer (including, but not limited to, hypopharyngeal cancer, oropharyngeal cancer, esophageal cancer, laryngeal cancer, and oral cavity cancer), non-small cell lung cancer, pancreatic cancer, gastric cancer, and breast cancer. More particular examples of such cancers include squamous tumors (including, squamous tumors of the lung, head and neck, cervical, etc.), glioblastoma, glioma, non-small cell lung cancer, lung cancer, colon cancer, head and neck cancer, breast cancer, squamous cell tumors, anal cancer, skin cancer, and vulvar cancer. In one embodiment of the invention, the composition is used to treat a subject having a solid tumor, e.g., a solid tumor likely to over-express the Epidermal Growth Factor Receptor (EGFR), or glioblastoma multiforme.

The term "administering" as used herein is meant to refer to the delivery of a substance (e.g., an anti-EGFR antibody drug conjugate) to achieve a therapeutic objective (e.g., the treatment of an EGFR- associated disorder). Modes of administration may be parenteral, enteral and topical. Parenteral administration is usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The term "therapeutically effective amount" or "effective amount" of an antibody as used herein refers to an amount effective in the prevention or treatment or alleviation of a symptom of a disorder for the treatment of which the antibody is effective.

The term "treatment" refers to both therapeutic treatment and prophylactic or preventative measures. Those patients in need of treatment include those already with the disorder as well as those in which the disorder is to be prevented.

Various aspects of the invention are described in further detail in the following subsections.

## II. Methods for Purifying Antibody Drug Conjugates and Compositions Thereof

The invention provides a method for purifying antibody drug conjugates (ADCs), and provides an effective means for removing undesired species of ADC, *e.g.,* drug loaded species of 6 or more, from a mixture of ADCs. While the methods of the invention may be used to separate any drug loaded species, in a preferred embodiment, the methods described herein are used to separate high drug loaded ADCs from ADCs having optimal drug to antibody ratios (DARs), e.g. a DAR of 4 or less. In certain embodiments, the methods of the invention may provide numerous advantages over traditional column chromatography, including improved recovery, as fractionation and subsequent pooling of fractions may not be necessary. It should be understood that the methods and compositions described throughout may be used to purify anti-EGFR antibody-auristatin ADCs, particularly, in a certain embodiment, anti-EGFR ADCs comprising Antibody 1 either coupled via a maleimidocaproyl linker to MMAF (mc-MMAF) or coupled via a maleimidocaproyl valine-citrulline linker to MMAE (vc-MMAE).

The method of the invention generally includes adding a hydrophobic resin to an ADC mixture such that undesired ADCs, *i.e.,* higher drug loaded ADCs, bind the resin and can be selectively removed from the mixture. In certain embodiments, separation of the ADCs may be achieved by contacting an ADC mixture (e.g., a mixture comprising a drug loaded species of ADC of 4 or less and a drug loaded species of ADC of 6 or more) with a hydrophobic resin, wherein the amount of resin is sufficient to allow binding of the drug loaded species which is being removed from the ADC mixture. The resin and ADC mixture are mixed together, such that the ADC species being removed (e.g., a drug loaded species of 6 or more) binds to the resin and can be separated from the other ADC species in the ADC mixture. The amount of resin used in the method is based on a weight ratio between the species to be removed and the resin, where the amount of resin used does not allow for significant binding of the drug loaded species that is desired. Thus, the invention provides methods for reducing the average DAR of an ADC mixture from, for example, 5.5 to less than 4. Further, the purification methods described herein may be used to isolate ADCs having any desired range of drug loaded species, e.g., a drug loaded species of 4 or less, a drug loaded species of 3 or less, a drug loaded species of 2 or less, a drug loaded species of 1 or less.

The invention provides a purification method whereby a certain species of molecule(s) binds to a surface based on hydrophobic interactions between the species and a hydrophobic resin. In one embodiment, method of the invention refers to a purification process that relies upon the intermixing of a hydrophobic resin and a mixture of ADCs, wherein the amount of resin added to the mixture determines which species (e.g., ADCs with a DAR of 6 or more) will bind.

Following production and purification of an antibody from an expression system (e.g., a mammalian expression system), the antibody is reduced and coupled to a drug through a conjugation reaction. The resulting ADC mixture often contains ADCs having a range of DARs, e.g., 1 to 8. In one embodiment, the ADC mixture comprises a drug loaded species of 4 or less and a drug loaded species of 6 or more. According to the methods of the invention, the ADC mixture may be purified using a process, such as, but not limited to, a batch process, such that ADCs having a drug loaded species of 4 or less are selected and separated from ADCs having a higher drug load (e.g., ADCs having a drug loaded species of 6 or more). Notably, the purification methods described herein may be used to isolate ADCs having any desired range of DAR, *e.g.,* a DAR of 4 or less, a DAR of 3 or less, a DAR of 2 or less.

Thus, in one embodiment, the method of the invention comprises contacting an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin. In a separate embodiment, the method of the invention comprises contacting an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin, wherein the hydrophobic resin weight is 3 to 12 times the weight of the drug loaded species of 6 or more in the ADC mixture.

The method of the invention provides an effective method of separating low and high DAR ADCs. In one embodiment, the method may be performed using a batch purification method. The batch purification process generally includes adding the ADC mixture to the hydrophobic resin in a vessel, mixing, and subsequently separating the resin from the supernatant. For example, in the context of batch purification, a hydrophobic resin may be prepared in or equilibrated to the desired equilibration buffer. A slurry of the hydrophobic resin may thus be obtained. The ADC mixture may then be contacted with the slurry to adsorb the specific species of ADC(s) to be separated by the hydrophobic resin. The solution comprising the desired ADCs that do not bind to the hydrophobic resin material may then be separated from the slurry, e.g., by filtration or by allowing the slurry to settle and removing the supernatant. The resulting slurry can be subjected to one or more washing steps. In order to elute bound ADCs, the salt concentration can be decreased. In one embodiment, the process used in the invention includes no more than 50 g of hydrophobic resin.

Thus, in one embodiment of the invention, a batch method may be used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin. In a separate embodiment, a batch method is used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin, wherein the hydrophobic resin weight is 3 to 12 times the weight of the drug loaded species of 6 or more in the ADC mixture.

Alternatively, in a separate embodiment, the invention may be performed using a circulation process, whereby the resin is packed in a container and the ADC mixture is passed over the hydrophobic resin bed until the specific species of ADC(s) to be separated have been removed. The supernatant (containing the desired ADC species) is then pumped from the container and the resin bed may be subjected to washing steps.

A circulation process may be used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin. In a separate embodiment, a circulation process is used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to an auristatin, wherein the hydrophobic resin weight is 3 to 12 times the weight of the drug loaded species of 6 or more in the ADC mixture.

Alternatively, in a separate embodiment of the invention, the purification method may be performed using a flow through process, whereby resin is packed in a container, e.g., a column, and the ADC mixture is passed over the packed resin such that the desired ADC species does not substantially bind to the resin and flows through the resin, and the undesired ADC species is bound to the resin. A flow through process may be performed in a single pass mode (where the ADC species of interest are obtained as a result of a single pass through the resin of the container) or in a multi-pass mode (where the ADC species of interest are obtained as a result of multiple passes through the resin of the container). The flow through process is performed such that the weight of resin selected binds to the undesired ADC population, and the desired ADCs (e.g., DAR 2-4) flow over the resin and are collected in the flow through after one or multiple passes.

In one embodiment of the invention, a flow through process may be used to contact an ADC mixture comprising a drug loaded species of 4 or less and a drug loaded species of 6 or more with a hydrophobic resin, wherein the amount of hydrophobic resin contacted with the ADC mixture is sufficient to allow binding of the drug loaded species of 6 or more to the resin but does not allow significant binding of the drug load species of 4 or less, where the drug load species of 4 or less passes over the resin and is subsequently collected after one or multiple passes, such that the composition comprising the desired ADCs (e.g. DAR 2-4) is obtained, wherein the composition comprises less than 15% of the drug loaded species of 6 or more, and wherein the ADC comprises an antibody conjugated to an auristatin. In a separate embodiment, a flow through process is used to contact an ADC mixture comprising a drug loaded species of 4 or less and a drug loaded species of 6 or more with a hydrophobic resin by passing the ADC mixture over the resin, wherein the amount of hydrophobic resin contacted with the ADC mixture is sufficient to allow binding of the drug loaded species of 6 or more to the resin but does not allow significant binding of the drug load species of 4 or less, where the drug load species of 4 or less passes over the resin and is subsequently collected, such that the composition comprising ADCs is obtained, wherein the composition comprises less than 15% of the drug loaded species of 6 or more, and wherein the ADC comprises an antibody conjugated to an auristatin, wherein the amount of hydrophobic resin weight is 3 to 12 times the weight of the drug loaded species of 6 or more in the ADC mixture.

In one embodiment of the invention, the resin is washed with a one or more washes following the flow through process in order to further recover ADCs having the desired DAR range (found in the wash filtrate). For example, a plurality of washes having decreasing conductivity may be used to further recover ADCs having the DAR of interest. The elution material obtained from the washing of the resin may be subsequently combined with the filtrate resulting from the flow through process for improved recovery of ADCs having the DAR of interest.

The purification methods of the invention are based on the use of a hydrophobic resin to separate high vs. low drug loaded species of ADC. Hydrophobic resin comprises hydrophobic groups which interact with the hydrophobic properties of the ADCs. Hydrophobic groups on the ADC interact with hydrophobic groups within the hydrophobic resin. The more hydrophobic a protein is the stronger it will interact with the hydrophobic resin.

Hydrophobic resin normally comprises a base matrix (e.g., cross-linked agarose or synthetic copolymer material) to which hydrophobic ligands (e.g., alkyl or aryl groups) are coupled. Many hydrophobic resins are available commercially. Examples include, but are not limited to, Phenyl Sepharose™ 6 Fast Flow with low or high substitution (Pharmacia LKB Biotechnology, AB, Sweden); Phenyl Sepharose™ High Performance (Pharmacia LKB Biotechnology, AB, Sweden); Octyl Sepharose™ High Performance (Pharmacia LKB Biotechnology, AB, Sweden); Fractogel™ EMD Propyl or Fractogel™ EMD Phenyl columns (E. Merck, Germany); Macro-Prep™ Methyl or Macro-Prep™. t-Butyl Supports (Bio-Rad, California); WP HI-Propyl (C$_3$)™ (J. T. Baker, New Jersey); and Toyopearl™ ether, hexyl, phenyl or butyl (TosoHaas, PA). In one embodiment, the hydrophobic resin is a butyl hydrophobic resin. In another embodiment, the hydrophobic resin is a phenyl hydrophobic resin. In another embodiment, the hydrophobic resin is a hexyl hydrophobic resin, an octyl hydrophobic resin, or a decyl hydrophobic resin. In one embodiment, the hydrophobic resin is a methacrylic polymer having n-butyl ligands (e.g. TOYOPEARL® Butyl-600M).

The methods of the invention are based, at least in part, on the discovery that a hydrophobic resin may be used in certain amounts to selectively bind to ADCs having certain DARs. The binding between the resin and ADCs having a given DAR is dependent upon the weight of the resin relative to the weight of the ADCs which are to be removed from the ADC mixture. By varying the amount of resin load (calculated based on the dry weight) contacted to the ADC mixture relative to the specific drug load species weight in the ADC mixture, the resin will selectively bind ADCs having, for example, a DAR of 8 or more, ADCs having a DAR of 6-8, ADCs having a DAR of 5-8, etc. Thus, the selectivity of the hydrophobic resin is dependent upon the weight ratio of the resin and the weight of the ADC species to be removed by the resin. In one embodiment, the hydrophobic resin weight contacted with the ADC mixture is 3 to 12 times the weight of the drug loaded species of 6 or more in the ADC mixture. In one embodiment, the hydrophobic resin weight contacted with the ADC mixture is 4 to 8 times the weight of the drug loaded species of 6 or more in the ADC mixture. In one embodiment, the hydrophobic resin weight contacted with the ADC mixture is 5 to 10 times the weight of the drug loaded species of 6 or more in the ADC mixture. In another embodiment, the hydrophobic resin weight contacted with the ADC mixture is 5 to 7 times the weight of the drug loaded species of 6 or more in the ADC mixture. In another embodiment, the hydrophobic resin weight contacted with the ADC mixture is 5 to 6 times the weight of the drug loaded species of 6 or more in the ADC mixture. For example, as described in Table 5 in the examples below, about 5-10 weights of resin (dry) were employed to reduce the 6 and 8 loaded drug species (3.2 mg resin / 0.54 mg 6/8 load species = approx. 6), resulting in an enriched composition (enriched for ADCs having DARs of less than 6). In another example, as described in Table 7 below, a resin weight of approximately 8 to 12 times that of the 6 and 8 drug load species was proven to be effective for reducing those species from the ADC mixture. In a further example, as described in Table 7 below, a resin weight of approximately 4 times that of the 6 and 8 drug load species was proven to be effective for significantly reducing those species from the ADC mixture.

The selectivity of the resin for ADCs may be impacted by the ionic strength of the resin mixture in combination with the ratios identified herein as providing appropriate load resin:ADC weight ratios that result in selective binding of ADCs having a certain desired DAR distribution, e.g., a DAR distribution of 6-8. Generally, by decreasing the ionic strength of the resin mixture, the hydrophobic resin will be less adsorbent, whereas an increase in the ionic strength of the resin mixture will provide a more adsorbent resin. Adsorption of ADCs to hydrophobic resin is favored by high salt concentrations, but the actual concentrations may vary over a wide range depending on the nature of the ADC and the particular hydrophobic resin chosen. In general, Na, K or $NH_4$ sulfates effectively promote ligand-protein interaction in hydrophobic resin. Salts may be formulated that influence the strength of the interaction as given by the following relationship: $(NH_4)_2SO_4$>$Na_2SO_4$>NaCl>$NH_4$Cl>NaBr>NaSCN. In general, salt concentrations of between about 0.75 and about 2 M ammonium sulfate or between about 1 and 4 M NaCl are useful. In one embodiment, the resin mixture has an ionic strength of 0-2 N NaCl. The ionic strength of the ADC mixture may be adjusted prior to, concurrently with, or following the addition of the hydrophobic resin.

In one embodiment, the method of the invention uses a hydrophobic resin weight which is 6 to 12 times the weight of the drug loaded species of 6 or more in the ADC mixture where the ADC mixture has an ionic strength of 0 to 1 N NaCl, or an equivalent ionic strength thereof. In a separate embodiment, the separation method of the invention is carried out using a hydrophobic resin weight which is 3 to 6 times the weight of the drug loaded species of 6 or more in the ADC mixture, and where the ADC mixture comprises between 1 to 2 N NaCl, or an equivalent ionic strength thereof. The method may also be carried out using a hydrophobic resin weight which is 3 to 7 times the weight of the drug loaded species of 6 or more in the ADC mixture, and wherein the auristatin is monomethylauristatin E (MMAE). An additional method for separating a drug loaded species of 6 or more includes contact of an ADC mixture with a hydrophobic resin weight that is 5 to 10 times the weight of the drug loaded species of 6 or more, wherein the auristatin is monomethylauristatin F (MMAF).

Additional purification or processing steps may be performed prior to or following the methods described herein. For example, in one embodiment, the ADC mixture is obtained following an ultrafiltration /diafiltration process. In another embodiment, the purified composition of ADCs is subjected to ultrafiltration / diafiltration.

In one embodiment, the method of the invention includes contacting an ADC mixture with a hydrophobic resin, wherein the amount of hydrophobic resin contacted with the ADC mixture is sufficient to allow binding of the drug loaded species of 6 or more to the resin but does not allow significant binding of the drug loaded species of 4 or less, and removing the hydrophobic resin from the ADC mixture. The hydrophobic resin binds the higher drug loaded species, e.g., drug loaded species of 6 or more, while the lower drug loaded species, *e.g.,* the drug loaded species of 4 or less, largely remains in the supernatant. The amount of hydrophobic resin which is contacted with the ADC mixture and does not allow significant binding of the drug loaded species of 4 or less is an amount of resin which, in one embodiment, binds 35% or less drug loaded species of 4 or less. In certain embodiments, significant binding of the drug loaded species of 4 or less is defined as 30% or less, 25% or less, 20% or less, 15 % or less, 10% or less, or 5% or less. In other embodiments, significant binding of the drug loaded species is defined as 30% to 1%, 25% to 1%, 20% to 1%, 15% to 1%, 10% to 1%, or 5% to 1%.

In one embodiment, the methods of the invention may be used to obtain compositions having low levels of an undesired ADC species, e.g., a drug loaded species of 6 or more. In one embodiment, the composition of the invention has 15% or less of the drug loaded species of 6 or more. In one embodiment, the composition of the invention has 14% or less of the drug loaded species of 6 or more. In one embodiment, the composition of the invention has 13% or less of the drug loaded species of 6 or more. In one embodiment, the composition of the invention has 12% or less of the drug loaded species of 6 or more. In one embodiment, the composition of the invention has 11% or less of the drug loaded species of 6 or more. In one embodiment, the composition of the invention has 10% or less of the drug loaded species of 6 or more. In one embodiment, the composition of the invention has 9% or less of the drug loaded species of 6 or more. In one embodiment, the composition of the invention has 8% or less of the drug loaded species of 6 or more. In one embodiment, the composition of the invention has 7% or less of the drug loaded species of 6 or more. In one embodiment, the composition of the invention has 6% or less of the drug loaded species of 6 or more. In one embodiment, the composition of the invention has 5% or less of the drug loaded species of 6 or more. In one embodiment, the composition of the invention has 4% or less of the drug loaded species of 6 or more. In further embodiments, the composition has 15% to 1% of the drug loaded species of 6 or more, 10% to 1% of the drug loaded species of 6 or more, 5% to 1% of the drug loaded species of 6 or more, 10% to 0.5% of the drug loaded species of 6 or more, or 5% to 0.5% of the drug loaded species of 6 or more.

In one embodiment, the methods of the invention may be used to produce a composition comprising ADCs with an average DAR of 4. Such a composition may be obtained by contacting an ADC mixture with an amount of hydrophobic resin in an species absorption process to form a resin mixture, wherein the ADC mixture comprises drug loaded species of 4 or less and drug loaded species of 6 or more, and wherein the amount of hydrophobic resin is 5 to 10 times the weight of the drug loaded species of 6 or more in the ADC mixture, and obtaining a supernatant from the resin mixture, such that the composition comprising ADCs with an average DAR of 4 or less is produced. In one embodiment, the composition of the invention comprises ADCs with an average DAR of 3.5 or less. In one embodiment of the invention, the composition comprises ADCs with an average DAR of 3 or less. In one embodiment, the composition of the invention comprises ADCs with an average DAR of 2-4. In one embodiment of the invention, the composition comprises ADCs with an average DAR of 2.4 - 3.6. In one embodiment, the composition comprises ADCs and has an average DAR of 4 or less, or, alternatively, an average DAR of 3.5 or less, an average DAR of 3 or less, or an average DAR of 2.5 or less.

In one embodiment, the methods of the invention may be used to produce a composition comprising ADCs with an average Drug-to-Antibody Ratio (DAR) of 4 or less and comprising less than 15% undesired ADCs. The method includes contacting an ADC mixture with a hydrophobic resin, wherein the amount of hydrophobic resin contacted with the ADC mixture is sufficient to allow binding of the undesired ADCs; and removing the hydrophobic resin from the ADC mixture, such that the composition with a mean DAR of 4 or less and comprising less than 15% undesired ADCs is produced. In one embodiment, the undesired ADCs are 6 and 8 drug loaded species. In one embodiment, the amount of hydrophobic resin added to the ADC mixture is a resin weight which is 5 to 10 times the weight of the undesired ADCs in the ADC mixture. In another embodiment, the amount of hydrophobic resin added to the ADC mixture is a resin weight which is 5 to 7 times the weight of the undesired ADCs in the ADC mixture. In one embodiment, the amount of hydrophobic resin added to the ADC mixture is a resin weight which is 3 to 12 times the weight of the undesired ADCs in the ADC mixture.

The DAR of an ADC may be measured according to common methods known in the art, including, but not limited to UV/VIS spectroscopic analysis of the ADC and analytical HIC and HPLC, e.g., HPLC-MS.

## III. Antibody Drug Conjugates

The compositions and methods described herein are based, at least in part, on antibody drug conjugates (ADCs) comprising anti-EGFR antibodies, or antigen-binding portions thereof, that specifically bind to EGFR conjugated to auristatin.

In particular, the present invention pertains to methods and compositions comprising an anti-EGFR antibody drug conjugate comprising an antibody or an antigen-binding portion thereof, that recognizes an EGFR epitope which is found in tumorigenic, hyperproliferative or abnormal cells, wherein the epitope is not detectable in normal or wild-type cells. Preferably, the antibody or antigen-binding portion thereof, does not bind to or recognize normal or wild-type cells containing normal or wild-type EGFR epitope in the absence of overexpression and in the presence of normal EGFR post-translational modification.

Anti-EGFR antibodies suitable for use in accordance with the present compositions and methods are typically monoclonal and can include, for example, chimeric (e.g., having a human constant region and mouse variable region), humanized, or human antibodies; single chain antibodies; or the like. The immunoglobulin molecules can be of any type (e.g., IgG, IgE, IgM, IgD, IgA and IgY), class (e.g., IgG1, IgG2, IgG3, IgG4, IgAl and IgA2) or subclass of immunoglobulin molecule. For example, the anti-EGFR antibody used in the anti-EGFR antibody drug conjugate of the invention may be Antibody 1. The sequences and characteristics of antibody 1 are described below (see also WO 2011/041319 and US20110076232 (see, e.g., antibody sequence of Figure 55), incorporated by reference in its entirety herein). Antibody 1 targets the over-expressed form of the epidermal growth factor receptor (EGFR) present in 50% of all cancers of epithelial origin.

In a particular embodiment of the present invention, the anti-EGFR antibody used in the anti-EGFR antibody drug conjugate of the invention recognizes amplified wild-type EGFR and the de2-7 EGFR. The anti-EGFR antibody of the invention demonstrates useful specificity, in that it recognizes de2-7 EGFR and amplified EGFR, but does not recognize normal, wild-type EGFR or the unique junctional peptide which is characteristic of de2-7 EGFR. Sequences for Antibody 1 are provided below.

As described above, Antibody 1 is a humanized anti-EGFR antibody. The heavy chain variable (VH) and constant (CH) regions of Antibody 1 are shown below as SEQ ID NOS: 1 and 5, respectively. The VH region CDR1, CDR2, and CDR3 (SEQ ID NOS: 2, 3, and 4, respectively) are indicated by underlining.

Heavy Chain Variable Region amino acid sequence (SEQ ID NO: 1) (CDRs are underlined):

QVQLQESGPGLVKPSQTLSLTCTVSGYSISSDFAWNWIRQPPGKGLEWMGYISYSGNTR CDR1 (SEQ ID NO: 2)
CDR2 (SEQ ID NO: 3)
YQPSLKSRITISRDTSKNOFFLKLNSVTAADTATYYCVTAGRGFPYWGQGTLVTVSS CDR3 (SEQ ID NO: 4)

Heavy Chain Constant Region amino acid sequence (SEQ ID NO: 5):

ASTKGPSVFPLAPSSKSTSGGTAALGCLVKDYFPEPVTVSWNSGALTSGVHTFPAVL

QSSGLYSLSSVVTVPSSSLGTQTYICNVNHKPSNTKVDKKVEPKSCDKTHTCPPCPAP

ELLGGPSVFLFPPKPKDTLMISRTPEVTCVVVDVSHEDPEVKFNWYVDGVEVHNAKT

KPREEQYNSTYRVVSVLTVLHQDWLNGKEYKCKVSNKALPAPIEKTISKAKGQPREP

QVYTLPPSRDELTKNQVSLTCLVKGFYPSDIAVEWESNGQPENNYKTTPPVLDSDGS

FFLYSKLTVDKSRWQQGNVFSCSVMHEALHNHYTQKSLSLSPGK

The light chain variable (VL) and constant (CL) regions of Antibody 1 are shown below as SEQ ID NOS: 6 and 10, respectively. The VL region CDR1, CDR2, and CDR3 (SEQ ID NOS: 7, 8, and 9, respectively) are indicated by underlining.

Light Chain Variable Region amino acid sequence (SEQ ID NO: 6) (CDRs are underlined)::

DIQMTQSPSSMSVSVGDRVTITCHSSQDINSNIGWLQQKPGKSFKGLIYHGTNLDDGVPS CDR1 (SEQ ID NO: 7)
CDR2 (SEQ ID NO: 8)
RFSGSGSGTDYTLTISSLQPEDFATYYCVQYAQFPWTFGGGTKLEIKR CDR3 (SEQ ID NO: 9)

Light Chain Constant Region amino acid sequence (SEQ ID NO: 10):

TVAAPSVFIFPPSDEQLKSGTASVVCLLNNFYPREAKVQWKVDNALQSGNSQESVTE

QDSKDSTYSLSSTLTLSKADYEKHKVYACEVTHQGLSSPVTKSFNRGEC

Thus, in one embodiment, the anti-EGFR antibody (used in the ADCs described herein) comprises a light chain variable region comprising a Complementarity Determining Region 1 (CDR1), CDR2, and CDR3 domain comprising the amino acid sequence as set forth in SEQ ID NO: 7, SEQ ID NO: 8, and SEQ ID NO: 9, respectively, and comprises a heavy chain variable region comprising a CDR1, CDR2, and CDR3 domain comprising the amino acid sequence as set forth in SEQ ID NO: 2, SEQ ID NO: 3, and SEQ ID NO: 4.

In one embodiment, the invention provides a formulation comprising an ADC comprising an anti-EGFR antibody (conjugated to an auristatin) having a light chain variable region comprising CDRs as described in the amino acid sequence of SEQ ID NO: 6, a heavy chain variable region comprising CDRs as described in the amino acid sequence of SEQ ID NO: 1.

In one embodiment, the anti-EGFR antibody (used in the ADCs described herein) comprises a light chain variable region comprising the amino acid sequence set forth in SEQ ID NO: 6 and a heavy chain variable region comprising the amino acid sequence set forth in SEQ ID NO: 1.

In a preferred embodiment, the ADC used in the methods and compositions of the invention comprises an anti-EGFR antibody, e.g., Antibody 1, and an auristatin. In one embodiment, the auristatin is monomethylauristatin E (MMAE), e.g., vc-MMAE. In one embodiment, the auristatin is or monomethylauristatin F (MMAF), .e.g, mc-MMAF.

Alternatively, other auristatin-based ADCs may be made in accordance with the methods of the invention, Examples of antibodies that may be used in making auristatin-ADCs include chimeric antibodies, human antibodies, and humanized antibodies.

Antibodies, including anti-EGFR antibodies, that may be used make ADCs, including anti-EGFR antibody drug conjugates, can be generated by any suitable method known in the art. For example, monoclonal antibodies can be prepared using a wide variety of techniques including, e.g., the use of hybridoma, recombinant, and phage display technologies, or a combination thereof. Hybridoma techniques are generally discussed in, for example, Harlow et al., Antibodies: A Laboratory Manual, (Cold Spring Harbor Laboratory Press, 2nd ed., 1988); and Hammerling, et al., In Monoclonal Antibodies and T-Cell Hybridomas, pp. 563-681 (Elsevier, N.Y., 1981). Examples of phage display methods that can be used to make the anti-CD70 antibodies include, e.g., those disclosed in Brinkman et al., 1995, J Immunol Methods 182:41-50; Ames et al., 1995, J Immunol Methods 184:177-186; Kettleborough et al., 1994, Eur J Immunol 24:952-958; Persic et al., 1997, Gene 187:9-18; Burton et al., 1994, Advances in Immunology 57:191-280; PCT Application No. PCT/GB91/01 134; PCT Publications WO 90/02809; WO 91/10737; WO 92/01047; WO 92/18619; WO 93/11236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108 (the disclosures of which are incorporated by reference herein).

Mammalian host cells for expressing the recombinant antibodies of the invention include Chinese Hamster Ovary (CHO cells) (including dhfr-CHO cells, described in Urlaub and Chasin (1980) Proc. Natl. Acad. Sci. USA 77:4216-4220, used with a DHFR selectable marker, e.g., as described in Kaufman and Sharp (1982) J. Mol. Biol. 159:601-621) and DG44 or DUXB11 cells (Urlaub et al. (1986) Som. Cell Molec. Genet. 12:555; Haynes et al. (1983) Nuc. Acid. Res. 11:687-706; Lau et al. (1984) Mol. Cell. Biol. 4:1469-1475), NS0 myeloma cells, monkey kidney line (e.g., CVI and COS, such as a COS 7 cell), SP2 cells, human embryonic kidney (HEK) cells, such as a HEK-293 cell, Chinese hamster fibroblast (e.g., R1610), human cervical carcinoma (e.g., HELA), murine fibroblast (e.g., BALBc/3T3), murine myeloma (P3x63-Ag3.653; NS0; SP2/O), hamster kidney line (e.g., HAK), murine L cell (e.g., L-929), human lymphocyte (e.g., RAJI), human kidney (e.g., 293 and 293T). Host cell lines are typically commercially available (e.g., from BD Biosciences, Lexington, Ky.; Promega, Madison, Wis.; Life Technologies, Gaithersburg, Md.) or from the American Type Culture Collection (ATCC, Manassas, Va.).

When recombinant expression vectors encoding the antibody are introduced into mammalian host cells, the antibodies are produced by culturing the host cells for a period of time sufficient to allow for expression of the antibodies in the host cells or secretion of the antibodies into the culture medium in which the host cells are grown. Antibodies can be recovered from the culture medium using standard protein purification methods.

In an exemplary system for recombinant expression of antibodies, a recombinant expression vector encoding both the antibody heavy chain and the antibody light chain is introduced into dhfr-CHO cells by calcium phosphate-mediated transfection. Within the recombinant expression vector, the antibody heavy and light chain cDNAs are each operatively linked to CMV enhancer/AdMLP promoter regulatory elements to drive high levels of transcription of the cDNAs. The recombinant expression vector also carries cDNA encoding DHFR, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium. Still further, the invention provides a method of synthesizing an antibody by culturing a host cell of the invention in a suitable culture medium until the antibody is synthesized. The method can further comprise isolating the antibody from the culture medium.

In a preferred embodiment, the anti-EGFR antibody, or an antigen-binding portion thereof, is conjugated to an auristatin (one or more). Auristatins have been shown to interfere with microtubule dynamics, GTP hydrolysis, and/or nuclear and cellular division and have anticancer and/or antifungal activity. Auristatins represent a group of dolastatin analogs that have generally been shown to possess anticancer activity by interfering with microtubule dynamics and GTP hydrolysis, thereby inhibiting cellular division. For example, Auristatin E (U.S. Patent No. 5,635,483, incorporated by reference herein) is a synthetic analogue of the marine natural product dolastatin 10, a compound that inhibits tubulin polymerization by binding to the same site on tubulin as the anticancer drug vincristine (G. R. Pettit, Prog. Chem. Org. Nat. Prod, 70: 1-79 (1997)). Dolastatin 10, auristatin PE, and auristatin E are linear peptides having four amino acids, three of which are unique to the dolastatin class of compounds. Exemplary embodiments of the auristatin subclass of mitotic inhibitors include, but are not limited to, monomethyl auristatin D (MMAD or auristatin D derivative), monomethyl auristatin E (MMAE or auristatin E derivative), monomethyl auristatin F (MMAF or an MMAF derivative), auristatin F phenylenediamine (AFP), auristatin EB (AEB), auristatin EFP (AEFP), and 5-benzoylvaleric acid-AE ester (AEVB). The synthesis and structure of auristatin derivatives are described in U.S. Patent Application Publication Nos. 2003-0083263, 2005-0238649 and 2005-0009751; International Patent Publication No. WO 04/010957, International Patent Publication No. WO 02/088172, and U.S. Pat. Nos. 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414, each of which is incorporated by reference herein.

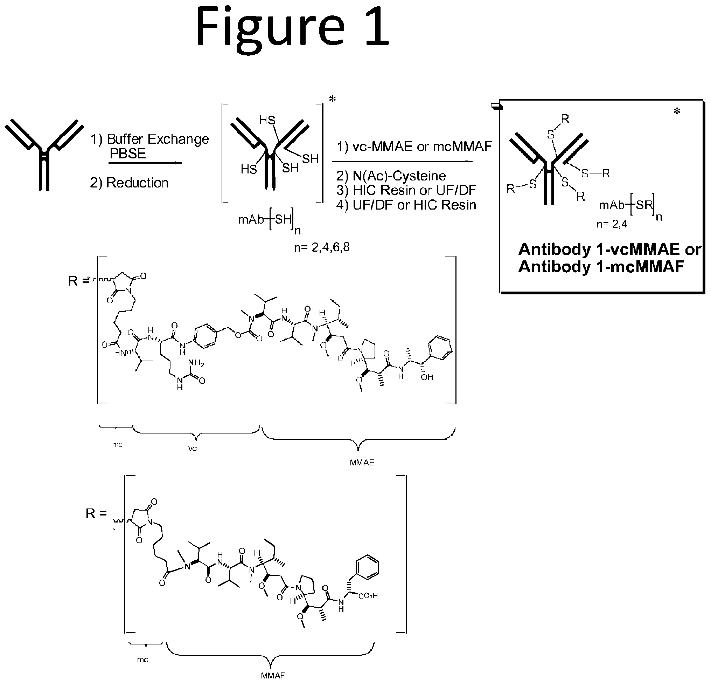

In one embodiment, the anti-EGFR antibody of the invention is conjugated to at least one MMAF (monomethylauristatin F). Monomethyl auristatin F (MMAF) inhibits cell division by blocking the polymerization of tubulin. It has a charged C-terminal phenylalanine residue that attenuates its cytotoxic activity compared to its uncharged counterpart MMAE. Because of its super toxicity, it cannot be used as a drug itself, but can be linked to a monoclonal antibody (mAb) that directs it to the cancer cells. In one embodiment, the linker to the anti-EGFR antibody is stable in extracellular fluid, but is cleaved by cathepsin once the conjugate has entered a tumor cell, thus activating the anti-mitotic mechanism. In one embodiment, Antibody 1 is conjugated to MMAF using a noncleavable maleimidocaproyl (mc) linkage. The structure of MMAF is provided in Figure 1.

In one embodiment, the anti-EGFR antibody of the invention is conjugated to at least one MMAE (mono-methyl auristatin E). Monomethyl auristatin E (MMAE, vedotin) inhibits cell division by blocking the polymerisation of tubulin. Because of its super toxicity, it also cannot be used as a drug itself. In recent cancer therapy developments, it is linked to a monoclonal antibody (mAb) that recognizes a specific marker expression in cancer cells and directs MMAE to the cancer cells. In one embodiment, the linker linking MMAE to the anti-EGFR antibody is stable in extracellular fluid (i.e., the medium or environment that is external to cells), but is cleaved by cathepsin once the ADC has bound to the specific cancer cell antigen and entered the cancer cell, thus releasing the toxic MMAE and activating the potent anti-mitotic mechanism. The structure of MMAE is provided in Figure 1.

Techniques for conjugating therapeutic agents to proteins, and in particular to antibodies, are well-known. (See, e.g., Arnon et al., "Monoclonal Antibodies For Immunotargeting Of Drugs In Cancer Therapy," in Monoclonal Antibodies And Cancer Therapy (Reisfeld et al. eds., Alan R. Liss, Inc., 1985); Hellstrom et al., "Antibodies For Drug Delivery," in Controlled Drug Delivery (Robinson et al. eds., Marcel Dekker, Inc., 2nd ed. 1987); Thorpe, "Antibody Carriers Of Cytotoxic Agents In Cancer Therapy: A Review," in Monoclonal Antibodies '84: Biological And Clinical Applications (Pinchera et al. eds., 1985); "Analysis, Results, and Future Prospective of the Therapeutic Use of Radiolabeled Antibody In Cancer Therapy," in Monoclonal Antibodies For Cancer Detection And Therapy (Baldwin et al. eds., Academic Press, 1985); and Thorpe et al., 1982, Immunol. Rev. 62:119-58. See also, e.g., PCT publication WO 89/12624.)

In one embodiment, the anti-EGFR-ADC comprises a linker region between the cytotoxic drug and the antibody. For example, such linker, spacer and/or stretcher compounds include, but are not limited to, the following: amino benzoic acid spacers (see, for example and without limitation, U.S. Patent Nos. 7,091,186 and 7,553,816, each of which is hereby incorporated by reference in its entirety); maleimidocaproyl; p-aminobenzylcarbamoyl (PAB); lysosomal enzyme-cleavable linkers (see, for example and without limitation, U.S. Patent No. 6,214,345, hereby incorporated by reference in its entirety); maleimidocaproyl-polyethylene 20 glycol (MC(PEG)6-OH); N-methyl-valine citrulline; N-succinimidyl 4-(N-maleimidomethyl) cyclohexane-1-carboxylate (SMCC) (see, for example and without limitation, Yoshitake et al. (1979) Eur. J. Biochem., 101, 395-399, hereby incorporated by reference in its entirety); N- succinimidyl 4-(2-pyridyldithio) butanoate (SPDB) (see, for example and without limitation, U.S. Patent No. 4,563,304, hereby incorporated by reference 25 in its entirety); N-Succinimidyl 4-(2-pyridylthio)pentanoate (SPP); valine-citrulline (vc); and other linker, spacer, and/or stretcher compounds (see, for example and without limitation, U.S. Patent Nos. 7,090,843, 7,223,837, and 7,659,241, and U.S. Patent Publication Nos. 2004/0018194, 2004/0121940, 2006/0116422, 2007/0258987, 2008/0213289, 2008/0241128, 2008/0311136, 2008/0317747, and 2009/0010945, each of which is hereby incorporated by reference in its entirety). Generally speaking, techniques for attaching and/or conjugating the agents set forth above, as well as other agents, to specific binding members of the present invention, particularly antibodies and fragments thereof, are known in the art. See, for example and without limitation, Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", In Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982), each of which is hereby incorporated by reference in its entirety.

A number of different reactions are available for covalent attachment of drugs to antibodies. This is often accomplished by reaction of the amino acid residues of the antibody molecule, including the amine groups of lysine, the free carboxylic acid groups of glutamic and aspartic acid, the sulfhydryl groups of cysteine and the various moieties of the aromatic amino acids. One of the most commonly used non-specific methods of covalent attachment is the carbodiimide reaction to link a carboxy (or amino) group of a compound to amino (or carboxy) groups of the antibody. Additionally, bifunctional agents such as dialdehydes or imidoesters have been used to link the amino group of a compound to amino groups of the antibody molecule. Also available for attachment of drugs to antibodies is the Schiff base reaction. This method involves the periodate oxidation of a drug that contains glycol or hydroxy groups, thus forming an aldehyde which is then reacted with the antibody molecule. Attachment occurs via formation of a Schiff base with amino groups of the antibody molecule. Isothiocyanates can also be used as coupling agents for covalently attaching drugs to antibodies. Other techniques are known to the skilled artisan and within the scope of the present invention. Non-limiting examples of such techniques are described in, e.g., U.S. Pat. Nos. 5,665,358; 5,643,573; and 5,556,623, which are incorporated by reference in their entireties herein.

In certain embodiments, an intermediate, which is the precursor of the linker, is reacted with the drug under appropriate conditions. In certain embodiments, reactive groups are used on the drug and/or the intermediate. The product of the reaction between the drug and the intermediate, or the derivatized drug, is subsequently reacted with the anti-EGFR antibody under appropriate conditions.

Other examples of conjugation methods are described in US Patent No. 7,837,980 (Seattle Genetics), Carter and Senter (2008) Cancer J, 14(3):154, as well as U.S. Published Application Nos. 2004-0157782 A1 and 2005-0238649 and International Patent Application No, PCT/US04/038392.

In one embodiment of the invention, the anti-EGFR antibody, or antigen-binding portion thereof, is conjugated to an auristatin which is MMAF. In one embodiment, the anti-EGFR ADC is Antibody 1-mc-MMAF. Antibody 1-mc-MMAF comprises antibody 1 (described above and in SEQ ID NOs: 1 to 10) covalently linked to one or more molecules of monomethyl auristatin F (MMAF) (see Figure 1 for structure). To generate Antibody 1-mc-MMAF, the interchain disulfide bonds of antibody 1 are reduced to sulfhydryl groups. MMAF is then coupled to the antibody via these sulfhydryl groups. Antibody 1-mc-MMAF is generated using a noncleavable linker, *i.e.,* a noncleavable maleimidocaproyl (mc) linkage, as shown in Figure 1.

In one embodiment of the invention, the anti-EGFR antibody, or antigen-binding portion thereof, is conjugated to an auristatin which is MMAE. In one embodiment, the anti-EGFR ADC comprises Antibody 1-vc-MMAE. Antibody 1-vc-MMAE comprises Antibody 1 (described above and in SEQ ID NOs: 1 to 10) covalently linked to one or more molecules of monomethyl auristatin E (MMAE) (see Figure 1 for structure). To generate Antibody 1-vc-MMAE, the interchain disulfide bonds of Antibody 1 are reduced to sulfhydryl groups. vcMMAE is then coupled to the antibody via these sulfhydryl groups. Antibody 1-vc-MMAE is generated using a valine citrulline linker (vc), as shown in Figure 1, thus forming Antibody 1-vc-MMAE

## IV. Composition Uses

In accordance with the present methods, a composition comprising anti-EGFR ADCs having a desired average DAR is administered to a subject having (or at risk of having) a disorder requiring treatment with the anti-EGFR antibody. The formulation comprising the anti-EGFR ADC may be administered either alone or in combination with other compositions in the prevention or treatment of the disorder requiring treatment with the anti-EGFR antibody.

As used herein, the term "a disorder in which EGFR activity is detrimental" is intended to include diseases and other disorders in which the presence of EGFR in a subject suffering from the disorder has been shown to be or is suspected of being either responsible for the pathophysiology of the disorder or a factor that contributes to a worsening of the disorder. Accordingly, a disorder in which EGFR activity is detrimental is a disorder in which inhibition of EGFR activity is expected to alleviate the symptoms and/or progression of the disorder. Such disorders may be evidenced, for example, by an increase in the activity of EGFR or an increase in the amount of EGFR present in a biological sample from a subject suffering from the disorder (e.g., an increase in the concentration of EGFR in a tissue sample, in serum, plasma, synovial fluid, etc. of the subject), which can be detected, for example, using an anti-EGFR antibody.

Thus, the ADC compositions of the invention having an average DAR of, for example, 2-4, may be used to treat cancer. Examples of cancer that may be treated include, but are not limited to, glioblastoma, non-small cell lung cancer, squamous non-small cell lung cancer (NSCLC), lung cancer, colon cancer, head and neck cancer, breast cancer, squamous cell tumors, anal cancer, skin cancer, and vulvar cancer. ADC compositions having a desired average DAR may also be used to treat a subject having a solid tumor likely to over-express the Epidermal Growth Factor Receptor (EGFR) or glioblastoma multiforme.

In one embodiment, the purified ADC compositions of the invention having an average DAR of, for example, 2-4, are used to treat colorectal cancer, head and neck cancer (including, but not limited to, hypopharyngeal cancer, oropharyngeal cancer, esophageal cancer, laryngeal cancer, and oral cavity cancer), non-small cell lung cancer, squamous non-small cell lung cancer (NSCLC), pancreatic cancer, gastric cancer, solid tumors, a solid tumor likely to over-express the Epidermal Growth Factor Receptor (EGFR), glioblastoma multiforme, and breast cancer. More particular examples of such cancers include squamous tumors (including, squamous tumors of the lung, head and neck, cervical, etc.), glioblastoma, glioma, lung cancer, colon cancer, head and neck cancer, breast cancer, squamous cell tumors, anal cancer, skin cancer, and vulvar cancer.

The unique specificity of the compositions comprising anti-EGFR ADCs provides diagnostic and therapeutic uses to identify, characterize, target and treat, reduce or eliminate a number of tumorigenic cell types and tumor types, for example, but not limited to, glioblastoma, non-small cell lung cancer, lung cancer, colon cancer, head and neck cancer, breast cancer, squamous cell tumors, anal cancer, skin cancer, a solid tumor likely to over-express the Epidermal Growth Factor Receptor (EGFR), glioblastoma multiforme, and vulvar cancer, without the problems associated with normal tissue uptake that may be seen with previously known EGFR antibodies. Thus, cells overexpressing EGFR (e.g. by amplification or expression of a mutant or variant EGFR), and in particular embodiments, those demonstrating aberrant post-translational modification may be recognized, isolated, characterized, targeted and treated or eliminated utilizing the antibody(ies) or fragments thereof of the present invention. The compositions of the invention may be used to treat EGFR positive tumors. Methods for detecting expression of EGFR in a tumor are known in the art, e.g., the EGFR pharmDx™ Kit (Dako). In contrast, an "EGFR negative tumor" is defined as a tumor having an absence of EGFR membrane staining above background in a tumor sample as determined by immunohistochemical techniques.

In one aspect of the invention, there is provided a method for treating a subject comprising administering a therapeutically effective amount of an anti-EGFR ADC in any of the compositions as described herein, wherein the subject has a disorder requiring treatment with the anti-EGFR antibody in the composition (e.g. a tumor, a cancerous condition, a precancerous condition, and any condition related to or resulting from hyperproliferative cell growth).

A composition comprising anti-EGFR ADCs can thus specifically categorize the nature of EGFR tumors or tumorigenic cells, by staining or otherwise recognizing those tumors or cells wherein EGFR overexpression, particularly amplification and/or EGFR mutation, particularly de2-7EGFR, is present.

Therefore, in a further aspect of the invention, there is provided a method of treatment of a tumor, a cancerous condition, a precancerous condition, and any condition related to or resulting from hyperproliferative cell growth comprising administration of a composition of the invention comprising an anti-EGFR ADC comprising Antibody 1.

Various delivery systems are known and can be used to administer the anti-EGFR ADC composition of the invention. Methods of introduction include but are not limited to intradermal, intramuscular, intraperitoneal, intravenous, subcutaneous, intranasal, epidural, and oral routes. The ADCs can be administered, for example by infusion or bolus injection, by absorption through epithelial or mucocutaneous linings (e.g., oral mucosa, rectal and intestinal mucosa, and the like) and can be administered together with other biologically active agents such as chemotherapeutic agents. Administration can be systemic or local. In one embodiment, the formulation of the invention is delivered to a subject intravenously. In another embodiment, the formulation of the invention is delivered to a subject subcutaneously. In one embodiment, the subject administers the formulation to himself/herself (self-administration).

The amount of the ADC that is effective in the treatment or prevention of a disorder requiring treatment with the anti-EGFR antibody in the formulation, e.g. a cancer, can be determined by standard clinical techniques. In addition, in vitro assays may optionally be employed to help identify optimal dosage ranges. The precise dose to be employed in the formulation will also depend on the route of administration, and the stage of immunological disorder or EGFR-expressing cancer, and should be decided according to the judgment of the practitioner and each patient's circumstances. In one embodiment, a therapeutically effective amount of the formulation is administered. The term "therapeutically effective amount" or "effective amount" of an antibody as used herein refers to an amount effective in the prevention or treatment or alleviation of a symptom of a disorder for the treatment of which the antibody is effective. An example of a therapeutically effective amount of the formulation is an amount sufficient to inhibit detrimental EGFR activity or treat a disorder in which EGFR activity is detrimental.

A dose of an anti-EGFR ADC can be administered, for example, daily, once per week (weekly), twice per week, thrice per week, four times per week, five times per week, biweekly, every three weeks, monthly, every four weeks, two weeks on / one week off or otherwise as needed.

Thus, pharmaceutical compositions according to the present invention, and for use in accordance with the present invention, may comprise, in addition to the active ingredient (ADC), a pharmaceutically acceptable excipient, carrier, buffer, stabilizer or other materials well known to those skilled in the art. Such materials should be non-toxic and should not interfere with the efficacy of the active ingredient. The precise nature of the carrier or other material may depend on the route of administration, which may be oral, or by injection, e.g. intravenous. In one embodiment, the pharmaceutical composition comprises an ADC (e.g., an anti-EGFR antibody such as Antibody 1 conjugated to a MMAE or MMAF), and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition.

In certain embodiments, the anti-EGFR ADC can be co-administered to a subject with one or more additional therapeutic agents to treat cancer. The term "co-administered" means the administration of two or more different pharmaceutical agents or treatments (e.g., radiation treatment) that are administered to a subject by combination in the same pharmaceutical composition or separate pharmaceutical compositions. Thus coadministration involves administration at the same time of a single pharmaceutical composition comprising two or more pharmaceutical agents or administration of two or more different compositions to the same subject at the same or different times.

Anti-EGFR ADCs can be co-administered with a therapeutically effective amount of one or more agents to treat a cancer, where examples of the agents include, such as radiation, alkylating agents, angiogenesis inhibitors, antibodies, antimetabolites, antimitotics, antiproliferatives, antivirals, aurora kinase inhibitors, apoptosis promoters (for example, Bcl-xL, Bcl-w and Bfl-1) inhibitors, activators of death receptor pathway, Bcr-Abl kinase inhibitors, BiTE (Bi-Specific T cell Engager) antibodies, antibody drug conjugates, biologic response modifiers, cyclin-dependent kinase inhibitors, cell cycle inhibitors, cyclooxygenase-2 inhibitors, DVDs (dual variable domain antibodies), leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNA's, mitogen-activated extracellular signal-regulated kinase inhibitors, multivalent binding proteins, non-steroidal anti-inflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (bromodomain) inhibitors, proteosome inhibitors, purine analogs, pyrimidine analogs, receptor tyrosine kinase inhibitors, etinoids/deltoids plant alkaloids, small inhibitory ribonucleic acids (siRNAs), topoisomerase inhibitors, temozolomide, ubiquitin ligase inhibitors, and the like, and in combination with one or more of these agents .

Anti-EGFR ADCs can be co-administered with a therapeutically effective amount of one or more agents to treat a cancer, including BiTE antibodies, which are bi-specific antibodies that direct T-cells to attack cancer cells by simultaneously binding the two cells. The T-cell then attacks the target cancer cell. Examples of BiTE antibodies include adecatumumab (Micromet MT201), blinatumomab (Micromet MT103) and the like. Without being limited by theory, one of the mechanisms by which T-cells elicit apoptosis of the target cancer cell is by exocytosis of cytolytic granule components, which include perforin and granzyme B. In this regard, Bcl-2 has been shown to attenuate the induction of apoptosis by both perforin and granzyme B. These data suggest that inhibition of Bcl-2 could enhance the cytotoxic effects elicited by T-cells when targeted to cancer cells (V.R. Sutton, D.L. Vaux and J.A. Trapani, J. of Immunology 1997, 158 (12), 5783).

Anti-EGFR ADCs can be co-administered with a therapeutically effective amount of one or more agents to treat a cancer, including siRNA. SiRNAs are molecules having endogenous RNA bases or chemically modified nucleotides. The modifications do not abolish cellular activity, but rather impart increased stability and/or increased cellular potency. Examples of chemical modifications include phosphorothioate groups, 2'-deoxynucleotide, 2'-OCH$_3$-containing ribonucleotides, 2'-F-ribonucleotides, 2'-methoxyethyl ribonucleotides, combinations thereof and the like. The siRNA can have varying lengths (e.g., 10-200 bps) and structures (e.g., hairpins, single/double strands, bulges, nicks/gaps, mismatches) and are processed in cells to provide active gene silencing. A double-stranded siRNA (dsRNA) can have the same number of nucleotides on each strand (blunt ends) or asymmetric ends (overhangs). The overhang of 1-2 nucleotides can be present on the sense and/or the antisense strand, as well as present on the 5'- and/ or the 3'-ends of a given strand.

Anti-EGFR ADCs can be co-administered with a therapeutically effective amount of one or more agents to treat a cancer, including DVDs and other multivalent binding proteins. Multivalent binding proteins are binding proteins comprising two or more antigen binding sites. Multivalent binding proteins are engineered to have the three or more antigen binding sites and are generally not naturally occurring antibodies. The term "multispecific binding protein" means a binding protein capable of binding two or more related or unrelated targets. Dual variable domain (DVD) binding proteins are tetravalent or multivalent binding proteins binding proteins comprising two or more antigen binding sites. Such DVDs may be monospecific (i.e., capable of binding one antigen) or multispecific (i.e., capable of binding two or more antigens). DVD binding proteins comprising two heavy chain DVD polypeptides and two light chain DVD polypeptides are referred to as DVD Ig's. Each half of a DVD Ig comprises a heavy chain DVD polypeptide, a light chain DVD polypeptide, and two antigen binding sites. Each binding site comprises a heavy chain variable domain and a light chain variable domain with a total of 6 CDRs involved in antigen binding per antigen binding site. Multispecific DVDs include DVD binding proteins that bind DLL4 and VEGF, or C-met and EGFR or ErbB3 and EGFR.

Anti-EGFR ADCs can be co-administered with a therapeutically effective amount of one or more agents to treat a cancer, including alkylating agents. Alkylating agents include altretamine, AMD-473, AP-5280, apaziquone, bendamustine, brostallicin, busulfan, carboquone, carmustine (BCNU), chlorambucil, CLORETAZINE® (laromustine, VNP 40101M), cyclophosphamide, decarbazine, estramustine, fotemustine, glufosfamide, ifosfamide, KW-2170, lomustine (CCNU), mafosfamide, melphalan, mitobronitol, mitolactol, nimustine, nitrogen mustard N-oxide, ranimustine, temozolomide, thiotepa, TREANDA® (bendamustine), treosulfan, rofosfamide and the like.

Anti-EGFR ADCs can be co-administered with a therapeutically effective amount of one or more agents to treat a cancer, including angiogenesis inhibitors. Angiogenesis inhibitors include endothelial-specific receptor tyrosine kinase (Tie-2) inhibitors, epidermal growth factor receptor (EGFR) inhibitors, insulin growth factor-2 receptor (IGFR-2) inhibitors, matrix metalloproteinase-2 (MMP-2) inhibitors, matrix metalloproteinase-9 (MMP-9) inhibitors, platelet-derived growth factor receptor (PDGFR) inhibitors, thrombospondin analogs, vascular endothelial growth factor receptor tyrosine kinase (VEGFR) inhibitors and the like.

Anti-EGFR ADCs can be co-administered with a therapeutically effective amount of one or more agents to treat a cancer, including antimetabolites. Antimetabolites include ALIMTA® (pemetrexed disodium, LY231514, MTA), 5-azacitidine, XELODA® (capecitabine), carmofur, LEUSTAT® (cladribine), clofarabine, cytarabine, cytarabine ocfosfate, cytosine arabinoside, decitabine, deferoxamine, doxifluridine, eflornithine, EICAR (5-ethynyl-1-β-D-ribofuranosylimidazole-4-carboxamide), enocitabine, ethnylcytidine, fludarabine, 5-fluorouracil alone or in combination with leucovorin, GEMZAR® (gemcitabine), hydroxyurea, ALKERAN®(melphalan), mercaptopurine, 6-mercaptopurine riboside, methotrexate, mycophenolic acid, nelarabine, nolatrexed, ocfosate, pelitrexol, pentostatin, raltitrexed, Ribavirin, triapine, trimetrexate, S-1, tiazofurin, tegafur, TS-1, vidarabine, UFT and the like.

Anti-EGFR ADCs can be co-administered with a therapeutically effective amount of one or more agents to treat a cancer, including antivirals. Antivirals include ritonavir, hydroxychloroquine and the like.

Anti-EGFR ADCs can be co-administered with a therapeutically effective amount of one or more agents to treat a cancer, including aurora kinase inhibitors. Aurora kinase inhibitors include ABT-348, AZD-1152, MLN-8054, VX-680, Aurora A-specific kinase inhibitors, Aurora B-specific kinase inhibitors and pan-Aurora kinase inhibitors and the like.

Anti-EGFR ADCs can be co-administered with a therapeutically effective amount of one or more agents to treat a cancer, including Bcl-2 protein inhibitors. Bcl-2 protein inhibitors include AT-101 ((-)gossypol), GENASENSE® (G3139 or oblimersen (Bcl-2-targeting antisense oligonucleotide)), IPI-194, IPI-565, N-(4-(4-((4'-chloro(1,1'-biphenyl)-2-yl)methyl)piperazin-1-yl)benzoyl)-4-(((1R)-3-(dimethylamino)-1-((phenylsulfanyl)methyl)propyl)amino)-3-nitrobenzenesulfonamide (ABT-737), N-(4-(4-((2-(4-chlorophenyl)-5,5-dimethyl-1-cyclohex-1-en-1-yl)methyl)piperazin-1-yl)benzoyl)-4-(((1R)-3-(morpholin-4-yl)-1-((phenylsulfanyl)methyl)propyl)amino)-3-((trifluoromethyl)sulfonyl)benzenesulfonamide (ABT-263), GX-070 (obatoclax), ABT-199, and the like.

Anti-EGFR ADCs can be co-administered with a therapeutically effective amount of one or more agents to treat a cancer, including Bcr-Abl kinase inhibitors, such as DASATINIB® (BMS-354825), GLEEVEC® (imatinib) and the like.

Anti-EGFR ADCs can be co-administered with a therapeutically effective amount of one or more agents to treat a cancer, including CDK inhibitors. CDK inhibitors include AZD-5438, BMI-1040, BMS-032, BMS-387, CVT-2584, flavopyridol, GPC-286199, MCS-5A, PD0332991, PHA-690509, seliciclib (CYC-202, R-roscovitine), ZK-304709 and the like.

Anti-EGFR ADCs can be co-administered with a therapeutically effective amount of one or more agents to treat a cancer, including COX-2 inhibitors. COX-2 inhibitors include ABT-963, ARCOXIA® (etoricoxib), BEXTRA® (valdecoxib), BMS347070, CELEBREX® (celecoxib), COX-189 (lumiracoxib), CT-3, DERAMAXX® (deracoxib), JTE-522, 4-methyl-2-(3,4-dimethylphenyl)-1-(4-sulfamoylphenyl-1H-pyrrole), MK-663 (etoricoxib), NS-398, parecoxib, RS-57067, SC-58125, SD-8381, SVT-2016, S-2474, T-614, VIOXX® (rofecoxib) and the like.

Anti-EGFR ADCs can be co-administered with a therapeutically effective amount of one or more agents to treat a cancer, including other EGFR inhibitors. EGFR inhibitors include EGFR antibodies, ABX-EGF, anti-EGFR immoliposomes, EGF-vaccine, EMD-7200, ERBITUX® (cetuximab), HR3, IgA antibodies, IRESSA® (gefitinib), TARCEVA® (erlotinib or OSI-774), TP-38, EGFR fusion protein, TYKERB® (lapatinib) and the like.

Anti-EGFR ADCs can be co-administered with a therapeutically effective amount of one or more agents to treat a cancer, including HER2 inhibitors. ErbB2 receptor inhibitors include CP-724-714, CI-1033 (canertinib), HERCEPTIN® (trastuzumab), TYKERB® (lapatinib), OMNITARG® (2C4, petuzumab), TAK-165, GW-572016 (ionafarnib), GW-282974, EKB-569, PI-166, dHER2 (HER2 vaccine), APC-8024 (HER-2 vaccine), anti-HER/2neu bispecific antibody, B7.her2IgG3, AS HER2 trifunctional bispecfic antibodies, mAB AR-209, mAB 2B-1 and the like.

Anti-EGFR ADCs can be co-administered with a therapeutically effective amount of one or more agents to treat a cancer, including histone deacetylase inhibitors, such as depsipeptide, LAQ-824, MS-275, trapoxin, suberoylanilide hydroxamic acid (SAHA), TSA, valproic acid and the like.

Anti-EGFR ADCs can be co-administered with a therapeutically effective amount of one or more agents to treat a cancer, including HSP-90 inhibitors include 17-AAG-nab, 17-AAG, CNF-101, CNF-1010, CNF-2024, 17-DMAG, geldanamycin, IPI-504, KOS-953, MYCOGRAB® (human recombinant antibody to HSP-90), NCS-683664, PU24FC1, PU-3, radicicol, SNX-2112, STA-9090 VER49009 and the like.

Anti-EGFR ADCs can be co-administered with a therapeutically effective amount of one or more agents to treat a cancer, including inhibitors of inhibitors of apoptosis proteins, such as HGS1029, GDC-0145, GDC-0152, LCL-161, LBW-242 and the like.

Anti-EGFR ADCs can be co-administered with a therapeutically effective amount of one or more agents to treat a cancer, including other ADCs, such as anti-CD22-MC-MMAF, anti-CD22-MC-MMAE, anti-CD22-MCC-DM1, CR-011-vcMMAE, PSMA-ADC, MEDI-547, SGN-19Am SGN-35, SGN-75 and the like.

Anti-EGFR ADCs can be co-administered with a therapeutically effective amount of one or more agents to treat a cancer, including activators of death receptor pathway, such as TRAIL, antibodies or other agents that target TRAIL or death receptors (e.g., DR4 and DR5) such as Apomab, conatumumab, ETR2-ST01, GDC0145, (lexatumumab), HGS-1029, LBY-135, PRO-1762 and trastuzumab.

Anti-EGFR ADCs can be co-administered with a therapeutically effective amount of one or more agents to treat a cancer, including kinesin inhibitors, such as Eg5 inhibitors such as AZD4877, ARRY-520; CENPE inhibitors such as GSK923295A and the like.

Anti-EGFR ADCs can be co-administered with a therapeutically effective amount of one or more agents to treat a cancer, including JAK-2 inhibitors, such as CEP-701 (lesaurtinib), XL019 and INCB018424 and the like.

Anti-EGFR ADCs can be co-administered with a therapeutically effective amount of one or more agents to treat a cancer, including MEK inhibitors, such as ARRY-142886, ARRY-438162 PD-325901, PD-98059 and the like.

Anti-EGFR ADCs (or formulations comprising anti-EGFR ADCs) can be co-administered with a therapeutically effective amount of one or more agents to treat a cancer, including mTOR inhibitors, such as AP-23573, CCI-779, everolimus, RAD-001, rapamycin, temsirolimus, ATP-competitive TORC1/TORC2 inhibitors, including PI-103, PP242, PP30, Torin 1 and the like.

Anti-EGFR ADCs can be co-administered with a therapeutically effective amount of one or more agents to treat a cancer, including non-steroidal anti-inflammatory drugs (NSAIDs), such as AMIGESIC® (salsalate), DOLOBID® (diflunisal), MOTRIN® (ibuprofen), ORUDIS® (ketoprofen), RELAFEN® (nabumetone), FELDENE® (piroxicam), ibuprofen cream, ALEVE® (naproxen) and NAPROSYN® (naproxen), VOLTAREN® (diclofenac), INDOCIN® (indomethacin), CLINORIL® (sulindac), TOLECTIN® (tolmetin), LODINE® (etodolac), TORADOL® (ketorolac), DAYPRO® (oxaprozin) and the like.

Anti-EGFR ADCs can be co-administered with a therapeutically effective amount of one or more agents to treat a cancer, including PDGFR inhibitors, such as C-451, CP-673, CP-868596 and the like.

Anti-EGFR ADCs can be co-administered with a therapeutically effective amount of one or more agents to treat a cancer, including platinum chemotherapeutics, such as cisplatin, ELOXATIN® (oxaliplatin) eptaplatin, lobaplatin, nedaplatin, PARAPLATIN® (carboplatin), satraplatin, picoplatin and the like.

Anti-EGFR ADCs can be co-administered with a therapeutically effective amount of one or more agents to treat a cancer, including polo-like kinase inhibitors, e.g., BI-2536 and the like.

Anti-EGFR ADCs can be co-administered with a therapeutically effective amount of one or more agents to treat a cancer, including phosphoinositide-3 kinase (PI3K) inhibitors, such as wortmannin, LY294002, XL-147, CAL-120, ONC-21, AEZS-127, ETP-45658, PX-866, GDC-0941, BGT226, BEZ235, XL765 and the like.

Anti-EGFR ADCs can be co-administered with a therapeutically effective amount of one or more agents to treat a cancer, including thrombospondin analogs, such as ABT-510 (thrombospondin mimetic), ABT-567, ABT-898 (thrombospondin-1 mimetic peptide), TSP-1 and the like.

Anti-EGFR ADCs can be co-administered with a therapeutically effective amount of one or more agents to treat a cancer, including VEGFR inhibitors, such as AVASTIN® (bevacizumab), ABT-869, AEE-788, ANGIOZYME™ (a ribozyme that inhibits angiogenesis (Ribozyme Pharmaceuticals (Boulder, CO.) and Chiron, (Emeryville, CA)), axitinib (AG-13736), AZD-2171, CP-547,632, IM-862, MACUGEN (pegaptamib), NEXAVAR® (sorafenib, BAY43-9006), pazopanib (GW-786034), vatalanib (PTK-787, ZK-222584), SUTENT® (sunitinib, SU-11248), VEGF trap, ZACTIMA™ (vandetanib, ZD-6474), GA101, ofatumumab, ABT-806 (mAb-806), ErbB3 specific antibodies, BSG2 specific antibodies, DLL4 specific antibodies and C-met specific antibodies, and the like.

Anti-EGFR ADCs can be co-administered with a therapeutically effective amount of one or more agents to treat a cancer, including antibiotics, such as intercalating antibiotics aclarubicin, actinomycin D, amrubicin, annamycin, adriamycin, BLENOXANE® (bleomycin), daunorubicin, CAELYX® or MYOCET® (liposomal doxorubicin), elsamitrucin, epirbucin, glarbuicin, ZAVEDOS® (idarubicin), mitomycin C, nemorubicin, neocarzinostatin, peplomycin, pirarubicin, rebeccamycin, stimalamer, streptozocin, VALSTAR® (valrubicin), zinostatin and the like.

Anti-EGFR ADCs can be co-administered with a therapeutically effective amount of one or more agents to treat a cancer, including topoisomerase inhibitors, such as aclarubicin, 9-aminocamptothecin, amonafide, amsacrine, becatecarin, belotecan, BN-80915, CAMPTOSAR® (irinotecan hydrochloride), camptothecin, CARDIOXANE® (dexrazoxine), diflomotecan, edotecarin, ELLENCE® or PHARMORUBICIN® (epirubicin), etoposide, exatecan, 10-hydroxycamptothecin, gimatecan, lurtotecan, mitoxantrone, orathecin, pirarbucin, pixantrone, rubitecan, sobuzoxane, SN-38, tafluposide, topotecan and the like.

Anti-EGFR ADCs can be co-administered with a therapeutically effective amount of one or more agents to treat a cancer, including therapeutic antibodies, such as AVASTIN® (bevacizumab), CD40-specific antibodies, chTNT-1/B, denosumab, ERBITUX® (cetuximab), HUMAX-CD4® (zanolimumab), IGF1R-specific antibodies, lintuzumab, PANOREX® (edrecolomab), RENCAREX® (WX G250), RITUXAN® (rituximab), ticilimumab, trastuzimab, CD20 antibodies types I and II and the like.

Anti-EGFR ADCs can be co-administered with a therapeutically effective amount of one or more agents to treat a cancer, including hormonal therapies, such as ARIMIDEX® (anastrozole), AROMASIN® (exemestane), arzoxifene, CASODEX® (bicalutamide), CETROTIDE® (cetrorelix), degarelix, deslorelin, DESOPAN® (trilostane), dexamethasone, DROGENIL® (flutamide), EVISTA® (raloxifene), AFEMA™ (fadrozole), FARESTON® (toremifene), FASLODEX® (fulvestrant), FEMARA® (letrozole), formestane, glucocorticoids, HECTOROL® (doxercalciferol), RENAGEL® (sevelamer carbonate), lasofoxifene, leuprolide acetate, MEGACE® (megesterol), MIFEPREX® (mifepristone), NILANDRON™ (nilutamide), NOLVADEX® (tamoxifen citrate), PLENAXIS™ (abarelix), prednisone, PROPECIA® (finasteride), rilostane, SUPREFACT® (buserelin), TRELSTAR® (luteinizing hormone releasing hormone (LHRH)), VANTAS® (Histrelin implant), VETORYL® (trilostane or modrastane), ZOLADEX® (fosrelin, goserelin) and the like.

Anti-EGFR ADCs can be co-administered with a therapeutically effective amount of one or more agents to treat a cancer, including deltoids and retinoids, such as seocalcitol (EB1089, CB1093), lexacalcitrol (KH1060), fenretinide, PANRETIN® (aliretinoin), ATRAGEN® (liposomal tretinoin), TARGRETIN® (bexarotene), LGD-1550 and the like.

Anti-EGFR ADCs can be co-administered with a therapeutically effective amount of one or more agents to treat a cancer, including PARP inhibitors, such as ABT-888 (veliparib), olaparib, KU-59436, AZD-2281, AG-014699, BSI-201, BGP-15, INO-1001, ONO-2231 and the like.

Anti-EGFR ADCs can be co-administered with a therapeutically effective amount of one or more agents to treat a cancer, including plant alkaloids, such as, but are not limited to, vincristine, vinblastine, vindesine, vinorelbine and the like.

Anti-EGFR ADCs can be co-administered with a therapeutically effective amount of one or more agents to treat a cancer, including proteasome inhibitors, such as VELCADE® (bortezomib), MG132, NPI-0052, PR-171 and the like.

Anti-EGFR ADCs can be co-administered with a therapeutically effective amount of one or more agents to treat a cancer, including immunologicals. Examples of immunologicals include interferons and other immune-enhancing agents. Interferons include interferon alpha, interferon alpha-2a, interferon alpha-2b, interferon beta, interferon gamma-la, ACTIMMUNE® (interferon gamma-lb) or interferon gamma-nl, combinations thereof and the like. Other agents include ALFAFERONE®,(IFN-α), BAM-002 (oxidized glutathione), BEROMUN® (tasonermin), BEXXAR® (tositumomab), CAMPATH® (alemtuzumab), CTLA4 (cytotoxic lymphocyte antigen 4), decarbazine, denileukin, epratuzumab, GRANOCYTE® (lenograstim), lentinan, leukocyte alpha interferon, imiquimod, MDX-010 (anti-CTLA-4), melanoma vaccine, mitumomab, molgramostim, MYLOTARG™ (gemtuzumab ozogamicin), NEUPOGEN® (filgrastim), OncoVAC-CL, OVAREX® (oregovomab), pemtumomab (Y-muHMFG1), PROVENGE® (sipuleucel-T), sargaramostim, sizofilan, teceleukin, THERACYS® (Bacillus Calmette-Guerin), ubenimex, VIRULIZIN® (immunotherapeutic, Lorus Pharmaceuticals), Z-100 (Specific Substance of Maruyama (SSM)), WF-10 (Tetrachlorodecaoxide (TCDO)), PROLEUKIN® (aldesleukin), ZADAXIN® (thymalfasin), ZENAPAX® (daclizumab), ZEVALIN® (90Y-Ibritumomab tiuxetan) and the like.

Anti-EGFR ADCs can be co-administered with a therapeutically effective amount of one or more agents to treat a cancer, including biological response modifiers, such as agents that modify defense mechanisms of living organisms or biological responses, such as survival, growth or differentiation of tissue cells to direct them to have anti-tumor activity and include krestin, lentinan, sizofiran, picibanil PF-3512676 (CpG-8954), ubenimex and the like.

Anti-EGFR ADCs can be co-administered with a therapeutically effective amount of one or more agents to treat a cancer, including pyrimidine analogs, such as cytarabine (ara C or Arabinoside C), cytosine arabinoside, doxifluridine, FLUDARA® (fludarabine), 5-FU (5-fluorouracil), floxuridine, GEMZAR® (gemcitabine), TOMUDEX® (ratitrexed), TROXATYL™ (triacetyluridine troxacitabine) and the like.

Anti-EGFR ADCs can be co-administered with a therapeutically effective amount of one or more agents to treat a cancer, including purine analogs, such as LANVIS® (thioguanine) and PURI-NETHOL® (mercaptopurine).

Anti-EGFR ADCs can be co-administered with a therapeutically effective amount of one or more agents to treat a cancer, including antimitotic agents, such as batabulin, epothilone D (KOS-862), N-(2-((4-hydroxyphenyl)amino)pyridin-3-yl)-4-methoxybenzenesulfonamide, ixabepilone (BMS 247550), paclitaxel, TAXOTERE® (docetaxel), PNU100940 (109881), patupilone, XRP-9881 (larotaxel), vinflunine, ZK-EPO (synthetic epothilone) and the like.

Anti-EGFR ADCs can be co-administered with a therapeutically effective amount of one or more agents to treat a cancer, including ubiquitin ligase inhibitors, such as MDM2 inhibitors, such as nutlins, NEDD8 inhibitors such as MLN4924 and the like.

Compounds of this invention can also be used as radiosensitizers that enhance the efficacy of radiotherapy. Examples of radiotherapy include external beam radiotherapy, teletherapy, brachytherapy and sealed, unsealed source radiotherapy and the like.

Anti-EGFR ADCs can be co-administered with a therapeutically effective amount of one or more agents to treat a cancer, including chemotherapeutic agents such as ABRAXANE™ (ABI-007), ABT-100 (farnesyl transferase inhibitor), ADVEXIN® (Ad5CMV-p53 vaccine), ALTOCOR® or MEVACOR® (lovastatin), AMPLIGEN® (poly I:poly C12U, a synthetic RNA), APTOSYN® (exisulind), AREDIA® (pamidronic acid), arglabin, L-asparaginase, atamestane (1-methyl-3,17-dione-androsta-1,4-diene), AVAGE® (tazarotene), AVE-8062 (combreastatin derivative) BEC2 (mitumomab), cachectin or cachexin (tumor necrosis factor), canvaxin (vaccine), CEAVAC® (cancer vaccine), CELEUK® (celmoleukin), CEPLENE® (histamine dihydrochloride), CERVARIX® (human papillomavirus vaccine), CHOP® (C: CYTOXAN® (cyclophosphamide); H: ADRIAMYCIN® (hydroxydoxorubicin); O: Vincristine (ONCOVIN®); P: prednisone), CYPAT™ (cyproterone acetate), combrestatin A4P, DAB(389)EGF (catalytic and translocation domains of diphtheria toxin fused via a His-Ala linker to human epidermal growth factor) or TransMID-107R™ (diphtheria toxins), dacarbazine, dactinomycin, 5,6-dimethylxanthenone-4-acetic acid (DMXAA), eniluracil, EVIZON™ (squalamine lactate), DIMERICINE® (T4N5 liposome lotion), discodermolide, DX-8951f (exatecan mesylate), enzastaurin, EPO906 (epithilone B), GARDASIL® (quadrivalent human papillomavirus (Types 6, 11, 16, 18) recombinant vaccine), GASTRIMMUNE®, GENASENSE®, GMK (ganglioside conjugate vaccine), GVAX® (prostate cancer vaccine), halofuginone, histerelin, hydroxycarbamide, ibandronic acid, IGN-101, IL-13-PE38, IL-13-PE38QQR (cintredekin besudotox), IL-13-pseudomonas exotoxin, interferon-a, interferon-y, JUNOVAN™ or MEPACT™ (mifamurtide), lonafarnib, 5,10-methylenetetrahydrofolate, miltefosine (hexadecylphosphocholine), NEOVASTAT®(AE-941), NEUTREXIN® (trimetrexate glucuronate), NIPENT® (pentostatin), ONCONASE® (a ribonuclease enzyme), ONCOPHAGE® (melanoma vaccine treatment), ONCOVAX® (IL-2 Vaccine), ORATHECIN™ (rubitecan), OSIDEM® (antibody-based cell drug), OVAREX® MAb (murine monoclonal antibody), paclitaxel, PANDIMEX™ (aglycone saponins from ginseng comprising 20(S)protopanaxadiol (aPPD) and 20(S)protopanaxatriol (aPPT)), panitumumab, PANVAC®-VF (investigational cancer vaccine), pegaspargase, PEG Interferon A, phenoxodiol, procarbazine, rebimastat, REMOVAB® (catumaxomab), REVLIMID® (lenalidomide), RSR13 (efaproxiral), SOMATULINE® LA (lanreotide), SORIATANE® (acitretin), staurosporine (Streptomyces staurospores), talabostat (PT100), TARGRETIN® (bexarotene), TAXOPREXIN® (DHA-paclitaxel), TELCYTA® (canfosfamide, TLK286), temilifene, TEMODAR® (temozolomide), tesmilifene, thalidomide, THERATOPE® (STn-KLH), thymitaq (2-amino-3,4-dihydro-6-methyl-4-oxo-5-(4-pyridylthio)quinazoline dihydrochloride), TNFERADE™ (adenovector: DNA carrier containing the gene for tumor necrosis factor-$\alpha$), TRACLEER® or ZAVESCA® (bosentan), tretinoin (Retin-A), tetrandrine, TRISENOX® (arsenic trioxide), VIRULIZIN®, ukrain (derivative of alkaloids from the greater celandine plant), vitaxin (anti-alphavbeta3 antibody), XCYTRIN® (motexafin gadolinium), XINLAY™ (atrasentan), XYOTAX™ (paclitaxel poliglumex), YONDELIS® (trabectedin), ZD-6126, ZINECARD® (dexrazoxane), ZOMETA® (zolendronic acid), zorubicin and the like.

In one embodiment, the formulation comprising the anti-EGFR-ADC is intravenously administered to a subject having glioblastoma in combination with radiation and/or TEMODAR® (temozolomide).

Further, in one embodiment, the composition of the invention can be provided as a pharmaceutical kit comprising (a) a container containing an anti-EGFR ADC in lyophilized form and (b) a second container containing a pharmaceutically acceptable diluent (e.g., sterile water) for injection. The pharmaceutically acceptable diluent can be used for reconstitution or dilution of the lyophilized ADC. Optionally associated with such container(s) can be a notice in the form prescribed by a governmental agency regulating the manufacture, use or sale of pharmaceuticals or biological products, which notice reflects approval by the agency of manufacture, use or sale for human administration.

The invention is further described in the following examples, which are in not intended to limit the scope of the invention.

## EXAMPLES

### Example 1: Conjugation of Auristatin vc-MMAE to Anti-EGFR Antibody 1

The following example describes the conjugation of an antibody to an auristatin to form an antibody drug conjugate (ADC), specifically the conjugation of MMAE to anti-EGFR Antibody 1. Generation of the Antibody 1 ADC with reduced drug loads of vc-MMAE molecules per antibody, involved a partial reduction of the mAb followed by reaction with Val-Cit-MMAE (vcMMAE) to complete the conjugation, as described in detail below.

*Disulfide Reduction of Antibody*

Reduction of Antibody 1 was achieved using TCEP (tricarboxyethyl phosphine). Recombinant monoclonal Antibody 1 was produced by a transfected Chinese hamster ovary (CHO) cell line and purified at Abbott Bioresearch Center (Worcester, MA). Following antibody purification, the antibody solution (148 mg/mL, 6 mL) was charged into a 50 mL polypropylene centrifuge tube. The antibody solution was then diluted to a total volume of 41 mL by adding PBSE Buffer (360 mL; 125 mM $K_2HPO_4$, 150 mM NaCl; 6.3 mM EDTA, pH 7.7). Protein content was 21.6 mg/ml as determined by $A_{280}$. 19 ml of antibody solution was charged into a reactor for a total of 410.6 mg. The antibody solution was warmed to 37 °C. Antibody 1 (20 mg/mL) was then partially reduced by the addition of TCEP (Sigma Aldrich Fine Chemical (St. Louis, MO)) to the antibody solution. Specifically, 9.67 mM TCEP solution (0.592 mL, 2.05 equiv) was added to the antibody solution (molar equivalents of TCEP :mAb was 2.05). Following the addition of TCEP, the antibody solution was incubated at 37°C for 1 hour. The reduction reaction was then chilled to 20°C. This process resulted in the reduction of the disulfide bonds of Antibody 1.

*Conjugation of MMAE and Antibody 1*

The following describes the process by which MMAE was conjugated to exemplary Antibody 1 following reduction of the antibody.

To conjugate the thiols of Antibody 1, Val-Cit-MMAE (vc-MMAE). Val-Cit (para-aminobenzylcarbamate-monomethylauristatin E; Sigma Aldrich Fine Chemical (St. Louis, MO)) was added to the antibody solution to a final vc-MMAE:reduced Cysteine (Cys) molar ratio of 1.15. The conjugation reaction was carried out in the presence of 10% v/v of DMSO (dimethylsulfoxide; Sigma Aldrich Fine Chemical (St. Louis, MO)), and allowed to proceed at 20°C for 45 minutes.

After the conjugation reaction, excess free N(acetyl)-Cysteine (Sigma Aldrich Fine Chemical (St. Louis, MO) (2.3 equivalents vs. vcMMAE charge) was added to quench unreacted vc-MMAE to produce N(acetyl)-Cys-vc-MMAE. The N(aceytl)-Cys quenching reaction was allowed to proceed at 20°C for approximately 30 minutes. The quenched reaction mixture (also referred to as the crude solution) was then purified, as described below in Example 2.

Alternatively, the following protocol was also used for a smaller scale reaction. conjugation was performed by charging 10mM vcMMAE DMSO solution (1.32 mL, 4.72 equiv.). Charge DMSO (0.86 mL). The reaction mixtures was then stirred at ambient temperature for 1 hour. Excess drug linker was quenched by the addition of 50 mM N-(acetyl) Cysteine (0.53 mL). The mixture was then stirred for about 15 minutes. The reaction mixture was then stored in the refrigerator.

*Analytical Analysis of Reaction Mixture*

Analytical analysis of the reaction mixture was performed. Analysis of the supernatant samples was accomplished by hydrophobic interaction chromatography-high-performance liquid chromatography (HIC-HPLC) using an TSKgel Butyl-NPR column (4.6 mm ID x 3.5 cm, 2.5 um;Tosoh Bioscience LLC, Japan)).

UV analysis of the protein content showed 386.4 mg of protein. HIC trace analysis showed that the average Drug to Antibody Ratio (DAR) for Antibody 1-vcMMAE was 3.85, as described below in Table 1. The average DAR was determined by summing up the 2, 4, 6 and 8 ADC product of multiplying PA% (PA% is the peak area percent as determined by the area measured under the peak at $A_{280}$) by requisite drug load and dividing by 100, e.g., [(6.3 PA% x 0) + (24.8 PA% x 2) + (34.8 x 4) + (20.9 x 6) + (8.8 x 8)] / 100 = 3.85.

Table 1: HIC Trace Analysis of Reaction Mixture

| HIC | | |
|---|---|---|
| DAR | PA% | Drug Load Equivalent |
| 0 | 6.34 | 0 |
| 2 | 24.84 | 49.680 |
| 4 | 34.84 | 139.360 |
| 6 | 20.92 | 125.520 |
| 8 | 8.79 | 70.320 |
| Average DAR | | 3.8488 |

As described in Table 1, the conjugation reaction resulted in a mixture of species of ADCs having a range of drug loads, *i.e.,* DARs of 2 to 8. A small percentage of ADCs had no drug load as described in Table 1.

**Example 2: Batch Purification of Antibody Drug Conjugate (ADC) Using a Hydrophobic Resin**

The following example describes batch purification of an ADC (Antibody 1-vc-MMAE), where the resulting purified composition had an average DAR of 2.8. The following purification process selectively removed the higher loaded ADCs, *i.e.,* the six and eight drug-loaded species, resulting in a purified distribution comprising lower ordered drug load species, *i.e.,* DARs of 2-4. The purification process utilized small amounts of a hydrophobic resin that could be titrated in to the crude antibody solution (or mixture) in order to selectively remove ADCs of varying degree of conjugation.

The purification process provides a practical, scalable process to selectively modulate the distribution of both Auristatin E and Auristatin F conjugates resulting from partial inter-chain disulfide reduction and subsequent alkylation with vc-MMAE or mc-MMAF. The purification method described below has been demonstrated on both a milligram to multi-gram scale in either batch mode or in circulation mode affording the purified distribution in 86% yield. An overview of the antibody reduction, conjugation, and purification process is described in Figure 1.

*Materials and Methods*

The buffers described herein were prepared as follows:
Buffer A (50 mM $K_2HPO_4$ buffer pH 7 Buffer / 2M NaCl) was prepared by charging $K_2HPO_4$ (0.87 g) ($K_2HPO_4$; Fisher Scientific) and NaCl (11.7 g) (NaCl; EMD) diluting with WIFI to approximately 90 mL. The resulting solution was treated with 1.0 N HCl to a final pH of 7.0 and further diluted to a total volume of 100 mL.

Buffer A' (50 mM $K_2HPO_4$ / 4M NaCl) was prepared by charging NaCl (2.92 g) into a flask followed by charging Mobile Phase A to achieve a final volume of 25 mL.

Buffer B (50 mM $K_2HPO_4$ buffer pH 7 Buffer) was prepared by charging $K_2HPO_4$ (0.87 g) and NaCl (11.7 g) diluting with WIFI (water-intended for injection; Gibco) to approximately 90 mL. The resulting solution was treated with 1.0 N HCl (1.0 N HCl; JT Baker), to a final pH of 7.0 and further diluted to a total volume of 100 mL.

Pre-treated Butyl-HIC (Bu-HIC) resin was prepared by briefly mixing the bulk container of ToyoPearl Butyl - 600M Resin slurry (ToyoPearl Bu-HIC Resin (600M); Tosoh Bioscience), pouring out (1 gram) into a coarse polypropylene filter. The slurry was filtered and rinsed with Buffer A (3 x 2 mL). The wet cake was dried by passing filtered nitrogen through the wet cake for 10 minutes or until no more droplets were observed on the bottom of the coarse funnel. The dry weight basis was calculated by subtracting the amount of water present on the wet cake. The amount of moisture was measured by Karl Fisher analysis (typically contains 55% water).

*Titration Screening Study to Determine Conditions for ADC DAR 2-4*

A solid phase titration study was performed to determine the conditions for removing ADCs having a DAR of 6-8. Analysis of the supernatant samples was accomplished by hydrophobic interaction chromatography-high-performance liquid chromatography (HIC-HPLC) using an TSKgel Butyl-NPR column (4.6 mm ID x 3.5 cm, 2.5 um; Tosoh Bioscience LLC, Japan)). The method consisted of a linear gradient from 100% buffer A [25 mM sodium phosphate, 1.5 M $(NH_4)_2SO_4$, pH 7.0] to 100% buffer B [75% v/v 25 mmol/L sodium phosphate (pH 7.0), 25% v/v isopropanol] in 12 minutes. The flow rate was set at 0.8 mL/min, inject 30 uL, the temperature was set at 30°C, and detection was followed at 280 nm.

*Sample Preparation for HIC-HPLC Analysis*

The sample was prepared by filtering off the reaction mixture slurry through a 5 um syringe filter. The filtrate was diluted 5-fold with Buffer A (30 uL/injection).

Eight conditions (Assays-Bu-0, Bu-1, Bu-2, Bu-4, Bu-8, Bu-16, Bu-32, and scale up) were tested with varying amounts of resin in each. Assay-Bu-0 was performed by charging 100 uL of crude Antibody 1-vcMMAE reaction solution (Example 1) (18 mg/mL) into a vial. Buffer A' (100 uL) was added, followed by the addition of 100 uL of Buffer B. The solution was then shaken on the lowest setting (orbital mixer). A sample of the supernatant was taken at 20 minutes. Supernatant was sampled and measured according to HIC-HPLC.

Specifically, sample preparation was conducted by removing 30 uL of the supernatant, diluting it with 120 uL of Buffer A, and measuring the contents by HIC-HPLC. See Table 5 for a summary of distribution.

Assays-Bu-1 to Bu-32 were all variants of Assay-Bu-0, which did not contain any hydrophobic interaction resin and was the control. Assay-Bu-1 was the same as Assay-Bu-0 except 0.8 mg of preconditioned n-Bu HIC 600M resin was added to the solution prior to adding Buffer B. Assay-Bu-2 was the same as Assay-Bu-0 except 1.6 mg of preconditioned n-Bu HIC 600M resin was added prior to adding Buffer B. Assay-Bu-4 was the same as Assay-Bu-0 except 3.2 mg of preconditioned n-Bu HIC 600M resin was added prior to adding Buffer B. Assay-Bu-8 was the same as Assay-Bu-0 except 6.4 mg of preconditioned n-Bu HIC 600M resin was added prior to adding Buffer B. Assay-Bu-16 was the same as Assay-Bu-0 except 12.8 mg of preconditioned n-Bu HIC 600M resin was added prior to adding Buffer B. Assay-Bu-32 was the same as Assay-Bu-0 except 25.6 mg of preconditioned n-Bu HIC 600M resin was added prior to adding Buffer B. For each of these experiments, the final NaCl concentration was 1.3 M NaCl. The results from the eight conditions are summarized in Tables 2-5 below.

Table 2 provides a summary of the distribution of various ADC species (e.g., antibody alone / unconjugated (% mAb), an ADC having a DAR of 2 (%2 Load), an ADC having a DAR of 4 (% 4 Load), etc.). The load to protein ratio described in Table 2 represents the dry weight of the resin vs. the calculated antibody protein weight. ADC weight is calculated by total protein content as measured by UV absorption at 280 nm multiplied by peak area% of the drug loaded species. As described in Table 2, the resin load:protein ratio impacted the % ADC having certain DARs. For example, a resin load vs. total protein of 1.8 (or 5.9 weights of resin versus the 6-8 load; see row "Assay-Bu-4" of Table 2) resulted in 94% purity of the 2 or 4 Drug-loaded Species (47% each) and no detectable level of ADCs having a DAR of 6 or 8, as determined by HIC-HPLC. Table 3 describes the amount of Bu-HIC resin that was added for each experiment described in Table 2, while Table 4 describes the calculation of net weight of drug loaded species present in the screen.

Table 2. Summary of Distribution as Measured by HIC-HPLC

| Experiment | Resin Load vs Total Protein | Resin load vs. 6-8 | % mAb | %2 Load | %4 Load | %6 Load | % 8 Load | Average DAR |
|---|---|---|---|---|---|---|---|---|
| Assay-Bu-0* | 0 | 0 | 3.4 | 30.1 | 36.6 | 21.8 | 8.1 | 4.02 |
| Assay-Bu-1* | 0.44 | 1.5 | 4.4 | 31 | 40.3 | 22 | 2.2 | 3.73 |
| Assay-Bu-2* | 0.9 | 3.0 | 4.4 | 37 | 47.3 | 4.3 | 0 | 2.89 |
| Assay-Bu-4* | 1.8 | 5.9 | 5.9 | 47 | 47 | 0 | 0 | 2.82 |
| Assay-Bu-8* | 3.5 | 11.7 | 10.4 | 74.2 | 15.5 | 0 | 0 | 2.10 |
| Assay-Bu-16 | 7.1 | 23.7 | 59.3 | 40.7 | 0 | 0 | 0 | 0.81 |
| Assay-Bu-32 | 14.2 | 47.5 | 100 | 0 | 0 | 0 | 0 | 0 |
| Assay (Scale-up) | 2 | 6.7 | 8.7 | 40 | 47 | 1.5 | 0 | 2.82 |
| * As measured at > 24 hours of residence time with addition of 1 volume% of IPA | | | | | | | | |

Table 3: Charge Amounts of Bu-HIC Resin

| Experiment | Weight of Total Protein in mgs in vial** | Wet Weight* Bu-Resin Charge (mg) | Dry Weight Bu-resin Charge (mg) | Dry Weight Resin / Weight of Total Protein | Dry weight vs. 6-8 |
|---|---|---|---|---|---|
| Assay-Bu-0 | 1.8 | 0 | 0 | 0 | 0 |
| Assay-Bu-1 | 1.8 | 1.8 | 0.8 | 0.44 | 1.5 |
| Assay-Bu-2 | 1.8 | 3.6 | 1.6 | 0.9 | 3.0 |
| Assay-Bu-4 | 1.8 | 7.2 | 3.2 | 1.8 | 6.0 |
| Assay-Bu-8 | 1.8 | 14.4 | 6.4 | 3.5 | 11.7 |
| Assay-Bu-16 | 1.8 | 28.8 | 12.8 | 7.1 | 23.7 |
| Assay-Bu-32 | 1.8 | 57.6 | 25.6 | 14.2 | 47.5 |
| *(water content of resin = 55%); ** Based on 280 nm protein concentration and volume delivered per vial | | | | | |

Table 4: Calculation of Net Weight of Drug Loaded Species Present in Screen

| Drug Load Species | PA%* of Drug Load Species | Calculated Net Weight of Drug Load Species** (mg) in vial |
|---|---|---|
| 0-Load (mAb) | 2.8 | 0.05 |
| 2-Load | 29.8 | 0.54 |
| 4-Load | 37.3 | 0.67 |
| 6-Load | 22.3 | 0.40 |
| 8-Load | 7.8 | 0.14 |
| 6/8 Load | 30.1 | 0.54 |
| 4/6/8 Load | 67.4 | 1.21 |
| 2/4/6/8 Load | 97.2 | 1.75 |

(continued)

| Drug Load Species | PA%* of Drug Load Species | Calculated Net Weight of Drug Load Species** (mg) in vial |
|---|---|---|
| 0/2/4/6/8 | 100 | 1.8 |
| * Odd Species were not included; ** 1.8 mg total protein / vial | | |

The above analytical HIC results demonstrated sufficient selectivity to reduce the presence of certain drug loaded species (e.g., ADCs having a DAR of 6-8). The data from Tables 2 and 5 suggest that, in the presence of an end ionic strength of ~ 1.3 M NaCl concentration, or an equivalent ionic strength thereof, reduction of the 8-loaded species can largely be achieved by utilizing a 0.5 (specifically 0.44% wt) wt resin charge; the reduction of the 6/8 loaded species can be achieved through the addition of about two weights of hydrophobic resin to crude conjugation reaction mixture. The 4-8 drug loaded species can largely be removed by using ~ 3.5 weights of resin (versus total protein (see "Assay-Bu-8" row of Table 5) and the 2-8 loaded species can be removed by utilizing ~ 7 weights of resin versus total protein (see "Assay-Bu-16" row of Table 5) . These data also suggest that the 4 weight load or 2 weight loading (versus total protein) on a dry weight basis was selective at removing the high drug loads, with minimal attrition of the 2 or 4 drug loaded species.

The weight ratio loading of resin to species to be reduced was calculated and summarized in Table 5.

Summaries of the analytical HIC study results are provided in Table 5.

Table 5: Summary of Drug Loaded Species* vs. Resin Charge

| Experiment | Net weight of resin in mg (dry Weight) | Weights of Resin (dry Weight) vs. 8 Load (0.14 mg/ vial) | Weights of Resin 6/8 Load (0.54 mg/vial) | Weights of Resin 4/6/8 Load (1.21 mg/vial) | Weights of Resin 2/4/6/8 Load (1.75 mg/ vial) | Weights of resin / Total Protein (1.8 mg/vial) |
|---|---|---|---|---|---|---|
| Assay-Bu-0 | 0 | 0 | 0 | 0 | 0 | 0 |
| Assay-Bu-1 | 0.8 | <u>5.7</u> | 1.5 | 0.7 | 0.5 | **0.44** |
| Assay-Bu-2 | 1.6 | <u>11.5</u> | 3.0 | 1.3 | 0.9 | 0.9 |
| Assay-Bu-4 | 3.2 | <u>22.9</u> | **5.9** | 2.6 | 1.8 | 1.8 |
| Assay-Bu-8 | 6.4 | <u>45.7</u> | <u>11.7</u> | <u>5.3</u> | 3.7 | 3.5 |
| Assay-Bu-16 | 12.8 | <u>91.4</u> | <u>23.7</u> | <u>10.6</u> | 7.3 | 7.1 |
| Assay-Bu-32 | 25.6 | <u>183</u> | <u>47.5</u> | <u>21.2</u> | 14.7 | 14.2 |
| * See Table 3 for calculation of drug load species<br>** Underlined values denote approximate amount of resin load where the amount of 6/8 species was less than the 2% (as shown in Table 2) | | | | | | |

*Summary*

In sum, the reaction mixture obtained from Example 1 was diluted with Buffer A' (4N NaCl, 0.05 M pH 7 $K_2HPO_4$ phosphate buffer, 1 mL/mL conjugation reaction mixture). The diluted reaction mixture was treated with the calculated amount of pre-treated Bu-HIC resin, filtered through a coarse polypropylene filter, and further diluted with Buffer B (0.05 M pH 7 $K_2HPO_4$ phosphate buffer, 1 mL/ mL conjugation reaction mixture). The calculated amount of resin, as shown in Table 5, depends on the drug load species that is to be removed from the crude mixture. For example, a resin weight of approximately 5 to 10 times that of the 6 and 8 drug load species was proven to be effective for removing these species from the crude mixture. The resin / diluted reaction mixture was stirred for the appropriate time, and monitored by analytical hydrophobic interaction chromatography for reduction of the specified drug conjugate products.

**Example 3: Scale Up of Batch Hydrophobic Interaction ADC Purification**

The optimal conditions for removing high DAR ADCs that were identified from the titration screen were scaled up for larger scale purification.

In order to first reduce the antibody, a solution containing Antibody 1 (151 mg/mL, 50 mL, 7.52g) was added to a 500 mL flask. The solution was diluted to a total volume of 395 mL by the addition of a solution prepared by mixing a pH 6, 15 mM Histidine buffer (30 mL) and PBSE Buffer (360 mL; 125 mM $K_2HPO_4$, 150 mM NaCl; 6.3 mM EDTA, pH 7.7). The resulting antibody solution was warmed to 37° C. 10.98 mM TCEP solution (12.1 mL, 2.05 equiv) was then added to the solution, which was stirred for 30 minutes. The antibody solution was then cooled to ambient temperature over 20 minutes.

Once reduced, Antibody 1 was conjugated to vcMMAE by adding 10mM vcMMAE DMSO solution (28.8 mL, 4.72 equiv.) to the antibody solution. DMSO (21.2 mL) was added next, whereupon the solution was stirred at ambient temperature for 45 minutes. Excess drug linker was quenched by the addition of 50 mM N-Acetyl cysteine (9.7 mL). The solution was stirred for about 15 minutes. UV protein concentration was determined to be 7.4 g of protein following the conjugation of Antibody 1 to vcMMAE, and HIC analysis showed a DAR of 4.1 (about a 25 PA% (or 1.85 g) combined 6-8 drug load species).

The crude reaction mixture was then diluted with an equal volume of 4N NaCl / 0.05 M pH 7 $K_2HPO_4$ buffer. 30.7 g pre-washed Bu HIC wet resin (KF = 56 wt% water; net 17.2 g resin on dry weight basis; 2.3 weights resin vs total protein; 9.3 weights resin vs 6-8 loaded species) was then added, followed by the addition of 50 mM $KH_2PO_4$ pH7 Buffer (460 mL). The antibody resin solution was gently stirred for 3 hours at room temperature. Alternatively, the solution was stored for 12 hours in the refrigerator, and subsequently stirred for an additional 2.5 hours.

Resin was then filtered off through a coarse polypropylene filter. Clear filtrate was poured into a new container. Weight of 1469 g (density = 1.06 g/mL) was determined. Analytical analysis of purified ADC solution showed that the solution had a UV protein content of 3.7 mg/ml or 5.07 g of total protein (a 67% overall yield). The HIC trace analysis showed a DAR of 2.8.

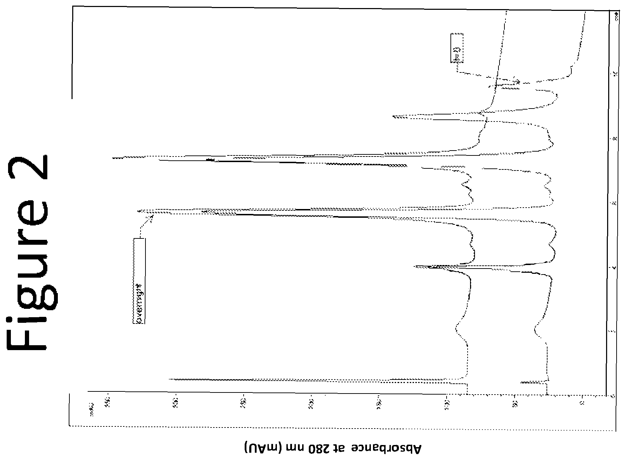

A graph showing an overlay of HIC-HPLC of the antibody solutions before and after purification is provided in Figure 2. The two late eluting peaks in Figure 2 represent ADCs having a DAR of 6-8 (retention time: 8.6 minutes and 9.6 minutes respectively). These peaks are missing following purification, demonstrating that the 0, 2, and 4 DAR ADC species are not affected and that this purification process is selective in that it removes only the high (e.g., 6 - 8) DAR ADC species.

*UF/DF (Concentration and Final Buffer Exchange)*

Following purification, the purified ADC solution was subjected to ultrafiltration / diafiltration (UF/DF) and final buffer exchange. The filtrate was added to the UF/DF reservoir, concentrating the solution to ~ 50 mg/mL and removing 1400 g on a Pall Centramate Omega 30K LV1 part OS030C12P1 serial number 31061058R at a transmembrane pressure of ~ 25 psi and a peristaltic pump speed of 80 - 100 mL/minute (approximately 1 hour to concentrate). After concentrating to ~ 50 mg/mL, 10 DV of a 15 mM pH 6.0 Histidine buffer was run. The UF/DF system was drained and subsequently flushed with 15 mM pH 6.0 Histidine buffer (2 x 20 mL). Concentration was measured at (127 g solution) 40.1 mg/mL. Diluted with 15 mM Histidine pH6.0 buffer to a concentration of 35.1 mg/mL (141 g BDS). The BDS was filtered through 0.45 micron syringe filter, followed by a 0.2 micron sterile filtration. Filtrate was charged into 12 vials at 100 mg each and Falcon tubes (3 x 35 mL).

The above UF/DF process may be used prior to or following the batch purification process.

In a separate experiment, 2 grams of pre-treated Toyopearl Butyl-600M HIC resin (Tosoh Bioscience, Japan)/ 1 gram of mAb and further dilution with 0.05 M pH 7 $K_2HPO_4$ phosphate buffer and stirred at ambient for 6 hours. The slurry was filtered through a coarse polypropylene filter and washed with Buffer A (2 wet cake bed volumes). The resulting filtrate and rinses were combined and buffer-exchanged into 0.015 M pH 6 histidine buffer by diafiltration to afford the purified Antibody 1-Val-Cit-MMAE referred to as Antibody 1-vcMMAEp. In this example, the overall yield was 67% of Antibody 1-vcMMAEp. Based on the amount of 0-4 drug loaded species in the crude reaction mixture, the purification yield was 87%.

**Example 4: Preparation of Anti-EGFR Antibody 1/ mc-MMAF ADC**

The following example describes the preparation of Anti-EGFR Antibody 1 mc-MMAF ADC.

*Reduction of Anti-EGFR Antibody 1*

Following antibody purification, the antibody solution (151 mg/mL, 86 mL) was charged into a 1 L flask. The antibody solution was then diluted to a total volume of 729 mL by adding PBSE Buffer (600 mL; 125 mM $K_2HPO_4$, 150 mM NaCl; 6.3 mM EDTA, pH 7.7) and 15 mM Histidine buffer (43 mL, pH 6). Protein content was 20.0 mg/ml as determined by UV spectroscopy ($A_{280}$). The solution containing Antibody 1 was heated to 37°C. A 9.67 mM TCEP solution (0.592 mL, 2.05 equiv) was then added to the solution of Antibody 1 under stirring for 30 minutes. The reaction was subsequently cooled to ambient temperature over 20 minutes.

*Conjugation of mcMMAF to Anti-EGFR Antibody 1*

Anti-EGFR Antibody 1 was subsequently conjugated to maleimidocaproyl-MMAF (Antibody 1-mcMMAF). Charge 10 mM mcMMAF/DMSO (38 mL, 4.72 equivalents). Charge DMSO (18.6 mL). Stir for 1 hour at ambient temperature. Excess mc-MMAF was quenched by the addition of 100 mM N-Acetyl cysteine (7.6 mL) and stirring for 15 minutes. The quenched reaction was placed in the refrigerator.

*Analysis of the Crude Antibody 1-mcMMAF Reaction Mixture*

The reaction mixture was analyzed to determine the protein concentration. UV spectroscopy ($A_{280}$) showed a protein concentration of 17.7 mg/mL. Analysis of the resulting HIC trace revealed a DAR of 3.93 (Table 6). The average DAR was determined by summation of the 2, 4, 6 and 8 ADC product of multiplying PA% by requisite drug load) and dividing by 100, e.g., [(5.72 PA% x 0) + (27.27 PA% x 2) + (41.08 x 4) + (16.79 x 6) + (9.13 x 8)] / 100 = 3.93.

Table 6: HIC Trace Analysis of Antibody 1-mc-MMAF Reaction Mixture

| DAR | PA% | Drug Load |
|---|---|---|
| 0 | 5.72 | 0 |
| 2 | 27.27 | 49.680 |
| 4 | 41.08 | 139.360 |
| 6 | 16.79 | 125.520 |
| 8 | 9.13 | 70.320 |
| Average DAR* | | 3.93 |
| * Summation of the Drug Load Equivalents / 100 | | |

As described in Table 6, the conjugation reaction resulted in a mixture of species of ADCs having a range of drug loads, *i.e*., DARs of 2 to 8. A small percentage of ADCs had no drug load as described in Table 6.

*UF/DF (Concentration and Final Buffer Exchange)*

The purified ADC solution was subjected to ultrafiltration /diafiltration (UF/DF) and final buffer exchange. Tangential flow filtration was performed on a Millipore Biomax Pellicon 3 88 $cm^2$ membrane. The sample was concentrated to 100 mg/mL at 20 psi (TMP) and 40 mL/min crossflow. The protein was subsequently diluted to a concentration of 60 mg/mL with 15 mM Histidine buffer (pH 6) by performing 10 DVs at approximately 20 psi (TMP) at a rate of 40 mL/min. The resulting solution was filtered through a 0.45 $\mu$m Millipak 20 filter (Millipore) . The protein concentration was determined to be 59.7 mg/mL via UV spectroscopy ($A_{280}$). A 58.6 mL sample of the UF/DF purified bulk mc-MMAF Antibody 1 solution was subsequently diluted with 15 mM Histidine buffer (pH 6.0) to a final volume of 100 mL. The concentration as determined by UV spectroscopy was 35.7 mg/mL. The protein solution was then filtered through a 0.2 $\mu$m Millipak 20 filter (Millipore) into a sterile 125 ml PETG bottle. The purified mc-MMAF Antibody 1 solution was frozen and stored in a -80°C cryofreezer.

**Example 5: Batch Purification of mc-MMAF ADC Using Hydrophobic Resin**

The purified Antibody 1-mc-MMAF from Example 4 was subjected to a resin treatment purification screen. The screen was performed by varying the total resin charge (0.5, 1, 2, and 3 wts; the purified Antibody 1-mc-MMAF varied from 9.5 mg/mL to 34 mg/mL), the NaCl concentration (0 N, 0.65 N, 1.3 N), and the residence time (0.5 hours, 4 hours, and 20 hours). Table 7 provides a summary of the distribution of the various ADC species as a function of the resin charge, NaCl concentration, and residence time. The DAR values were determined by analysis of the HIC trace as described above. The calculated yield was determined by UV spectroscopy and are summarized in Table 8.

Table 7: Summary of Distribution as Measured by HIC-HPLC

| Description | Resin Load vs. Total Protein | Resin Loading vs. 6-8 drug Load | Residence Time (Hours) | Salt Concentration (Molarity of NaCl) | 0 Load | 2 Load | 4 Load | 6 Load | 8 Load | DAR | Calculated Yield (280 nm) |
|---|---|---|---|---|---|---|---|---|---|---|---|
| TO ON NaCl | 0 | 0 | 0 | 0 | 5 | 28.1 | 43.2 | 17 | 6.7 | 3.85 | N/A |
| TO 0.6N NaCl | 0 | 0 | 0 | 0.65 | 5.1 | 29.1 | 42.6 | 16.6 | 6.6 | 3.81 | N/A |
| T01.3N NaCl | 0 | 0 | 0 | 1.3 | 5.1 | 29.7 | 42.3 | 16.5 | 6.5 | 3.8 | N/A |
| M30 ON NaCl 0.5 wt Resin | 0.5 | 2.1 | 0.5 | 0 | 5.2 | 30 | 43.5 | 16.2 | 5 | 3.71 | 97% |
| M30 ON NaCl 1 wt Resin | 1 | 4.2 | 0.5 | 0 | 5.5 | 31.3 | 44.4 | 15.4 | 3.4 | 3.6 | 94% |
| M30 ON NaCl 2 wt Resin | 2 | 8.4 | 0.5 | 0 | 6.1 | 35.2 | 47.8 | 10.5 | 0.4 | 3.28 | 85% |
| M30 ON NaCl 3 wt Resin | 3 | 12.6 | 0.5 | 0 | 6.7 | 38.2 | 48.9 | 6.2 | 0 | 3.09 | 78% |
| M30 0.6N NaCl 0.5 wt Resin | 0.5 | 2.1 | 0.5 | 0.65 | 5.4 | 31.2 | 44.7 | 15.8 | 2.9 | 3.59 | 90% |
| M30 0.6N NaCl 1 wt Resin | 1 | 4.2 | 0.5 | 0.65 | 5.9 | 34.5 | 47.8 | 11.2 | 0.6 | 3.32 | 86% |
| M30 0.6N NaCl 2 wt Resin | 2 | 8.4 | 0.5 | 0.65 | 6.7 | 38.7 | 50.1 | 4.5 | 0 | 3.05 | 74% |
| **M30 0.6N NaCl 3 wt Resin** | **3** | **12.6** | **0.5** | **0.65** | **8** | **44.4** | **46.5** | **1.1** | **0** | **2.81** | **63%** |
| M30 1.3N NaCl 0.5 wt Resin | 0.5 | 2.1 | 0.5 | 1.3 | 5.5 | 33.2 | 46 | 13.8 | 1.4 | 3.44 | 93% |
| M30 1.3N NaCl 1 wt Resin | 1 | 4.2 | 0.5 | 1.3 | 6.2 | 35.8 | 49 | 9 | 0 | 3.22 | 81% |
| **M30 1.3N NaCl 2 wt Resin** | **2** | **8.4** | **0.5** | **1.3** | **7.7** | **42** | **48.1** | **2.2** | **0** | **2.9** | **68%** |
| **M30 1.3N NaCl 3 wt Resin** | **3** | **12.6** | **0.5** | **1.3** | **9.4** | **52.4** | **38.2** | **0** | **0** | **2.58** | **55%** |

(continued)

| Description | Resin Load vs. Total Protein | Resin Loading vs. 6-8 drug Load | Residence Time (Hours) | Salt Concentration (Molarity of NaCl) | 0 Load | 2 Load | 4 Load | 6 Load | 8 Load | DAR | Calculated Yield (280 nm) |
|---|---|---|---|---|---|---|---|---|---|---|---|
| H4 0N NaCl 0.5 wt Resin | 0.5 | 2.1 | 4 | 0 | 5.3 | 30 | 44.2 | 16.2 | 4.3 | 3.68 | 1% |
| H4 0N NaCl 1 wt Resin | 1 | 4.2 | 4 | 0 | 5.6 | 31.3 | 46.7 | 14.6 | 1.8 | 3.51 | 90% |
| H4 0N NaCl 2 wt Resin | 2 | 8.4 | 4 | 0 | 6.4 | 36.6 | 48.9 | 8.1 | 0 | 3.17 | 84% |
| H4 0N NaCl 3 wt Resin | 3 | 12.6 | 4 | 0 | 7.2 | 40.3 | 48.4 | 4 | 0 | 2.98 | 72% |
| H4 0.6N NaCl 0.5 wt Resin | 0.5 | 2.1 | 4 | 0.65 | 5.6 | 32 | 45.7 | 14.8 | 1.9 | 3.51 | 92% |
| H4 0.6N NaCl 1 wt Resin | 1 | 4.2 | 4 | 0.65 | 6.1 | 34.6 | 48.5 | 10.8 | 0 | 3.28 | 86% |
| **H4 0.6N NaCl 2 wt Resin** | **2** | **8.4** | **4** | **0.65** | **7.3** | **40.8** | **49** | **2.8** | **0** | **2.94** | **70%** |
| **H4 0.6N NaCl 3 wt Resin** | **3** | **12.6** | **4** | **0.65** | **8.8** | **49.4** | **41.8** | **0** | **0** | **2.66** | **59%** |
| H4 1.3N NaCl 0.5 wt Resin | 0.5 | 2.1 | 4 | 1.3 | 5.6 | 33.3 | 46.7 | 13.6 | 0.8 | 3.41 | 88% |
| H4 1.3N NaCl 1 wt Resin | 1 | 4.2 | 4 | 1.3 | 6.2 | 36 | 49.3 | 8.4 | 0 | 3.2 | 80% |
| **H4 1.3N NaCl 2 wt Resin** | **2** | **8.4** | **4** | **1.3** | **7.8** | **44.7** | **47.1** | **0.5** | **0** | **2.81** | **64%** |
| **H4 1.3N NaCl 3 wt Resin** | **3** | **12.6** | **4** | **1.3** | **10.6** | **56.1** | **33.4** | **0** | **0** | **2.46** | **48%** |
| H20 0N NaCl 0.5 wt Resin | 0.5 | 2.1 | 20 | 0 | 5.1 | 29.4 | 44.6 | 16.4 | 4.4 | 3.71 | 100% |
| H20 0N NaCl 1 wt Resin | 1 | 4.2 | 20 | 0 | 5.4 | 31.4 | 47.1 | 14.3 | 1.8 | 3.51 | 95% |

| Description | Resin Load vs. Total Protein | Resin Loading vs. 6-8 drug Load | Residence Time (Hours) | Salt Concentration (Molarity of NaCl) | 0 Load | 2 Load | 4 Load | 6 Load | 8 Load | DAR | Calculated Yield (280 nm) |
|---|---|---|---|---|---|---|---|---|---|---|---|
| H20 0N NaCl 2 wt Resin | 2 | 8.4 | 20 | 0 | 6.1 | 34.7 | 50 | 9.1 | 0 | 3.24 | 85% |
| H20 0N NaCl 3 wt Resin | 3 | 12.6 | 20 | 0 | 7.3 | 40.5 | 48.4 | 3.8 | 0 | 2.97 | 70% |
| H20 0.6N NaCl 0.5 wt Resin | 0.5 | 2.1 | 20 | 0.65 | 5.5 | 28.9 | 47.2 | 16.3 | 2.1 | 3.61 | 91% |
| H20 0.6N NaCl 1 wt Resin | 1 | 4.2 | 20 | 0.65 | 5.9 | 33.9 | 49.7 | 10.5 | 0 | 3.3 | 83% |
| **H20 0.6N NaCl 2 wt Resin** | **2** | **8.4** | **20** | **0.65** | **7.2** | **39.7** | **50.6** | **2.5** | **0** | **2.97** | **71%** |
| **H20 0.6N NaCl 3 wt Resin** | **3** | **12.6** | **20** | **0.65** | **9.2** | **49** | **41.8** | **0** | **0** | **2.65** | **58%** |
| H20 1.3N NaCl 0.5 wt Resin | 0.5 | 2.1 | 20 | 1.3 | 7.1 | 45.9 | 37.8 | 9.1 | 0 | 2.98 | 90% |
| H20 1.3N NaCl 1 wt Resin | 1 | 4.2 | 20 | 1.3 | 6.2 | 35 | 50 | 8.8 | 0 | 3.23 | 77% |
| **H20 1.3N NaCl 2 wt Resin** | **2** | **8.4** | **20** | **1.3** | **7.8** | **43.8** | **48.4** | **0** | **0** | **2.81** | **67%** |
| **H20 1.3N NaCl 3 wt Resin** | **3** | **12.6** | **20** | **1.3** | **10.8** | **56.9** | **32.3** | **0** | **0** | **2.43** | **47%** |

EP 4 137 160 A1

**Bold font in** Table 7 indicates conditions that removed 6-loaded species < 3 PA% (peak area%); the 8-loaded species were undetectable under these experimental conditions. "T0" refers to a residence time of 0 minutes (i.e. control experiment with no resin); "M30" refers to a residence time of 0.5 hours; "H4" refers to a residence time of 4 hours; "H20" refers to a residence time of 20 hours.

Table 8: UV Antibody 1-mc-MMAF Concentration Results as a Function of the Resin Treatment Purification Screen

| 0.5 Hours Residence Time | 0.5 Hours Residence Time | **Protein Recovery of 0 N NaCl** | **Protein Recovery of 0.6 N NaCl** | **Protein Recovery of 1.3 N NaCl** |
|---|---|---|---|---|
| **Resin Loading (Dry Weight Basis) vs. Protein Load** | **Resin Loading (Dry Weight Basis) vs 6-8 Load** | | | |
| **0** | [0] | **100%** | **99%** | **98%** |
| 0.5 | [2.1] | **97%** | 90% | 93% |
| 1 | [4.2] | **94%** | 86% | 81% |
| 2 | [8.4] | 85% | 74% | 68% |
| 3 | [12.6] | 78% | 63% | 55% |
| | | | | |
| | | | | |
| 4 Hours Residence Time | 4 Hours Residence Time | | | |
| **Resin Loading (Dry Weight Basis) vs. Protein Load** | **Resin Loading (Dry Weight Basis) vs 6-8 Load** | | | |
| 0 | 0 | **100%** | **99%** | **98%** |
| 0.5 | 2.1 | **101%** | 92% | 88% |
| 1 | 4.2 | **90%** | 86% | 80% |
| 2 | 8.4 | 84% | 70% | 64% |
| 3 | 12.6 | 72% | 59% | 48% |
| | | | | |
| 20 Hours Hour Residence Time | 20 Hours Hour Residence Time | | | |
| **Resin Loading (Dry Weight Basis) vs. Protein Load** | **Resin Loading (Dry Weight Basis) vs 6-8 Load** | | | |
| 0 | 0 | **100%** | **99%** | **98%** |
| 0.5 | 2.1 | **100%** | 91% | 90% |
| 1 | 4.2 | **95%** | 83% | 77% |
| 2 | 8.4 | 85% | 71% | 67% |
| 3 | 12.6 | 70% | 58% | 47% |

In sum, the resin titration screens describes in Tables 7 and 8 were performed to determine the impact of the resin load, NaCl concentration, and residence time on the purification process (DAR, protein concentration) obtained from the UF/DF purified Antibody 1-mc-MMAF ADC from Example 4.. The calculated amount of resin as shown in Table 7, depends on the drug load species to be removed from the crude distribution. A series of reaction conditions using Antibody 1-mcMMAF from Example 4 were tested as described below (referred to in Tables 7 and 8). Buffer A contains the following: 4.35g $K_2HPO_4$; 58.5g NaCl; 495 mL water (WFI); pH adjusted to 7.0 with 5 mL IN HCl.

*Reaction 1: 0 N NaCl; No Resin:*

1) Charge Antibody 1-mcMMAF (1.00 mL, 35 mg) into a 4 mL vial
2) Charge 0 mg Bu-HIC Resin
3) Shake
4) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50x).
5) Assay for UV protein concentration and perform HIC analysis.

*Reaction 2: 0 N NaCl; 0.5 wt Resin:*

1) Charge Antibody 1-mcMMAF (1.00 mL, 35 mg) into a 4 mL vial
2) Charge 17.5 mg Bu-HIC Resin
3) Shake
4) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50x).
5) Assay for UV protein concentration and perform HIC analysis.

*Reaction 3: 0 N NaCl; 1 wt Resin:*

1) Charge Antibody 1-mcMMAF (1.00 mL, 35 mg) into a 4 mL vial
2) Charge 350 mg Bu-HIC Resin
3) Shake
4) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50x).
5) Assay for UV protein concentration and perform HIC analysis.

*Reaction 4: 0 N NaCl; 2 wt Resin:*

1) Charge Antibody 1-mcMMAF (1.00 mL, 35 mg) into a 4 mL vial
2) Charge 70 mg Bu-HIC Resin
3) Shake
4) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50x).
5) Assay for UV protein concentration and perform HIC analysis.

*Reaction 5: 0 N NaCl; 3 wt Resin:*

1) Charge Antibody 1-mcMMAF (1.00 mL, 35 mg) into a 4 mL vial
2) Charge 105 mg Bu-HIC Resin
3) Shake
4) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50x).
5) Assay for UV protein concentration and perform HIC analysis.

*Reaction 6: 0.65 N NaCl; No Resin:*

1) Charge Antibody 1-mcMMAF (1.00 mL, 35 mg) into a 4 mL vial
2) Charge 0.195 mL of a 4 N NaCl / 0.05 M K$_2$HPO$_4$ pH 7 buffer *(Buffer A')*.
3) Charge 0 mg Bu-HIC Resin
4) Shake
5) Pull supernatant sample at 0.5, 4, and 20 hours (remove 50 μL through syringe filter, dilute 20 μL of this supernatant sample with 2N NaCl / 0.05 M K$_2$HPO$_4$ pH 7 buffer (Buffer A) to afford a 1/50X).
6) Assay for UV protein concentration and perform HIC analysis.

*Reaction 7: 0.65 N NaCl; 0.5 wt Resin:*

1) Charge Antibody 1-mcMMAF (1.00 mL, 35 mg) into a 4 mL vial
2) Charge 0.195 mL of a 4 N NaCl / 0.05 M K$_2$HPO$_4$ pH 7 buffer *(Buffer A')*.
3) Charge 17.5 mg Bu-HIC Resin
4) Shake
5) Pull supernatant sample at 0.5, 4, and 20 hours (remove 50 μL through syringe filter, dilute 20 μL of this supernatant sample with 2N NaCl / 0.05 M K$_2$HPO$_4$ pH 7 buffer (Buffer A) to afford a 1/50X).
6) Assay for UV protein concentration and perform HIC analysis.

*Reaction 8: 0.65 N NaCl; 1 wt Resin:*

1) Charge Antibody 1-mcMMAF (1.00 mL, 35 mg) into a 4 mL vial
2) Charge 0.195 mL of a 4 N NaCl / 0.05 M K$_2$HPO$_4$ pH 7 buffer *(Buffer A')*.
3) Charge 35 mg Bu-HIC Resin
4) Shake
5) Pull supernatant sample at 0.5, 4, and 20 hours (remove 50 μL through syringe filter, dilute 20 μL of this supernatant sample with 2N NaCl / 0.05 M K$_2$HPO$_4$ pH 7 buffer (Buffer A) to afford a 1/50X).
6) Assay for UV protein concentration and perform HIC analysis.

*Reaction 9: 0.65 N NaCl; 2 wt Resin:*

1) Charge Antibody 1-mcMMAF (1.00 mL, 35 mg) into a 4 mL vial
2) Charge 0.195 mL of a 4 N NaCl / 0.05 M K$_2$HPO$_4$ pH 7 buffer *(Buffer A')*.
3) Charge 70 mg Bu-HIC Resin
4) Shake
5) Pull supernatant sample at 0.5, 4, and 20 hours (remove 50 μL through syringe filter, dilute 20 μL of this supernatant sample with 2N NaCl / 0.05 M K$_2$HPO$_4$ pH 7 buffer (Buffer A) to afford a 1/50X).
6) Assay for UV protein concentration and perform HIC analysis.

*Reaction 10: 0.65 N NaCl; 3 wt Resin:*

1) Charge Antibody 1-mcMMAF (1.00 mL, 35 mg) into a 4 mL vial
2) Charge 0.195 mL of a 4 N NaCl / 0.05 M K$_2$HPO$_4$ pH 7 buffer *(Buffer A')*.
3) Charge 105 mg Bu-HIC Resin
4) Shake
5) Pull supernatant sample at 0.5, 4, and 20 hours (remove 50 μL through syringe filter, dilute 20 μL of this supernatant sample with 2N NaCl / 0.05 M K$_2$HPO$_4$ pH 7 buffer (Buffer A) to afford a 1/50X).
6) Assay for UV protein concentration and perform HIC analysis.

*Reaction 11: 1.3 N NaCl; No Resin:*

1) Charge Antibody 1-mcMMAF (1.00 mL, 35 mg) into a 4 mL vial
2) Charge 0.48 mL of a 4 N NaCl/0.05 M $K_2HPO_4$ pH 7 Buffer *(Buffer A')*.
3) Charge 0 mg Bu-HIC Resin
4) Shake
5) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50X).
6) Assay for UV protein concentration and perform HIC analysis.

*Reaction 12: 1.3N NaCl; 0.5 wt Resin:*

1) Charge Antibody 1-mcMMAF (1.00 mL, 35 mg) into a 4 mL vial
2) Charge 0.48 mL of a 4 N NaCl/0.05 M $K_2HPO_4$ pH 7 Buffer *(Buffer A')*.
3) Charge 17.5 mg Bu-HIC Resin
4) Shake
5) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50X).
6) Assay for UV protein concentration and perform HIC analysis.

*Reaction 13: 1.3N NaCl; 1 wt Resin:*

1) Charge Antibody 1-mcMMAF (1.00 mL, 35 mg) into a 4 mL vial
2) Charge 0.48 mL of a 4 N NaCl/0.05 M $K_2HPO_4$ pH 7 Buffer *(Buffer A')*.
3) Charge 35 mg Bu-HIC Resin
4) Shake
5) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50X).
6) Assay for UV protein concentration and perform HIC analysis.

*Reaction 14: 1.3N NaCl; 2 wt Resin:*

1) Charge Antibody 1-mcMMAF (1.00 mL, 35 mg) into a 4 mL vial
2) Charge 0.48 mL of a 4 N NaCl/0.05 M $K_2HPO_4$ pH 7 Buffer *(Buffer A')*.
3) Charge 70 mg Bu-HIC Resin
4) Shake
5) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50X).
6) Assay for UV protein concentration and perform HIC analysis.

*Reaction 15: 1.3N NaCl; 3 wt Resin:*

1) Charge Antibody 1-mcMMAF (1.00 mL, 35 mg) into a 4 mL vial
2) Charge 0.48 mL of a 4 N NaCl/0.05 M $K_2HPO_4$ pH 7 Buffer *(Buffer A')*.
3) Charge 105 mg Bu-HIC Resin
4) Shake
5) Pull supernatant sample at 0.5, 4, and 20 hours (remove 50 $\mu$L through syringe filter, dilute 20 $\mu$L of this supernatant sample with 2N NaCl / 0.05 M $K_2HPO_4$ pH 7 buffer (Buffer A) to afford a 1/50X).

6) Assay for UV protein concentration and perform HIC analysis.

*Resin Preparation and Calculations:*

Karl Fischer (KF) titration of resins:

Butyl = 72 wt% water (28% potency). Note all charges of resin are based on a dry weight basis.
A sample calculation of dry weight resin charge: 35 mg resin charge/0.28 dry weight potency= 125 mg of wet resin charge.

Generally, the potency of resin was calculated by 100% - w/w% of water from Karl Fisher analysis.

**Example 6: Purification of ADC Mixtures Having Average DARs of 2.7, 4 and 5.5**

The following example describes batch purification of several different ADC mixtures comprising either Antibody 1-vc-MMAE or Antibody 1-mc-MMAF having either an average DAR of 2.7 or a more heavily loaded average DAR of 5.5. Additionally, an ADC mixture comprising Antibody 1-vcMMAE with an average DAR of 4 is also described. More specifically, a screen was performed to determine the impact of the resin weight, NaCl concentration, on the purification process (DAR, protein concentration) of two differentially loaded ADCs (antibody 1-vc-MMAE and antibody 1-mc-MMAF) with varying amounts of 6 and 8 loaded species as inputs in the purification process. A series of reaction conditions were tested as described below.

The five crude ADC mixtures (1-5) that were used in Example 6 were prepared as described below. Four crude ADC mixtures (1) Antibody 1-vcMMAE DAR 2.7 (avg), (2) Antibody 1-mcMMAF DAR 2.7 (avg), (3) Antibody 1-vcMMAE DAR 5.5 (avg), and (4) Antibody 1-mcMMAF DAR 5.5 (avg) were prepared in accordance with the methods described in Example 1 and Figure 1 where the reduction of antibody 1 was achieved using TCEP (1.3 or 2.65 molar equivalents) and conjugation was achieved using and of either mc-MMAF or vcMMAE (3 or 6 equivalents) resulting in the preparation of antibody-1-vcMMAE DAR 2.7 (avg) and 5.5 (avg) ADC mixtures and antibody-1-mcMMAF DAR 2.7 (avg) and 5.5 (avg) ADC mixtures. Additionally, a fifth crude ADC mixture (5) Antibody 1-vcMMAE DAR 4 (avg) prepared in accordance of Example 1.

The screening procedure was performed according to the following protocol. First, a respective amount of wet Bu-HIC Resin (representatively prepared as described in Example 2 in the Materials and Method Section) was weighed into a 4 mL vial. The amount of resin was based on a few calculations. First, the amount of dry resin needed was based on the mass amount of 6 and 8 loaded species. The mass amount was calculated based on the crude ADC starting material solution as follows:

Mass amount of species to remove:

$$\Sigma(6 \text{ load} + 8 \text{ load}), \text{mg} = 20 \text{ mg of crude ADC } (1\text{-}5) \text{ x } (\Sigma(6 \text{ load pa\%} + 8 \text{ load pa\%}))/100$$

For example for Vial 13:

$$\Sigma(6 \text{ load} + 8 \text{ load}), \text{mg} = 20 \text{ mg of mAb x } (10.7/100) = 2.14 \text{ mg } \Sigma(6 \text{ load} + 8 \text{ load})$$

Next, the total mass amount of 6 load and 8 load was multiplied by the target weight of resin. For example for Vial 13:

$$5 \text{ weights of resin} = 5 \text{ x } 2.14 \text{ mg } \Sigma(6 \text{ load} + 8 \text{ load}) = 10.7 \text{ mg dry resin}$$

$$7.5 \text{ weights of resin} = 7.5 \text{ x } 2.14 \text{ mg } \Sigma(6 \text{ load} + 8 \text{ load}) = 16.1 \text{ mg dry resin}$$

$$10 \text{ weights of resin} = 10 \text{ x } 2.14 \text{ mg } \Sigma(6 \text{ load} + 8 \text{ load}) = 21.4 \text{ mg dry resin}$$

Lastly, the resin was corrected for water, sodium chloride, and $K_2HPO_4$ content. The inorganic salt correction was made because the resin was previously isolated by filtration and washed with 1.95M NaCl/0.05M $K_2HPO_4$ solution. ( The Bu-HIC resin was filtered and washed multiple times with 1.95M NaCl/0.05M $K_2HPO_4$ solution. The moisture content of the resin was determined by KF (Karl Fisher moisture titration) analysis at 59.0 w/w%. The w/w % concentration of the wash components (10.6 w/w% NaCl, 0.8w/w % $K_2HPO_4$, 88.6% Water) was then used to estimate the masses of NaCl and $K_2HPO_4$ in the wet resin (51.8g wet resin, 30.6g water, 3.6g NaCl, and 0.3g $K_2HPO_4$) which were then subtracted to calculate the dry resin amount (17.3g).)

For example for Vial 13 at 5 weights of dry resin:

10.7 mg dry resin / (0.334 mg dry resin/ mg wet resin) = 32.0 mg wet resin

Following the addition of the Bu-HIC resin, the crude ADC mixtures (1-5) (1.1 - 1.2 mL, 20 mg) was charged into a 4 mL vial. The volume of crude ADC solution was adjusted to target 20mg of total protein.

Next, a range of sodium chloride solutions was prepared. 0.55 - 0.6 mL, (approximately ½ the volume of ADC solution), of the respective molarity of sodium chloride solution/50mM $K_2PO_4$/pH 7 was charged into various vials. The initial concentration of NaCl solutions were 0 M, 1.95 M, 3.9 M, and 5.85 M at constant concentration of 50mM $K_2HPO_4$ at pH 7. After addition to the ADC solutions, the NaCl concentration was reduced to 0, 0.65, 1.3 and 1.95 M NaCl on account of the dilution. The vial contents (ADC of DAR 2.5 or 5.5 + salt solution at various concentrations) were then shaken overnight (approximately 20 hrs)

Following stirring overnight (>20 hours), a supernatant sample was taken (remove 0.6 mL through syringe filter, dilute 75 μL of this supernatant sample with 924 μL 1.95N NaCl / 0.05 M $K_2HPO_4$ pH 7 buffer). Subsequent HPLC-HIC analysis was performed to determine the PA% of each loaded species, as well as protein recovery. The results of the study are shown below in Tables 9 and 10.

## Table 9:  Summary of Distribution as Measured by HIC-HPLC of vc-MMAE[1]

| Vial | Input Σ(6L+8L), pa% | Salt Concentration, Molarity of NaCl | Resin load vs Σ(6L+8L) | 0 Load pa% | 2 Load pa% | 4 Load pa% | 6 Load pa% | 8 Load pa% | DAR-Overall | %Protein Recovery-Overall* |
|---|---|---|---|---|---|---|---|---|---|---|
| 13 | 10.7 | 0 | 5 | 16.4 | 51.6 | 30.9 | 1.0 | 0.0 | 2.35 | 106 |
| 14 | 10.7 | 0 | 7.5 | 16.9 | 53.5 | 29.2 | 0.4 | 0.0 | 2.28 | 102 |
| 15 | 10.7 | 0 | 10 | 17.8 | 55.7 | 26.2 | 0.2 | 0.0 | 2.20 | 97 |
| 16 | 10.7 | 0.65 | 5 | 16.9 | 52.5 | 29.5 | 1.0 | 0.0 | 2.32 | 104 |
| 17 | 10.7 | 0.65 | 7.5 | 18.0 | 55.0 | 26.8 | 0.2 | 0.0 | 2.20 | 97 |
| 18 | 10.7 | 0.65 | 10 | 18.8 | 57.8 | 22.6 | 0.3 | 0.4 | 2.13 | 93 |

[1] Odd species were not included

| Vial | Input Σ(6L+8L), pa% | Salt Concentration, Molarity of NaCl | Resin load vs Σ(6L+8L) | 0 Load pa% | 2 Load pa% | 4 Load pa% | 6 Load pa% | 8 Load pa% | DAR-Overall | %Protein Recovery-Overall* |
|---|---|---|---|---|---|---|---|---|---|---|
| 19 | 10.7 | 1.3 | 5 | 17.5 | 52.9 | 27.9 | 0.6 | 1.0 | 2.30 | 100 |
| 20 | 10.7 | 1.3 | 7.5 | 20.2 | 55.2 | 23.3 | 0.6 | 0.7 | 2.13 | 86 |
| 21 | 10.7 | 1.3 | 10 | 20.0 | 57.9 | 21.2 | 0.4 | 0.5 | 2.08 | 88 |
| 22 | 10.7 | 1.95 | 5 | 25.2 | 52.2 | 18.6 | 1.5 | 2.5 | 2.08 | 69 |
| 23 | 10.7 | 1.95 | 7.5 | 19.2 | 55.1 | 24.8 | 0.3 | 0.6 | 2.17 | 92 |
| 24 | 10.7 | 1.95 | 10 | 21.9 | 58.2 | 18.8 | 0.5 | 0.5 | 2.00 | 77 |
| 37 | 60.2 | 0 | 5 | 1.4 | 26.8 | 70.5 | 1.3 | 0.0 | 3.41 | 39 |
| 38 | 60.2 | 0 | 7.5 | 2.9 | 52.2 | 41.2 | 1.2 | 2.4 | 2.94 | 21 |
| 39 | 60.2 | 0 | 10 | 6.1 | 89.0 | 4.9 | 0.0 | 0.0 | 2.00 | 10 |
| 40 | 60.2 | 0.65 | 5 | 2.0 | 30.7 | 65.8 | 0.8 | 0.7 | 3.30 | 31 |
| 41 | 60.2 | 0.65 | 7.5 | 6.9 | 87.7 | 5.4 | 0.0 | 0.0 | 1.98 | 9 |
| 42 | 60.2 | 0.65 | 10 | 21.8 | 78.2 | 0.0 | 0.0 | 0.0 | 1.47 | 3 |
| 43 | 60.2 | 1.3 | 5 | 7.5 | 89.7 | 1.0 | 1.8 | 0.0 | 2.60 | 21 |
| 44 | 60.2 | 1.3 | 7.5 | 44.3 | 55.7 | 0.0 | 0.0 | 0.0 | 1.09 | 1 |
| 45 | 60.2 | 1.3 | 10 | 100.0 | 0.0 | 0.0 | 0.0 | 0.0 | 0.00 | 0 |
| 46 | 60.2 | 1.95 | 5 | 1.9 | 28.3 | 68.4 | 0.7 | 0.7 | 3.37 | 20 |
| 47 | 60.2 | 1.95 | 7.5 | 100.0 | 0.0 | 0.0 | 0.0 | 0.0 | 0.00 | 0 |
| 48 | 60.2 | 1.95 | 10 | 0.00 | 0.00 | 0.00 | 0.00 | 0.00 | 0.00 | 0 |
| 49 | 25.9 | 0 | 5 | 7.0 | 44.4 | 47.0 | 1.6 | 0.0 | 2.91 | 88 |
| 50 | 25.9 | 0 | 7.5 | 8.1 | 50.6 | 41.3 | 0.0 | 0.0 | 2.68 | 75 |
| 51 | 25.9 | 0 | 10 | 9.3 | 58.2 | 32.5 | 0.0 | 0.0 | 2.49 | 66 |
| 52 | 25.9 | 0.65 | 5 | 7.5 | 46.2 | 45.9 | 0.3 | 0.0 | 2.81 | 82 |
| 53 | 25.9 | 0.65 | 7.5 | 8.9 | 53.8 | 37.3 | 0.0 | 0.0 | 2.59 | 69 |

| Vial | Input Σ(6L+8L), pa% | Salt Concentration, Molarity of NaCl | Resin load vs Σ(6L+8L) | 0 Load pa% | 2 Load pa% | 4 Load pa% | 6 Load pa% | 8 Load pa% | DAR-Overall | %Protein Recovery-Overall* |
|---|---|---|---|---|---|---|---|---|---|---|
| 54 | 25.9 | 0.65 | 10 | 11.4 | 66.6 | 22.0 | 0.0 | 0.0 | 2.24 | 55 |
| 55 | 25.9 | 1.3 | 5 | 8.1 | 47.0 | 44.3 | 0.5 | 0.0 | 2.76 | 76 |
| 56 | 25.9 | 1.3 | 7.5 | 9.9 | 54.5 | 35.6 | 0.0 | 0.0 | 2.53 | 63 |
| 57 | 25.9 | 1.3 | 10 | 14.3 | 68.8 | 16.9 | 0.0 | 0.0 | 2.07 | 43 |
| 58 | 25.9 | 1.95 | 5 | 8.3 | 46.9 | 43.9 | 0.8 | 0.0 | 2.77 | 74 |
| 59 | 25.9 | 1.95 | 7.5 | 10.2 | 55.1 | 34.7 | 0.0 | 0.0 | 2.50 | 58 |
| 60 | 25.9 | 1.95 | 10 | 14.2 | 72.1 | 13.7 | 0.0 | 0.0 | 2.02 | 40 |

* Protein recovery was calculated using UV by HPLC at 280 nm.

Table 10: Summary of Distribution as Measured by HIC-HPLC of mc-MMAF[2]

| Vial | Input Σ(6L+8L), pa% | Salt Concentration, Molarity of NaCl | Resin load vs Σ(6L+8L) | 0 Load pa% @280nm | 2 Load pa% @280nm | 4 Load pa% @280nm | 6 Load pa% @280nm | 8 Load pa% @280nm | DAR-Overall | %Protein Recovery-Overall* |
|---|---|---|---|---|---|---|---|---|---|---|
| 1 | 9.4 | 0 | 5 | 17.8 | 37.2 | 31.3 | 6.7 | 0.9 | 2.64 | 97 |
| 2 | 9.4 | 0 | 7.5 | 17.8 | 37.8 | 31.6 | 6.0 | 0.6 | 2.61 | 97 |
| 3 | 9.4 | 0 | 10 | 18.1 | 38.3 | 31.4 | 5.4 | 0.2 | 2.55 | 95 |

| Vial | Input Σ(6L+8L), pa% | Salt Concentration, Molarity of NaCl | Resin load vs Σ(6L+8L) | 0 Load pa% @280nm | 2 Load pa% @280nm | 4 Load pa% @280nm | 6 Load pa% @280nm | 8 Load pa% @280nm | DAR-Overall | %Protein Recovery-Overall* |
|---|---|---|---|---|---|---|---|---|---|---|
| 4 | 9.4 | 0.65 | 5 | 18.1 | 39.2 | 32.4 | 5.1 | 0.2 | 2.55 | 97 |
| 5 | 9.4 | 0.65 | 7.5 | 18.5 | 39.8 | 32.2 | 3.6 | 0.4 | 2.49 | 95 |
| 6 | 9.4 | 0.65 | 10 | 18.6 | 40.0 | 32.2 | 3.0 | 0.5 | 2.47 | 95 |
| 7 | 9.4 | 1.3 | 5 | 19.4 | 42.3 | 33.9 | 4.5 | 0.3 | 2.50 | 95 |
| 8 | 9.4 | 1.3 | 7.5 | 19.7 | 42.3 | 30.3 | 2.6 | 0.3 | 2.37 | 90 |
| 9 | 9.4 | 1.3 | 10 | 20.5 | 45.3 | 28.9 | 1.8 | 0.2 | 2.31 | 86 |
| 10 | 9.4 | 1.95 | 5 | 19.0 | 40.8 | 31.6 | 3.8 | 0.2 | 2.45 | 95 |
| 11 | 9.4 | 1.95 | 7.5 | 19.9 | 42.7 | 30.8 | 1.9 | 0.1 | 2.33 | 89 |
| 12 | 9.4 | 1.95 | 10 | 21.2 | 45.2 | 28.1 | 1.2 | 0.1 | 2.21 | 82 |
| 25 | 56.8 | 0 | 5 | 1.7 | 9.6 | 36.3 | 29.3 | 14.1 | 4.93 | 84 |
| 26 | 56.8 | 0 | 7.5 | 0.8 | 10.8 | 41.8 | 27.4 | 9.5 | 4.69 | 72 |
| 27 | 56.8 | 0 | 10 | 0.8 | 11.9 | 46.1 | 26.9 | 4.4 | 4.45 | 61 |
| 28 | 56.8 | 0.65 | 5 | 0.9 | 12.0 | 45.4 | 25.3 | 4.7 | 4.43 | 67 |
| 29 | 56.8 | 0.65 | 7.5 | 1.0 | 16.5 | 59.4 | 12.6 | 0.5 | 3.89 | 45 |
| 30 | 56.8 | 0.65 | 10 | 1.6 | 20.1 | 64.1 | 5.3 | 0.0 | 3.59 | 35 |
| 31 | 56.8 | 1.3 | 5 | 1.0 | 15.4 | 54.5 | 16.7 | 2.2 | 4.08 | 51 |
| 32 | 56.8 | 1.3 | 7.5 | 1.6 | 22.8 | 64.7 | 2.9 | 0.0 | 3.48 | 30 |
| 33 | 56.8 | 1.3 | 10 | 3.2 | 36.2 | 51.8 | 0.0 | 0.0 | 3.01 | 18 |
| 34 | 56.8 | 1.95 | 5 | 1.5 | 18.9 | 57.0 | 9.5 | 3.6 | 3.88 | 38 |
| 35 | 56.8 | 1.95 | 7.5 | 3.0 | 33.1 | 57.2 | 0.0 | 0.0 | 3.11 | 18 |
| 36 | 56.8 | 1.95 | 10 | 7.9 | 58.1 | 22.6 | 0.0 | 0.0 | 2.29 | 7 |

* Protein recovery was calculated using UV by HPLC at 280 nm. ` Odd species were not included.

In sum, the resin titration screen described in Example 6, Tables 9 and 10 was performed to determine the impact of the resin load and NaCl concentration on the purification process (DAR, protein concentration) obtained from the crude Antibody-1-mcMMAF and Antibody-1-vcMMAE ADC mixtures (1) - (5) possessing a DAR (avg) range of 2.7 to 5.5. The calculated amount of resin as shown in Tables 9 and 10, depends on the drug load species to be removed from the crude distribution. For example, a resin weight of approximately 5 to 10 times that of the 6 and 8 drug load species was proven to be effective for removing these species from the crude reaction mixture.

### Example 7: Batch and Flow Through Purification of Antibody 1-vc-MMAE

*Batch Purification Method*

Generation of a crude distribution of an ADC, *i.e.*, Antibody 1-vc-MMAE, was performed in accordance with the methods described in Example 1.

The reaction mixture was then treated with Bu-HIC resin that was previously washed with 50 mM potassium phosphate, 2 M NaCl, and buffer at a pH of 6.8. The resin / reaction mixture was subsequently stirred, and monitored by analytical hydrophobic interaction chromatography for removal of drug conjugate products (according to the methods described in the previous examples). Results of this additional experiment describing batch purification of Antibody 1-vc-MMAE are described in Table 11. Retention time referred to in Table 11 is the time for which the compound takes to elute off of the analytical HPLC analysis.

Table 11: Peak Area % Results (PA%) of the Reaction Mixture Before (Labeled "Reaction" Below) and After Purification (Labeled "Purified" Below)

| HIC pa% results @ 280nm | | | |
|---|---|---|---|
| Retention time (min) | DAR | Reaction | Purified |
| 6.4 | 0 | 5.15 | 6.54 |
| 7.2 | 1 | 0.36 | 1.46 |
| 8.4 | 2 | 27.26 | 33.37 |
| 10.2 | 4 | 40.74 | 50.54 |
| 11.3 | 5 | 6.03 | 5.86 |
| 11.8 | 6 | 11.45 | 1.67 |
| 12.2 | 7 | 1.67 | 0.55 |
| 12.4 | 7 | 2.00 | |
| 12.7 | 8 | 5.32 | |
| DAR(avg) = | | 3.9 | 3.1 |
| | E4/E2 = | 1.5 | 1.5 |
| Σ (>E6), pa% 280nm = | | 20.4 | 2.2 |

As described in Table 11, batch purification of Antibody-1-vc-MMAE resulted in a lower average DAR relative to the initial reaction mixture.

*Flow Through Purification Method*

Alternatively, the purification of Antibody 1-vc-MMAE may be performed using a flow through purification mode.

Flow through purification was generally performed according to the following method: A two liter batch of Tosoh Bioscience Butyl 600 M resin was made. Resin was filtered into a 2 L sintered funnel (note the funnel had been previously washed with IPA and dried). Filtered resin was washed with 2 x 2 L of 50 mM potassium phosphate, 2 M NaCl phosphate buffer at pH 6.8. The resin potency was determined to be 27% by Karl Fisher moisture analysis (analysis showed the presence of 73 w/w% water; note in this example the modest amount of inorganic residue (NaCl and $K_2HPO_4$) was not used in calculating the potency of the resin).

Reduction / conjugation methods described in Example 1 were used, starting with 134.9 g of Antibody 1. One change relative to the protocol described in Example 1 was that 2.15 equiv TCEP was used (which resulted in a slightly higher average DAR). The process resulted in 6-8 load species that were 33.8 pa%. Thus 45.6 g of 6-8 load species was present in the crude reaction mixture.

Using a 5x loading of hydrophobic resin, 228.5 g dry weight hydrophobic resin was required (*i.e.* 45.6 g of 6-8 load species multiplied by 5 = 228.5 g of potency adjusted resin). At a resin potency of 27% (note: potency of resin is calculated by 100% - w/w% of water from Karl Fisher analysis), 856 g wet weight resin equivalent to 228.5 grams of potency adjusted resin (i.e. calculation: 228.5 grams of potency adjusted resin / (27 grams dry-weight resin/100 grams wet resin)) = 856 g wet resin required) was loaded into a stainless steel column that was 4 inches x 7 inches. The crude reaction mixture was then pumped from a sterile 20 L carboy over the resin bed through a pressure sensor and a peristaltic pump using size 35 Pharmed tubing and into a second 20 L sterile carboy at 185 ml/min. The collected filtrate was then pumped across the resin bed again, collecting the desired ADC mixture containing the lower DAR species in the final filtrate. The flow through process used was a double pass process.

The resin bed was then washed numerous times to remove residual unbound lower DAR species while leaving the high DAR species (drug loads 6-8) bound to the resin. Specifically, first, the resin bed was washed with 1200 mL 1 N NaCl (95 mS) prepared by diluting 600 ml of the 50 mM potassium phosphate, 2 M NaCl to 1200ml with WFI. The resin bed was then washed with 1200 ml 0.75 N NaCl (71 mS) prepared by diluting 450 ml of the 50 mM potassium phosphate, 2 M NaCl to 1200 mL with WFI. A third wash was performed using 1200 mL 0.5 N NaCl (50 mS) prepared by diluting 300 ml of the 50 mM potassium phosphate, 2 M NaCl with 900 ml WFI. A fourth wash was performed using 1200 mL 0.25 N NaCl (26 mS) prepared by diluting 150 ml of the 50 mM potassium phosphate, 2 M NaCl to 1200 ml with WFI. The filtrate from resin washes was largely collected and combined with the final filtrate from the above flow through process, affording the bulk (*i.e.*, final filtrate + washes).

Notably, the washing of the resin bed is optional, as purified ADCs having a DAR of 2-4 were obtained in the final filtrate from the initial multi-pass procedure described above. The first wash provided about 10% recovery from the wash, while the subsequent washes resulted in about 1-2% recovery.

The bulk was concentrated by tangential flow filtration (TFF) to approximately 1200 g of concentrated ADC solution and then exchanged with 10 diavolumes of 15 mM Histidine buffer at pH 6 to yield the desired DAR 0 - 4 species of Antibody 1-vc-MMAE at a final protein concentration of 35 mg/mL (isolated 81 grams, 66% yield, 91% recovery vs DAR 0-4). Thus, the flow through purification method was successful at separating DAR species, *i.e.*, high load species of 6-8, away from the lower DAR species (described in more detail below in Table 12).

A comparison of the batch purification mode to the flow through purification mode (Table 12) was performed. In both cases, the loading of resin vs. protein was 5 weights of resin vs. the mass of the high load DAR 6-8 species of Antibody 1-vc-MMAE. Although there was a slight variance of the relative amount of the individual species (due to the slightly higher equivalents of TCEP used in the flow through experiment example), the effectiveness of removing the higher DAR species of both methods was comparable. The material referred to in Table 12 in the "Flow Through Method" column includes the combined filtrate from the flow through method and the material from the washes (referred to collectively as "bulk").

Table 12. Comparison of Batch vs Flow-Through Methods

| HIC PA% results @ 280 nm | | | |
|---|---|---|---|
| Retention time, (min) | DAR | Purified (Batch) | Purified (Flow Through Method) |
| 4.3 | 0 | 10.7 | 7.9 |
| 5.9 | 2 | 46.2 | 41.6 |
| 7.5 | 4 | 41.7 | 47.0 |
| 8.8 | 6 | 1.4 | 3.5 |
| 9.9 | 8 | Not detected | Not detected |
| | DAR = | 2.7 | 2.9 |

In conclusion, both the batch and flow through purification methods were used to enrich for ADCs having DARs of 2-4. Both purification methods relied on the ratio of protein (ADC) weight (coupled with fraction of high drug load species) to the load of resin used, where a hydrophobic resin weight which is 5 to 6 times the weight of the drug loaded species of 6-8 (6 or more) in the ADC mixture resulted in substantially reduced levels of ADCs having a DAR of 6-8. As described in Table 12, both processes resulted in compositions comprising at least 95% ADCs having a DAR of 4 or less or compositions comprising ADCs with less than 4% of the drug loaded species or 6 or more.

It is understood that the examples and embodiments described herein are for illustrative purposes only and that various modifications or changes in light thereof will be suggested to persons skilled in the art and are to be included within the spirit and purview of the appended claims. All publications, patents, and patent applications cited herein are hereby incorporated by reference in their entirety for all purposes.

Preferred embodiments of the present invention are described below and are referred to as embodiments E1 to E50

1. A method of obtaining a composition comprising Antibody Drug Conjugates (ADCs), said method comprising

contacting an ADC mixture comprising a drug loaded species of 4 or less and a drug loaded species of 6 or more with a hydrophobic resin, wherein the amount of hydrophobic resin contacted with the ADC mixture is sufficient to allow binding of the drug loaded species of 6 or more to the resin but does not allow significant binding of the drug loaded species of 4 or less; and
removing the hydrophobic resin from the ADC mixture, such that the composition comprising ADCs is obtained, wherein the composition comprises less than 15% of the drug loaded species of 6 or more, and
wherein the ADC comprises an antibody conjugated to an auristatin.

2. The method of E1, wherein the composition comprises less than 10% of the drug loaded species of 6 or more.

3. The method of E1, wherein the composition comprises 5% or less of the drug loaded species of 6 or more.

4. The method of any one of E1-3, wherein the hydrophobic resin weight is 3 to 12 times the weight of the drug loaded species of 6 or more in the ADC mixture.

5. The method of any one of E1-3, wherein the hydrophobic resin weight is 4 to 8 times the weight of the drug loaded species of 6 or more in the ADC mixture.

6. The method of any one of E1-3, wherein the hydrophobic resin weight is 5 to 10 times the weight of the drug loaded species of 6 or more in the ADC mixture.

7. The method of any one of E1-3, wherein the hydrophobic resin weight is 6 to 12 times the weight of the drug loaded species of 6 or more in the ADC mixture, and wherein the ADC mixture comprises between 0 to 1 N NaCl, or an equivalent ionic strength thereof.

8. The method of E4, wherein the hydrophobic resin weight is 3 to 6 times the weight of the drug loaded species of 6 or more in the ADC mixture, and wherein the ADC mixture comprises between 1 to 2N NaCl, or an equivalent ionic strength thereof.

9. The method of any one of E1-3, wherein the hydrophobic resin weight is 3 to 7 times the weight of the drug loaded species of 6 or more in the ADC mixture, and wherein the auristatin is monomethylauristatin E (MMAE).

10. The method of any one of E1-3, wherein the hydrophobic resin weight is 5 to 10 times the weight of the drug loaded species of 6 or more in the ADC mixture, and wherein the auristatin is monomethylauristatin F (MMAF).

11. The method of any one of E1-3, wherein the hydrophobic resin weight is 3 to 7 times the weight of the drug loaded species of 6 or more in the ADC mixture, and wherein the auristatin is monomethylauristatin E (MMAE).

12. A method of producing a composition comprising ADCs with an average Drug-to-Antibody Ratio (DAR) of 4.5 or less and comprising less than 15% undesired ADCs, said method comprising

contacting an ADC mixture with a hydrophobic resin, wherein the amount of hydrophobic resin contacted with the ADC mixture is sufficient to allow binding of the undesired ADCs; and
removing the hydrophobic resin from the ADC mixture, such that the composition with an average DAR of 4.5 or less and comprising less than 15% undesired ADCs is produced,
wherein the ADC comprises an antibody conjugated to an auristatin.

13. The method of E12, wherein the composition with an average DAR of 4.5 or less comprises less than 10% undesired ADCs.

14. The method of E13, wherein the undesired ADCs are 6 and 8 drug loaded species.

15. The method of any one of E12-14, wherein the amount of hydrophobic resin added to the ADC mixture is a resin weight which is 3 to 12 times the weight of the undesired ADCs in the ADC mixture.

16. The method of any one of E12-14, wherein the amount of hydrophobic resin added to the ADC mixture is a resin weight which is 4 to 8 times the weight of the drug loaded species of 6 or more in the ADC mixture.

17. The method of any one of E12-14, wherein the amount of hydrophobic resin added to the ADC mixture is a resin weight which is 5 to 7 times the weight of the drug loaded species of 6 or more in the ADC mixture.

18. The method of any one of E12-14, wherein the hydrophobic resin weight is 6 to 12 times the weight of the drug loaded species of 6 or more in the ADC mixture and wherein the ADC mixture comprises between 0 to 1 N NaCl, or an equivalent ionic strength thereof.

19. The method of any one of E12-14, wherein the hydrophobic resin weight is 3 to 6 times the weight of the drug loaded species of 6 or more in the ADC mixture and wherein the ADC mixture comprises between 1 to 2 N NaCl, or an equivalent ionic strength thereof.

20. The method of any one of E12-14, wherein the hydrophobic resin weight is 3 to 7 times the weight of the drug loaded species of 6 or more in the ADC mixture, and wherein the auristatin is monomethylauristatin E (MMAE).

21. The method of any one of E12-14, wherein the hydrophobic resin weight is 5 to 10 times the weight of the drug loaded species of 6 or more in the ADC mixture, and wherein the auristatin is monomethylauristatin F (MMAF).

22. The method of any one of E12-14, wherein the hydrophobic resin weight is 3 to 7 times the weight of the drug loaded species of 6 or more in the ADC mixture, and wherein the auristatin is monomethylauristatin E (MMAE).

23. The method of any one of E13-23, wherein the composition has an average DAR of 4 or less.

24. The method of any one of E13-23, wherein the composition has an average DAR of 3.5 or less.

25. The method of any one of E13-23, wherein the composition has an average DAR of 3 or less.

26. The method of any one of E13-23, wherein the composition has an average DAR of 2.5 or less.

27. The method of any one of E1-26, wherein the ADC mixture was obtained following an ultrafiltration /diafiltration process.

28. The method of any one of E1-27, wherein the hydrophobic resin is a butyl hydrophobic resin.

29. The method of any one of E1-28, which is a batch process, a circulation process or a flow through process.

30. The method of any one of E1-29, wherein the ADC comprises an anti-Epidermal Growth Factor Receptor (EGFR) antibody.

31. The method of E30, wherein the anti-EGFR antibody comprises a light chain variable region comprising a Complementarity Determining Region 1 (CDR1), CDR2, and CDR3 domain comprising the amino acid sequence as set forth in SEQ ID NO: 7, SEQ ID NO: 8, and SEQ ID NO: 9, respectively, and comprises a heavy chain variable region comprising a CDR1, CDR2, and CDR3 domain comprising the amino acid sequence as set forth in SEQ ID NO:2, SEQ ID NO:3, and SEQ ID NO: 4.

32. The method of E30, wherein the anti-EGFR antibody comprises a light chain variable region comprising the amino acid sequence set forth in SEQ ID NO: 6 and a heavy chain variable region comprising the amino acid sequence set forth in SEQ ID NO: 1.

33. The method of any one of E1-32, wherein the auristatin is either monomethylauristatin E (MMAE) or monomethylauristatin F (MMAF).

34. The method of E33, wherein the MMAE is conjugated to the antibody via a valine-citrulline (vc) linker.

35. The method of E33, wherein the MMAF is conjugated to the antibody via a malemidocaproyl (mc) linker.

36. A composition obtained through any one of the methods of E1-35.

37. A method of treating cancer in a subject comprising administering the composition of E36 to the subject such that cancer is treated.

38. A composition comprising ADCs, wherein 70% of ADCs present have a drug loaded species of 4 or less, and wherein the ADC comprises an anti-EGFR antibody and an auristatin.

39. The composition of E38, wherein 75% of ADCs present have a drug loaded species of 4 or less.

40. The composition of E38, wherein 80% of ADCs present have a drug loaded species of 4 or less.

41. The composition of E38, wherein 85% of ADCs present have a drug loaded species of 4 or less.

42. The composition of E38, wherein 90% of ADCs present have a drug loaded species of 4 or less.

43. The composition of E38, wherein 95% of ADCs present have a drug loaded species of 4 or less.

44. The composition of any one of E38-43, wherein the anti-EGFR antibody comprises a light chain variable region comprising a Complementarity Determining Region 1 (CDR1), CDR2, and CDR3 domain comprising the amino acid sequence as set forth in SEQ ID NO: 7, SEQ ID NO: 8, and SEQ ID NO: 9, respectively, and comprises a heavy chain variable region comprising a CDR1, CDR2, and CDR3 domain comprising the amino acid sequence as set forth in SEQ ID NO: 2, SEQ ID NO: 3, and SEQ ID NO: 4.

45. The composition of any one of E38-43, wherein the anti-EGFR antibody comprises a light chain variable region comprising the amino acid sequence set forth in SEQ ID NO: 6 and a heavy chain variable region comprising the amino acid sequence set forth in SEQ ID NO: 1.

46. The composition of any one of E38-45, wherein the auristatin is either monomethylauristatin E (MMAE) or monomethylauristatin F (MMAF).

47. The composition of E46, wherein the MMAE is conjugated to the antibody via a valine-citrulline (vc) linker..

48. The composition of E46, wherein the MMAF is conjugated to the antibody via a malemidocaproyl (mc) linker.

49. A pharmaceutical composition comprising the composition of any one of E38-48 and a pharmaceutically acceptable carrier.

50. A method of treating cancer in a subject comprising administering the pharmaceutical composition of E49 to the subject, such that cancer is treated.

**Claims**

1. A method of obtaining a composition comprising Antibody Drug Conjugates (ADCs), said method comprising

   contacting an ADC mixture comprising a drug loaded species of 4 or less and a drug loaded species of 6 or more with a hydrophobic resin, wherein the amount of hydrophobic resin contacted with the ADC mixture is sufficient to allow binding of the drug loaded species of 6 or more to the resin but does not allow significant binding of the drug loaded species of 4 or less; and
   removing the hydrophobic resin from the ADC mixture, such that the composition comprising ADCs is obtained, wherein the composition comprises less than 15% of the drug loaded species of 6 or more, and
   wherein the ADC comprises an antibody conjugated to an auristatin.

2. The method of claim 1, wherein the composition comprises less than 10% of the drug loaded species of 6 or more, optionally wherein the composition comprises 5% or less of the drug loaded species of 6 or more.

3. The method of claim 1 or claim 2, wherein the hydrophobic resin weight is:

(a) 3 to 12 times the weight of the drug loaded species of 6 or more in the ADC mixture;
(b) 4 to 8 times the weight of the drug loaded species of 6 or more in the ADC mixture;
(c) 5 to 10 times the weight of the drug loaded species of 6 or more in the ADC mixture;
(d) 5 to 10 times the weight of the drug loaded species of 6 or more in the ADC mixture, and wherein the auristatin is monomethylauristatin F (MMAF);
(e) 3 to 7 times the weight of the drug loaded species of 6 or more in the ADC mixture, and wherein the auristatin is monomethylauristatin E (MMAE);
(f) 6 to 12 times the weight of the drug loaded species of 6 or more in the ADC mixture, and wherein the ADC mixture comprises between 0 to 1 N NaCl, or an equivalent ionic strength thereof; or
(g) 3 to 6 times the weight of the drug loaded species of 6 or more in the ADC mixture, and wherein the ADC mixture comprises between 1 to 2N NaCl, or an equivalent ionic strength thereof.

4. A method of producing a composition comprising ADCs with an average Drug-to-Antibody Ratio (DAR) of 4.5 or less and comprising less than 15% undesired ADCs, said method comprising

contacting an ADC mixture with a hydrophobic resin, wherein the amount of hydrophobic resin contacted with the ADC mixture is sufficient to allow binding of the undesired ADCs; and
removing the hydrophobic resin from the ADC mixture, such that the composition with an average DAR of 4.5 or less and comprising less than 15% undesired ADCs is produced,
wherein the ADC comprises an antibody conjugated to an auristatin.

5. The method of claim 4, wherein the composition with an average DAR of 4.5 or less comprises less than 10% undesired ADCs, optionally wherein the undesired ADCs are 6 and 8 drug loaded species.

6. The method of claim 4 or claim 5, wherein the amount of hydrophobic resin added to the ADC mixture is a resin weight which is:

(a) 3 to 12 times the weight of the undesired ADCs in the ADC mixture;
(b) 4 to 8 times the weight of the drug loaded species of 6 or more in the ADC mixture;
(c) 5 to 7 times the weight of the drug loaded species of 6 or more in the ADC mixture;
(d) 6 to 12 times the weight of the drug loaded species of 6 or more in the ADC mixture and wherein the ADC mixture comprises between 0 to 1 N NaCl, or an equivalent ionic strength thereof;
(e) 3 to 6 times the weight of the drug loaded species of 6 or more in the ADC mixture and wherein the ADC mixture comprises between 1 to 2 N NaCl, or an equivalent ionic strength thereof;
(f) 3 to 7 times the weight of the drug loaded species of 6 or more in the ADC mixture, and wherein the auristatin is monomethylauristatin E (MMAE); or
(g) 5 to 10 times the weight of the drug loaded species of 6 or more in the ADC mixture, and wherein the auristatin is monomethylauristatin F (MMAF).

7. The method of claim 5 or 6, wherein the composition has an average DAR of 4 or less, 3.5 or less, 3 or less, or 2.5 or less.

8. The method of any one of claims 1-7, wherein:

(a) the ADC mixture was obtained following an ultrafiltration /diafiltration process;
(b) the hydrophobic resin is a butyl hydrophobic resin; and/or
(c) the method is a batch process, a circulation process or a flow through process.

9. The method of any one of claims 1-8, wherein the ADC comprises an anti-Epidermal Growth Factor Receptor (EGFR) antibody.

10. The method of claim 9, wherein the anti-EGFR antibody comprises:

(a) a light chain variable region comprising a Complementarity Determining Region 1 (CDR1), CDR2, and CDR3 domain comprising the amino acid sequence as set forth in SEQ ID NO: 7, SEQ ID NO: 8, and SEQ ID NO: 9, respectively, and comprises a heavy chain variable region comprising a CDR1, CDR2, and CDR3 domain comprising the amino acid sequence as set forth in SEQ ID NO: 2, SEQ ID NO: 3, and SEQ ID NO: 4; or
(b) a light chain variable region comprising the amino acid sequence set forth in SEQ ID NO: 6 and a heavy chain variable region comprising the amino acid sequence set forth in SEQ ID NO: 1.

11. The method of any one of claims 1-10, wherein the auristatin is either monomethylauristatin E (MMAE) or monomethylauristatin F (MMAF), optionally wherein the MMAE is conjugated to the antibody via a valine-citrulline (vc) linker or a malemidocaproyl (mc) linker.

12. A composition obtained through any one of the methods of claims 1-11.

13. A composition comprising ADCs, wherein 70%, 75%, 80%, 85%, 90% or 95% of ADCs present have a drug loaded species of 4 or less, and wherein the ADC comprises an anti-EGFR antibody and an auristatin.

14. The composition of claim 13, wherein the anti-EGFR antibody comprises:

(a) a light chain variable region comprising a Complementarity Determining Region 1 (CDR1), CDR2, and CDR3 domain comprising the amino acid sequence as set forth in SEQ ID NO: 7, SEQ ID NO: 8, and SEQ ID NO: 9, respectively, and comprises a heavy chain variable region comprising a CDR1, CDR2, and CDR3 domain comprising the amino acid sequence as set forth in SEQ ID NO: 2, SEQ ID NO: 3, and SEQ ID NO: 4; or
(b) a light chain variable region comprising the amino acid sequence set forth in SEQ ID NO: 6 and a heavy chain variable region comprising the amino acid sequence set forth in SEQ ID NO: 1.

15. The composition of claim 13 or claim 14, wherein the auristatin is either monomethylauristatin E (MMAE) or monomethylauristatin F (MMAF ), optionally wherein the MMAE is conjugated to the antibody via a valine-citrulline (vc) linker or a malemidocaproyl (mc) linker.

Figure 1

# Figure 2

Absorbance at 280 nm (mAU)

# Figure 3

1) Buffer Exchange PBSE

2) TCEP
1.3 equiv. or
2.05 equiv. or
2.65 equiv.

$mAb\text{-}(SH)_n$

n= 2,4,6,8

1) vc-MMAE or mcMMAF

2) N(Ac)-Cysteine

$mAb\text{-}(SR)_n$

n= 2,4,6,8

**Antibody 1-vcMMAE DAR (avg) 2.7, 4, 5.5 or**
**Antibody 1-mcMMAF DAR (avg) 2.7, 5.5**

**ADC Mixtures (1)-(5)**

HIC Resin
See Example 6
Table 9 - 10

$mAb\text{-}(SR)_n$

n= 2,4

EP 4 137 160 A1

Europäisches Patentamt

European Patent Office

Office européen des brevets

# EUROPEAN SEARCH REPORT

**Application Number**

EP 22 17 9414

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KEVIN J HAMBLETT ET AL: "Effects of drug loading on the antitumor activity of a monoclonal antibody drug conjugate", CLINICAL CANCER RESEARCH, THE AMERICAN ASSOCIATION FOR CANCER RESEARCH, US, vol. 10, no. 20, 15 October 2004 (2004-10-15), pages 7063-7070, XP002623986, ISSN: 1078-0432, DOI: 10.1158/1078-0432.CCR-04-0789 | 1-8,11, 12 | INV. A61K47/68 A61K38/05 A61P35/00 |
| A | * page 7064, column 1, paragraph 4 – column 2, paragraph 2 * <br> * page 7065, column 2, paragraph 5 – page 7066, column 1, paragraph 1 * <br> * figures 1B, 1C * | 9,10, 13-15 | |
| X | JEAN-PHILIPPE STEPHAN ET AL: "Anti-CD22-MCC-DM1 and MC-MMAF Conjugates: Impact of Assay Format on Pharmacokinetic Parameters Determination", BIOCONJUGATE CHEMISTRY, vol. 19, no. 8, 1 August 2008 (2008-08-01), pages 1673-1683, XP055067530, ISSN: 1043-1802, DOI: 10.1021/bc800059t | 1-8,11, 12 | |
| A | * table 1 * <br> * page 1674, column 1, paragraph 2 – page 1675, column 1, paragraph 1 * <br> * figure 3 * <br> * page 1679, column 2, paragraph 3 * | 9,10, 13-15 | TECHNICAL FIELDS SEARCHED (IPC) <br><br> A61K |
| X,P | WO 2013/163631 A2 (CYTOMX THERAPEUTICS INC [US]) 31 October 2013 (2013-10-31) <br> * examples 9, 10 * | 12,13,15 | |
| A,D | WO 2011/041319 A2 (LUDWIG INST CANCER RES [US]; OLD LLOYD J [US]; JOHNS TERRANCE GRANT [A] 7 April 2011 (2011-04-07) <br> * examples 22, 25 * <br> * figures 55A, 55B * | 1-15 | |

The present search report has been drawn up for all claims

| Place of search | Date of completion of the search | Examiner |
|---|---|---|
| The Hague | 10 January 2023 | Monami, Amélie |

EPO FORM 1503 03.82 (P04C01)

## EP 4 137 160 A1

### ANNEX TO THE EUROPEAN SEARCH REPORT
### ON EUROPEAN PATENT APPLICATION NO.

EP 22 17 9414

This annex lists the patent family members relating to the patent documents cited in the above-mentioned European search report.
The members are as contained in the European Patent Office EDP file on
The European Patent Office is in no way liable for these particulars which are merely given for the purpose of information.

10-01-2023

| Patent document cited in search report | | Publication date | Patent family member(s) | | Publication date |
|---|---|---|---|---|---|
| WO 2013163631 | A2 | 31-10-2013 | AU | 2013251310 A1 | 06-11-2014 |
| | | | AU | 2018203262 A1 | 31-05-2018 |
| | | | BR | 112014026354 A2 | 27-06-2017 |
| | | | CA | 2871458 A1 | 31-10-2013 |
| | | | CN | 104540518 A | 22-04-2015 |
| | | | CN | 110078827 A | 02-08-2019 |
| | | | CO | 7160045 A2 | 15-01-2015 |
| | | | EP | 2841093 A2 | 04-03-2015 |
| | | | HK | 1204581 A1 | 27-11-2015 |
| | | | IL | 265788 A | 30-06-2019 |
| | | | JP | 2015516813 A | 18-06-2015 |
| | | | JP | 2019108331 A | 04-07-2019 |
| | | | KR | 20150035538 A | 06-04-2015 |
| | | | PE | 20150605 A1 | 28-05-2015 |
| | | | PH | 12014502641 A1 | 02-02-2015 |
| | | | RU | 2014147684 A | 20-06-2016 |
| | | | SG | 11201406943X A | 30-12-2014 |
| | | | US | 2013315906 A1 | 28-11-2013 |
| | | | US | 2016193332 A1 | 07-07-2016 |
| | | | US | 2017196996 A1 | 13-07-2017 |
| | | | US | 2018333507 A1 | 22-11-2018 |
| | | | US | 2021023243 A1 | 28-01-2021 |
| | | | WO | 2013163631 A2 | 31-10-2013 |
| | | | ZA | 201407715 B | 23-12-2015 |
| | | | ZA | 201507859 B | 30-08-2017 |
| WO 2011041319 | A2 | 07-04-2011 | AR | 080663 A1 | 02-05-2012 |
| | | | TW | 201124155 A | 16-07-2011 |
| | | | US | 2011076232 A1 | 31-03-2011 |
| | | | UY | 32915 A | 29-04-2011 |
| | | | WO | 2011041319 A2 | 07-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61792834
- WO 9005144 A1, Winter
- WO 2011041319 A
- US 20110076232 A
- GB 9101134 W
- WO 9002809 A
- WO 9110737 A
- WO 9201047 A
- WO 9218619 A
- WO 9311236 A
- WO 9515982 A
- WO 9520401 A
- US 5698426 A
- US 5223409 A
- US 5403484 A
- US 5580717 A
- US 5427908 A
- US 5750753 A
- US 5821047 A
- US 5571698 A
- US 5516637 A
- US 5780225 A
- US 5658727 A
- US 5733743 A
- US 5969108 A
- US 5635483 A
- US 20030083263
- US 20050238649 A
- US 20050009751 A
- WO 04010957 A
- WO 02088172 A
- US 6323315 B
- US 6239104 B
- US 6034065 A
- US 5780588 A
- US 5665860 A
- US 5663149 A

- US 5599902 A
- US 5554725 A
- US 5530097 A
- US 5521284 A
- US 5504191 A
- US 5410024 A
- US 5138036 A
- US 5076973 A
- US 4986988 A
- US 4978744 A
- US 4879278 A
- US 4816444 A
- US 4486414 A
- WO 8912624 A
- US 7091186 B
- US 7553816 B
- US 6214345 B
- US 4563304 A
- US 7090843 B
- US 7223837 B
- US 7659241 B
- US 20040018194 A
- US 20040121940 A
- US 20060116422 A
- US 20070258987 A
- US 20080213289 A
- US 20080241128 A
- US 20080311136 A
- US 20080317747 A
- US 20090010945 A
- US 5665358 A
- US 5643573 A
- US 5556623 A
- US 7837980 B
- US 20040157782 A1
- US 04038392 W

**Non-patent literature cited in the description**

- **KING et al.** *J Med. Chem.,* 2002, vol. 45, 4336
- **HOLLANDER et al.** *Bioconjugate Chem,* 2008, vol. 19, 358
- **BURKE et al.** *Bioconjugate Chem,* 2009, vol. 20, 1242
- **ZHAO et al.** *J Med. Chem.,* 2011, vol. 54, 3606
- **ALLEY et al.** *Proc Amer Assoc Cancer Res,* 2004, vol. 45

- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **HOLLIGER, P. et al.** *Proc. Natl. Acad. Sci. USA,* 1993, vol. 90, 6444-6448
- **POLJAK, R.J. et al.** *Structure,* 1994, vol. 2, 1121-1123

- Antibody Engineering. Springer-Verlag, 2001, 790
- **KABAT et al.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 877-883
- **PADLAN.** *FASEB J,* 1995, vol. 9, 133-139
- **MACCALLUM.** *J Mol Biol,* 1996, vol. 262 (5), 732-45
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **BRINKMAN et al.** *J Immunol Methods,* 1995, vol. 182, 41-50
- **AMES et al.** *J Immunol Methods,* 1995, vol. 184, 177-186
- **KETTLEBOROUGH et al.** *Eur J Immunol,* 1994, vol. 24, 952-958
- **PERSIC et al.** *Gene,* 1997, vol. 187, 9-18
- **BURTON et al.** *Advances in Immunology,* 1994, vol. 57, 191-280
- **URLAUB ; CHASIN.** *Proc. Natl. Acad. Sci. USA,* 1980, vol. 77, 4216-4220
- **KAUFMAN ; SHARP.** *J. Mol. Biol.,* 1982, vol. 159, 601-621
- **URLAUB et al.** *Som. Cell Molec. Genet.,* 1986, vol. 12, 555
- **HAYNES et al.** *Nuc. Acid. Res.,* 1983, vol. 11, 687-706
- **LAU et al.** *Mol. Cell. Biol.,* 1984, vol. 4, 1469-1475
- **G. R. PETTIT.** *Prog. Chem. Org. Nat. Prod,* 1997, vol. 70, 1-79
- Monoclonal Antibodies For Immunotargeting Of Drugs In Cancer Therapy. **ARNON et al.** Monoclonal Antibodies And Cancer Therapy. Alan R. Liss, Inc, 1985
- Antibodies For Drug Delivery. **HELLSTROM et al.** Controlled Drug Delivery. Marcel Dekker, Inc, 1987
- Antibody Carriers Of Cytotoxic Agents In Cancer Therapy: A Review. **THORPE et al.** Monoclonal Antibodies '84: Biological And Clinical Applications. 1985
- Analysis, Results, and Future Prospective of the Therapeutic Use of Radiolabeled Antibody In Cancer Therapy. Monoclonal Antibodies For Cancer Detection And Therapy. Academic Press, 1985
- **THORPE et al.** *Immunol. Rev.,* 1982, vol. 62, 119-58
- **YOSHITAKE et al.** *Eur. J. Biochem.,* 1979, vol. 101, 395-399
- Monoclonal Antibodies For Immunotargeting Of Drugs In Cancer Therapy. **AMON et al.** Monoclonal Antibodies And Cancer Therapy. Alan R. Liss, Inc, 1985, 243-56
- Antibodies For Drug Delivery. **HELLSTROM et al.** Controlled Drug Delivery. Marcel Dekker, Inc, 1987, 623-53
- Antibody Carriers Of Cytotoxic Agents In Cancer Therapy: A Review. **THORPE et al.** Monoclonal Antibodies '84: Biological And Clinical Applications. 1985, 475-506
- Analysis, Results, And Future Prospective Of The Therapeutic Use Of Radiolabeled Antibody In Cancer Therapy. Monoclonal Antibodies For Cancer Detection And Therapy. Academic Press, 1985, 303-16
- **THORPE et al.** The Preparation And Cytotoxic Properties Of Antibody-Toxin Conjugates. *Immunol. Rev.,* 1982, vol. 62, 119-58
- **CARTER ; SENTER.** *Cancer J,* 2008, vol. 14 (3), 154
- **V.R. SUTTON ; D.L. VAUX ; J.A. TRAPANI.** *J. of Immunology,* 1997, vol. 158 (12), 5783